# EUROPEAN PATENT SPECIFICATION

(11) **EP 2 784 162 B1**
(45) Date of publication and mention of the grant of the patent: **08.04.2015**
(21) Application number: 14170383.5
(22) Date of filing: 12.12.2013
(51) Int. Cl.: C12N 15/63

(54) **Engineering of systems, methods and optimized guide compositions for sequence manipulation**
Konstruktion von Systemen, Verfahren und optimierte Führungszusammensetzungen zur Sequenzmanipulation
Ingénierie de systèmes, procédés et compositions de guidage optimisé pour manipulation de séquence

(30) Priority: 12.12.2012 US 201261736527 P; 02.01.2013 US 201361748427 P; 30.01.2013 US 201361758468 P; 25.02.2013 US 201361769046 P; 15.03.2013 US 201361791409 P; 15.03.2013 US 201361802174 P; 28.03.2013 US 201361806375 P; 20.04.2013 US 201361814263 P; 06.05.2013 US 201361819803 P; 28.05.2013 US 201361828130 P; 17.06.2013 US 201361835931 P; 17.06.2013 US 201361836127 P
(43) Date of publication of application: 01.10.2014
(62) Divisional of application: 13818570.7
(73) Proprietor: The Broad Institute, Inc., Cambridge, MA 02142 (US); MASSACHUSETTS INSTITUTE OF TECHNOLOGY, Cambridge, MA 02142-1324 (US); President and Fellows of Harvard College, Cambridge, MA 02138-3876 (US)
(72) Inventor: Cong, Le, Cambridge, MA 02138 (US); Zhang, Feng, Cambridge, MA 02139 (US); Hsu, Patrick, Cambridge, MA 02138 (US); Ran, Fei, Boston, MA 02115 (US)
(74) Representative: Williams, Gareth Owen

(56) References cited:
- US-A1- 2010 076 057
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055067740, ISSN: 0036-8075, DOI: 10.1126/science.1225829 & M. JINEK ET AL: "A Programmable Dual-RNA-Guided DNA Endonuclease in Adaptive Bacterial Immunity (Supplementary Material)", SCIENCE, vol. 337, no. 6096, 28 June 2012 (2012-06-28), pages 816-821, XP055067747, ISSN: 0036-8075, DOI: 10.1126/science.1225829
- G. GASIUNAS ET AL: "PNAS Plus: Cas9-crRNA ribonucleoprotein complex mediates specific DNA cleavage for adaptive immunity in bacteria", PROCEEDINGS OF THE NATIONAL ACADEMY OF SCIENCES, vol. 109, no. 39, 25 September 2012 (2012-09-25), pages E2579-E2586, XP055068588, ISSN: 0027-8424, DOI: 10.1073/pnas.1208507109
- L. CONG ET AL: "Multiplex Genome Engineering Using CRISPR/Cas Systems", SCIENCE, vol. 339, no. 6121, 15 February 2013 (2013-02-15), pages 819-823, XP055102030, ISSN: 0036-8075, DOI: 10.1126/science.1231143 & L. CONG ET AL: "Supplementary Material to : Multiplex Genome Engineering Using CRISPR/Cas Systems", SCIENCE, vol. 339, no. 6121, 3 January 2013 (2013-01-03), pages 819-823, XP055067744, ISSN: 0036-8075, DOI: 10.1126/science.1231143
- JIANG WENYAN ET AL: "RNA-guided editing of bacterial genomes using CRISPR-Cas systems", NATURE BIOTECHNOLOGY,, vol. 31, no. 3, 29 January 2013 (2013-01-29), pages 233-239, XP002699849,
- BLAKE WIEDENHEFT ET AL: "RNA-guided genetic silencing systems in bacteria and archaea", NATURE, vol. 482, no. 7385, 15 February 2012 (2012-02-15), pages 331-338, XP055116249, ISSN: 0028-0836, DOI: 10.1038/nature10886
- ELITZA DELTCHEVA ET AL: "CRISPR RNA maturation by trans-encoded small RNA and host factor RNase III", NATURE, vol. 471, no. 7340, 31 March 2011 (2011-03-31), pages 602-607, XP055068535, ISSN: 0028-0836, DOI: 10.1038/nature09886
- MICHAEL P TERNS ET AL: "CRISPR-based adaptive immune systems", CURRENT OPINION IN MICROBIOLOGY, vol. 14, no. 3, 1 June 2011 (2011-06-01), pages 321-327, XP055097823, ISSN: 1369-5274, DOI: 10.1016/j.mib.2011.03.005
- GAJ THOMAS ET AL: "ZFN, TALEN, and CRISPR/Cas-based methods for genome engineering", TRENDS IN BIOTECHNOLOGY, vol. 31, no. 7, 1 July 2013 (2013-07-01), pages 397-405, XP028571313, ISSN: 0167-7799, DOI: 10.1016/J.TIBTECH.2013.04.004
- WANG HAOYI ET AL: "One-Step Generation of Mice Carrying Mutations in Multiple Genes by CRISPR/Cas-Mediated Genome Engineering", CELL, vol. 153, no. 4, 9 May 2013 (2013-05-09), pages 910-918, XP028538358, ISSN: 0092-8674, DOI: 10.1016/J.CELL.2013.04.025
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-451, XP055115843, ISSN: 0092-8674, DOI: 10.1016/j.cell.2013.06.044
- KRZYSZTOF CHYLINSKI ET AL: "The tracrRNA and Cas9 families of type II CRISPR-Cas immunity systems", RNA BIOLOGY, vol. 10, no. 5, 1 May 2013 (2013-05-01), pages 726-737, XP055116068, ISSN: 1547-6286, DOI: 10.4161/rna.24321
- WOONG Y HWANG ET AL: "Efficient genome editing in zebrafish using a CRISPR-Cas system", NATURE BIOTECHNOLOGY, vol. 31, no. 3, 29 January 2013 (2013-01-29), pages 227-229, XP055086625, ISSN: 1087-0156, DOI: 10.1038/nbt.2501

## Description

### FIELD OF THE INVENTION

The present disclosure generally relates to systems, methods and compositions used for the control of gene expression involving sequence targeting, such as genome perturbation or gene-editing, that may use vector systems related to Clustered Regularly Interspaced Short Palindromic Repeats (CRISPR) and components thereof.

### STATEMENT AS TO FEDERALLY SPONSORED RESEARCH

This invention was made with government support awarded by the National Institutes of Health, NIH Pioneer Award DP1MH100706. The government has certain rights in the invention.

### BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

### SUMMARY OF THE INVENTION

There exists a pressing need for alternative and robust systems and techniques for sequence targeting with a wide array of applications. This invention addresses this need and provides related advantages. The CRISPR/Cas or the CRISPR-Cas system (both terms are used interchangeably throughout this application) does not require the generation of customized proteins to target specific sequences but rather a single Cas enzyme can be programmed by a short RNA molecule to recognize a specific DNA target, in other words the Cas enzyme can be recruited to a specific DNA target using said short RNA molecule. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering and optimization of these genome engineering tools, which are aspects of the claimed invention.

In one aspect, the disclosure provides a vector system comprising one or more vectors. In some embodiments, the system comprises: (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a cell, e.g., eukaryotic cell, wherein the CRISPR complex comprises a CRISPR enzyme complexed with (1) the guide sequence that is hybridized to the target sequence, and (2) the tracr mate sequence that is hybridized to the tracr sequence; and (b) a second regulatory element operably linked to an enzyme-coding sequence encoding said CRISPR enzyme comprising a nuclear localization sequence; wherein components (a) and (b) are located on the same or different vectors of the system. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the system comprises the tracr sequence under the control of a third regulatory element, such as a polymerase III promoter. In some embodiments, the tracr sequence exhibits at least 50%, 60%, 70%, 80%, 90%, 95%, or 99% of sequence complementarity along the length of the tracr mate sequence when optimally aligned. In some embodiments, the CRISPR complex comprises one or more nuclear localization sequences of sufficient strength to drive accumulation of said CRISPR complex in a detectable amount in the nucleus of a eukaryotic cell. Without wishing to be bound by theory, it is believed that a nuclear localization sequence is not necessary for CRISPR complex activity in eukaryotes, but that including such sequences enhances activity of the system, especially as to targeting nucleic acid molecules in the nucleus. In some embodiments, the CRISPR enzyme is a type II CRISPR system enzyme. In some embodiments, the CRISPR enzyme is a Cas9 enzyme. In some embodiments, the Cas9 enzyme is *S. pneumonias, S. pyogenes,* or *S*. *thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length. In general, and throughout this specification, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell).

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol III promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter. Also encompassed by the term "regulatory element" are enhancer elements, such as WPRE; CMV enhancers; the R-U5' segment in LTR of HTLV-1 (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.).

Advantageous vectors include lentiviruses and adeno-associated viruses, and types of such vectors can also be selected for targeting particular types of cells.

In one aspect, the disclosure provides a vector comprising a regulatory element operably linked to an enzyme-coding sequence encoding a CRISPR enzyme comprising one or more nuclear localization sequences. In some embodiments, said regulatory element drives transcription of the CRISPR enzyme in a eukaryotic cell such that said CRISPR enzyme accumulates in a detectable amount in the nucleus of the eukaryotic cell. In some embodiments, the regulatory element is a polymerase II promoter. In some embodiments, the CRISPR enzyme is a type II CRISPR system enzyme. In some embodiments, the CRISPR enzyme is a Cas9 enzyme. In some embodiments, the Cas9 enzyme is *S*. *pneumoniae, S. pyogenes* or *S*. *thermophilus* Cas9, and may include mutated Cas9 derived from these organisms. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity.

In one aspect, the disclosure provides a CRISPR enzyme comprising one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S*. *pneumoniae, S. pyogenes* or *S*. *thermophilus* Cas9, and may include mutated Cas9 derived from these organisms. The enzyme may be a Cas9 homolog or ortholog. In some embodiments, the CRISPR enzyme lacks the ability to cleave one or more strands of a target sequence to which it binds.

In one aspect, the disclosure provides a eukaryotic host cell comprising (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. In some embodiments, the host cell comprises components (a) and (b). In some embodiments, component (a), component (b), or components (a) and (b) are stably integrated into a genome of the host eukaryotic cell. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the eukaryotic host cell further comprises a third regulatory element, such as a polymerase III promoter, operably linked to said tracr sequence. In some embodiments, the tracr sequence exhibits at least 50%, 60%, 70%, 80%, 90%, 95%, or 99% of sequence complementarity along the length of the tracr mate sequence when optimally aligned. In some embodiments, the CRISPR enzyme comprises one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S*. *pneumoniae, S. pyogenes* or *S. thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length. In an aspect, the disclosure provides a non-human eukaryotic organism; preferably a multicellular eukaryotic organism, comprising a eukaryotic host cell according to any of the described embodiments. In other aspects, the disclosure provides a eukaryotic organism; preferably a multicellular eukaryotic organism, comprising a eukaryotic host cell according to any of the described embodiments. The organism in some embodiments of these aspects may be an animal; for example a mammal. Also, the organism may be an arthropod such as an insect. The organism also may be a plant. Further, the organism may be a fungus.

In one aspect, the disclosure provides a kit comprising one or more of the components described herein. In some embodiments, the kit comprises a vector system and instructions for using the kit. In some embodiments, the vector system comprises (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. In some embodiments, the kit comprises components (a) and (b) located on the same or different vectors of the system. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the system further comprises a third regulatory element, such as a polymerase III promoter, operably linked to said tracr sequence. In some embodiments, the tracr sequence exhibits at least 50%, 60%, 70%, 80%, 90%, 95%, or 99% of sequence complementarity along the length of the tracr mate sequence when optimally aligned. In some embodiments, the CRISPR enzyme comprises one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes* or *S*. *thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length.

In one aspect, the disclosure provides a method of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering one or more vectors to said eukaryotic cell, wherein the one or more vectors drive expression of one or more of: the CRISPR enzyme, the guide sequence linked to the tracr mate sequence, and the tracr sequence. In some embodiments, said vectors are delivered to the eukaryotic cell in a subject. In some embodiments, said modifying takes place in said eukaryotic cell in a cell culture. In some embodiments, the method further comprises isolating said eukaryotic cell from a subject prior to said modifying. In some embodiments, the method further comprises returning said eukaryotic cell and/or cells derived therefrom to said subject.

In one aspect, the disclosure provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. In some embodiments, the method further comprises delivering one or more vectors to said eukaryotic cells, wherein the one or more vectors drive expression of one or more of: the CRISPR enzyme, the guide sequence linked to the tracr mate sequence, and the tracr sequence.

In one aspect, the disclosure provides a method of generating a model eukaryotic cell comprising a mutated disease gene. In some embodiments, a disease gene is any gene associated with an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, thereby generating a model eukaryotic cell comprising a mutated disease gene. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect, the disclosure provides a method for developing a biologically active agent that modulates a cell signaling event associated with a disease gene. In some embodiments, a disease gene is any gene associated with an increase in the risk of having or developing a disease. In some embodiments, the method comprises (a) contacting a test compound with a model cell of any one of the described embodiments; and (b) detecting a change in a readout that is indicative of a reduction or an augmentation of a cell signaling event associated with said mutation in said disease gene, thereby developing said biologically active agent that modulates said cell signaling event associated with said disease gene.

In one aspect, the disclosure provides a recombinant polynucleotide comprising a guide sequence upstream of a tracr mate sequence, wherein the guide sequence when expressed directs sequence-specific binding of a CRISPR complex to a corresponding target sequence present in a eukaryotic cell. In some embodiments, the target sequence is a viral sequence present in a eukaryotic cell. In some embodiments, the target sequence is a proto-oncogene or an oncogene.

In one aspect the disclosure provides for a method of selecting one or more prokaryotic cell(s) by introducing one or more mutations in a gene in the one or more prokaryotic cell (s), the method comprising: introducing one or more vectors into the prokaryotic cell (s), wherein the one or more vectors drive expression of one or more of: a CRISPR enzyme, a guide sequence linked to a tracr mate sequence, a tracr sequence, and a editing template; wherein the editing template comprises the one or more mutations that abolish CRISPR enzyme cleavage; allowing homologous recombination of the editing template with the target polynuceltide in the cell(s) to be selected; allowing a CRISPR complex to bind to a target polynucleotide to effect cleavage of the target polynucleotide within said gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, wherein binding of the CRISPR complex to the target polynuceotide induces cell death, thereby allowing one or more prokaryotic cell(s) in which one or more mutations have been introduced to be selected. In a preferred embodiment, the CRISPR enzyme is Cas9. In another aspect of the invention the cell to be selected may be a eukaryotic cell. Aspects of the invention allow for selection of specific cells without requiring a selection marker or a two-step process that may include a counter-selection system.

In some aspects the disclosure provides a non-naturally occurring or engineered composition comprising a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence wherein (a), (b) and (c) are arranged in a 5' to 3' orientation, wherein when transcribed, the tracr mate sequence hybridizes to the tracr sequence and the guide sequence directs sequence-specific binding of a CRISPR complex to the target sequence, wherein the CRISPR complex comprises a CRISPR enzyme complexed with (1) the guide sequence that is hybridized to the target sequence, and (2) the tracr mate sequence that is hybridized to the tracr sequence,
or
a CRISPR enzyme system, wherein the system is encoded by a vector system comprising one or more vectors comprising I. a first regulatory element operably linked to a CRISPR-Cas system chimeric RNA (chiRNA) polynucleotide sequence, wherein the polynucleotide sequence comprises (a) one or more guide sequences capable of hybridizing to one or more target sequences in a eukaryotic cell, (b) a tracr mate sequence, and (c) one or more tracr sequences, and II. a second regulatory element operably linked to an enzyme-coding sequence encoding a CRISPR enzyme comprising at least one or more nuclear localization sequences, wherein (a), (b) and (c) are arranged in a 5' to 3'orientation, wherein components I and II are located on the same or different vectors of the system, wherein when transcribed, the tracr mate sequence hybridizes to the tracr sequence and the guide sequence directs sequence-specific binding of a CRISPR complex to the target sequence, wherein the CRISPR complex comprises the CRISPR enzyme complexed with (1) the guide sequence that is hybridized to the target sequence, and (2) the tracr mate sequence that is hybridized to the tracr sequence, or a multiplexed CRISPR enzyme system, wherein the system is encoded by a vector system comprising one or more vectors comprising I. a first regulatory element operably linked to (a) one or more guide sequences capable of hybridizing to a target sequence in a cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, wherein the CRISPR complex comprises the CRISPR enzyme complexed with (1) the guide sequence that is hybridized to the target sequence, and (2) the tracr mate sequence that is hybridized to the tracr sequence, and wherein in the multiplexed system multiple guide sequences and a single tracr sequence is used; and wherein one or more of the guide, tracr and tracr mate sequences are modified to improve stability.

In aspects of the invention, the modification comprises an engineered secondary structure. For example, the modification can comprise a reduction in a region of hybridization between the tracr mate sequence and the tracr sequence. For example, the modification also may comprise fusing the tracr mate sequence and the tracr sequence through an artificial loop. The modification may comprise the tracr sequence having a length between 40 and 120bp. In embodiments, the tracr sequence is between 40 bp and full length of the tracr. In certain embodiments, the length of tracRNA includes at least nucleotides 1-67 and in some embodiments at least nucleotides 1-85 of the wild type tracRNA. In some embodiments, at least nucleotides corresponding to nucleotides 1-67 or 1-85 of wild type S. pyogenes Cas9 tracRNA may be used. Where the CRISPR system uses enzymes other than Cas9, or other than SpCas9, then corresponding nucleotides in the relevant wild type tracRNA may be present. In some embodiments, the length of tracRNA includes no more than nucleotides 1-67 or 1-85 of the wild type tracRNA. The modification may comprise sequence optimization. In certain aspects, sequence optimization may comprise reducing the incidence of polyT sequences in the tracr and/or tracr mate sequence. Sequence optimization may be combined with reduction in the region of hybridization between the tracr mate sequence and the tracr sequence; for example, a reduced length tracr sequence.

In an aspect the disclosure provides the CRISPR-Cas system or CRISPR enzyme system wherein the modification comprises reduction in polyT sequences in the tracr and/or tracr mate sequence. In some aspects, one or more Ts present in a poly-T sequence of the relevant wild type sequence (that is, a stretch of more than 3, 4, 5, 6, or more contiguous T bases; in some embodiments, a stretch of no more than 10, 9, 8, 7, 6 contiguous T bases) may be substituted with a non-T nucleotide, e.g., an A, so that the string is broken down into smaller stretches of Ts with each stretch having 4, or fewer than 4 (for example, 3 or 2) contiguous Ts. Bases other than A may be used for substitution, for example C or G, or non-naturally occuring nucleotides or modified nucleotides. If the string of Ts is involved in the formation of a hairpin (or stem loop), then it is advantageous that the complementary base for the non-T base be changed to complement the non-T nucleotide. For example, if the non-T base is an A, then its complement may be changed to a T, e.g., to preserve or assist in the preservation of secondary structure. For instance, 5'-TTTTT can be altered to become 5'-TTTAT and the complementary 5'-AAAAA can be changed into 5'-ATAAA.

In an aspect the disclosure provides the CRISPR-Cas system or CRISPR enzyme system wherein the modification comprises adding a polyT terminator sequence. In an aspect the disclosure provides the CRISPR-Cas system or CRISPR enzyme system wherein the modification comprises adding a polyT terminator sequence in tracr and/or tracr mate sequences. In an aspect the disclosure provides the CRISPR-Cas system or CRISPR enzyme system wherein the modification comprises adding a polyT terminator sequence in the guide sequence. The polyT terminator sequence may comprise 5 contiguous T bases, or more than 5.

In an aspect the disclosure provides the CRISPR-Cas system or CRISPR enzyme system wherein the modification comprises altering loops and/or hairpins. In an aspect the disclosure provides the CRISPR-Cas system or CRISPR enzyme system wherein the modification comprises providing a minimum of two hairpins in the guide sequence. In an aspect the disclosure provides the CRISPR-Cas system or CRISPR enzyme system wherein the modification comprises providing a hairpin formed by complementation between the tracr and tracr mate (direct repeat) sequence. In an aspect the disclosure provides the CRISPR-Cas system or CRISPR enzyme system wherein the modification comprises providing one or more further hairpin(s) at or towards the 3' end of the tracrRNA sequence. For example, a hairpin may be formed by providing self complementary sequences within the tracRNA sequence joined by a loop such that a hairpin is formed on self folding. In an aspect the disclosure provides the CRISPR-Cas system or CRISPR enzyme system wherein the modification comprises providing additional hairpins added to the 3' of the guide sequence. In an aspect the disclosure provides the CRISPR-Cas system or CRISPR enzyme system wherein the modification comprises extending the 5' end of the guide sequence. In an aspect the disclosure provides the CRISPR-Cas system or CRISPR enzyme system wherein the modification comprises providing one or more hairpins in the 5' end of the guide sequence. In an aspect the disclosure provides the CRISPR-Cas system or CRISPR enzyme system wherein the modification comprises appending the sequence (5'-AGGACGAAGTCCTAA) to the 5' end of the guide sequence. Other sequences suitable for forming hairpins will be known to the skilled person, and may be used in certain aspects of the invention. In some aspects, at least 2, 3, 4, 5, or more additional hairpins are provided. In some aspects, no more than 10, 9, 8, 7, 6 additional hairpins are provided. In an aspect the disclosure provides the CRISPR-Cas system or CRISPR enzyme system wherein the modification comprises two hairpins. In an aspect the disclosure provides the CRISPR-Cas system or CRISPR enzyme system wherein the modification comprises three hairpins. In an aspect the disclosure provides the CRISPR-Cas system or CRISPR enzyme system wherein the modification comprises at most five hairpins.

In an aspect the disclosure provides the CRISPR-Cas system or CRISPR enzyme system wherein the modification comprises providing cross linking, or providing one or more modified nucleotides in the polynucleotide sequence. Modified nucleotides and/or cross linking may be provided in any or all of the tracr, tracr mate, and/or guide sequences, and/or in the enzyme coding sequence, and/or in vector sequences. Modifications may include inclusion of at least one non naturally occurring nucleotide, or a modified nucleotide, or analogs thereof Modified nucleotides may be modified at the ribose, phosphate, and/or base moiety. Modified nucleotides may include 2'-O-methyl analogs, 2'-deoxy analogs, or 2'-fluoro analogs. The nucleic acid backbone may be modified, for example, a phosphorothioate backbone may be used. The use of locked nucleic acids (LNA) or bridged nucleic acids (BNA) may also be possible. Further examples of modified bases include, but are not limited to, 2-aminopurine, 5-bromo-uridine, pseudouridine, inosine, 7-methylguanosine.

It will be understood that any or all of the above modifications may be provided in isolation or in combination in a given CRISPR-Cas system or CRISPR enzyme system. Such a system may include one, two, three, four, five, or more of said modifications.

In an aspect the invention provides the CRISPR-Cas system or CRISPR enzyme system wherein the CRISPR enzyme is a type II CRISPR system enzyme, e.g., a Cas9 enzyme. In an aspect the disclosure provides the CRISPR-Cas system or CRISPR enzyme system wherein the CRISPR enzyme is comprised of less than one thousand amino acids, or less than four thousand amino acids. In an aspect the disclosure provides the CRISPR-Cas system or CRISPR enzyme system wherein the Cas9 enzyme is StCas9 or StlCas9, or the Cas9 enzyme is a Cas9 enzyme from an organism selected from the group consisting of genus Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium or Corynebacter. In an aspect the invention provides the CRISPR-Cas system or CRISPR enzyme system wherein the CRISPR enzyme is a nuclease directing cleavage of both strands at the location of the target sequence.

In an aspect the disclosure provides the CRISPR-Cas system or CRISPR enzyme system wherein the first regulatory element is a polymerase III promoter. In an aspect the disclosure provides the CRISPR-Cas system or CRISPR enzyme system wherein the second regulatory element is a polymerase II promoter.

In an aspect the disclosure provides the CRISPR-Cas system or CRISPR enzyme system wherein the guide sequence comprises at least fifteen nucleotides.

In an aspect the disclosure provides the CRISPR-Cas system or CRISPR enzyme system wherein the modification comprises optimized tracr sequence and/or optimized guide sequence RNA and/or co-fold structure of tracr sequence and/or tracr mate sequence(s) and/or stabilizing secondary structures of tracr sequence and/or tracr sequence with a reduced region of base-pairing and/or tracr sequence fused RNA elements; and/or, in the multiplexed system there are two RNAs comprising a tracer and comprising a plurality of guides or one RNA comprising a plurality of chimerics.

In aspects the chimeric RNA architecture is further optimized according to the results of mutagenesis studies. In chimeric RNA with two or more hairpins, mutations in the proximal direct repeat to stabilize the hairpin may result in ablation of CRISPR complex activity. Mutations in the distal direct repeat to shorten or stabilize the hairpin may have no effect on CRISPR complex activity. Sequence randomization in the bulge region between the proximal and distal repeats may significantly reduce CRISPR complex activity. Single base pair changes or sequence randomization in the linker region between hairpins may result in complete loss of CRISPR complex activity. Hairpin stabilization of the distal hairpins that follow the first hairpin after the guide sequence may result in maintenance or improvement of CRISPR complex activity. Accordingly, in preferred embodiments, the chimeric RNA architecture may be further optimized by generating a smaller chimeric RNA which may be beneficial for therapeutic delivery options and other uses and this may be achieved by altering the distal direct repeat so as to shorten or stabilize the hairpin. In further preferred embodiments, the chimeric RNA architecture may be further optimized by stabilizing one or more of the distal hairpins. Stabilization of hairpins may include modifying sequences suitable for forming hairpins. In some aspects, at least 2, 3, 4, 5, or more additional hairpins are provided. In some aspects, no more than 10, 9, 8, 7, 6 additional hairpins are provided. In some aspects stabilization may be cross linking and other modifications. Modifications may include inclusion of at least one non naturally occurring nucleotide, or a modified nucleotide, or analogs thereof. Modified nucleotides may be modified at the ribose, phosphate, and/or base moiety. Modified nucleotides may include 2'-O-methyl analogs, 2'-deoxy analogs, or 2'-fluoro analogs. The nucleic acid backbone may be modified, for example, a phosphorothioate backbone may be used. The use of locked nucleic acids (LNA) or bridged nucleic acids (BNA) may also be possible. Further examples of modified bases include, but are not limited to, 2-aminopurine, 5-bromo-uridine, pseudouridine, inosine, 7-methylguanosine.

In an aspect the disclosure provides the CRISPR-Cas system or CRISPR enzyme system wherein the CRISPR enzyme is codon-optimized for expression in a eukaryotic cell.

Accordingly, in some aspects, the length of tracRNA required in a construct of the invention, e.g., a chimeric construct, need not necessarily be fixed, and in some aspects it can be between 40 and 120bp, and in some aspects up to the full length of the tracr, e.g., in some aspects, until the 3' end of tracr as punctuated by the transcription termination signal in the bacterial genome. In certain embodiments, the length of tracRNA includes at least nucleotides 1-67 and in some embodiments at least nucleotides 1-85 of the wild type tracRNA. In some embodiments, at least nucleotides corresponding to nucleotides 1-67 or 1-85 of wild type S. pyogenes Cas9 tracRNA may be used. Where the CRISPR system uses enzymes other than Cas9, or other than SpCas9, then corresponding nucleotides in the relevant wild type tracRNA may be present. In some embodiments, the length oftracRNA includes no more than nucleotides 1-67 or 1-85 of the wild type tracRNA With respect to sequence optimization (e.g., reduction in polyT sequences), e.g., as to strings of Ts internal to the tracr mate (direct repeat) or tracrRNA, in some aspects, one or more Ts present in a poly-T sequence of the relevant wild type sequence (that is, a stretch of more than 3, 4, 5, 6, or more contiguous T bases; in some embodiments, a stretch of no more than 10, 9, 8, 7, 6 contiguous T bases) may be substituted with a non-T nucleotide, e.g., an A, so that the string is broken down into smaller stretches of Ts with each stretch having 4, or fewer than 4 (for example, 3 or 2) contiguous Ts. If the string of Ts is involved in the formation of a hairpin (or stem loop), then it is advantageous that the complementary base for the non-T base be changed to complement the non-T nucleotide. For example, if the non-T base is an A, then its complement may be changed to a T, e.g., to preserve or assist in the preservation of secondary structure. For instance, 5'-TTTTT can be altered to become 5'-TTTAT and the complementary 5'-AAAAA can be changed into 5'-ATAAA. As to the presence of polyT terminator sequences in tracr + tracr mate transcript, e.g., a polyT terminator (TTTTT or more), in some aspects it is advantageous that such be added to end of the transcript, whether it is in two RNA (tracr and tracr mate) or single guide RNA form. Concerning loops and hairpins in tracr and tracr mate transcripts, in some aspects it is advantageous that a minimum of two hairpins be present in the chimeric guide RNA. A first hairpin can be the hairpin formed by complementation between the tracr and tracr mate (direct repeat) sequence. A second hairpin can be at the 3' end of the tracrRNA sequence, and this can provide secondary structure for interaction with Cas9. Additional hairpins may be added to the 3' of the guide RNA, e.g., in some aspects of the invention to increase the stability of the guide RNA. Additionally, the 5' end of the guide RNA, in some aspects, may be extended. In some aspects, one may consider 20bp in the 5' end as a guide sequence. The 5' portion may be extended. One or more hairpins can be provided in the 5' portion, e.g., in some aspects, this may also improve the stability of the guide RNA. In some aspects, the specific hairpin can be provided by appending the sequence (5'-AGGACGAAGTCCTAA) to the 5' end of the guide sequence, and, in some aspects , this may help improve stability. Other sequences suitable for forming hairpins will be known to the skilled person, and may be used in certain aspects. In some aspects, at least 2, 3, 4, 5, or more additional hairpins are provided. In some aspects, no more than 10, 9, 8, 7, 6 additional hairpins are provided. The foregoing also provides aspects involving secondary structure in guide sequences. In some aspects there may be cross linking and other modifications, e.g., to improve stability. Modifications may include inclusion of at least one non naturally occurring nucleotide, or a modified nucleotide, or analogs thereof. Modified nucleotides may be modified at the ribose, phosphate, and/or base moiety. Modified nucleotides may include 2'-O-methyl analogs, 2'-deoxy analogs, or 2'-fluoro analogs. The nucleic acid backbone may be modified, for example, a phosphorothioate backbone may be used. The use of locked nucleic acids (LNA) or bridged nucleic acids (BNA) may also be possible. Further examples of modified bases include, but are not limited to, 2-aminopurine, 5-bromo-uridine, pseudouridine, inosine, 7-methylguanosine. Such modifications or cross linking may be present in the guide sequence or other sequences adjacent the guide sequence.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

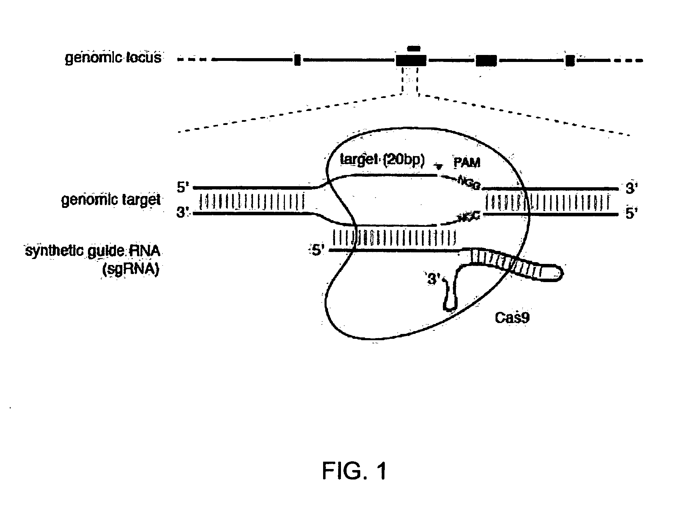

**Figure 1** shows a schematic model of the CRISPR system. The Cas9 nuclease from Streptococcus pyogenes (yellow) is targeted to genomic DNA by a synthetic guide RNA (sgRNA) consisting of a 20-nt guide sequence (blue) and a scaffold (red). The guide sequence base-pairs with the DNA target (blue), directly upstream of a requisite 5'-NGG protospacer adjacent motif (PAM; magenta), and Cas9 mediates a double-stranded break (DSB) ~3 bp upstream of the PAM (red triangle).
**Figure 2A-F** illustrates an exemplary CRISPR system, a possible mechanism of action, an example adaptation for expression in eukaryotic cells, and results of tests assessing nuclear localization and CRISPR activity.
**Figure 3A-C** illustrates an exemplary expression cassette for expression of CRISPR system elements in eukaryotic cells, predicted structures of example guide sequences, and CRISPR system activity as measured in eukaryotic and prokaryotic cells.
**Figure 4A-D** illustrates results of an evaluation of SpCas9 specificity for an example target.
**Figure 5A-G** illustrates an exemplary vector system and results for its use in directing homologous recombination in eukaryotic cells.
**Figure 6A-C** illustrates a comparison of different tracrRNA transcripts for Cas9-mediated gene targeting.
**Figure 7A-D** illustrates an exemplary CRISPR system, an example adaptation for expression in eukaryotic cells, and results of tests assessing CRISPR activity.
**Figure 8A-C** illustrates exemplary manipulations of a CRISPR system for targeting of genomic loci in mammalian cells.
**Figure 9A-B** illustrates the results of a Northern blot analysis of crRNA processing in mammalian cells.
**Figure 10A-C** illustrates a schematic representation of chimeric RNAs and results of SURVEYOR assays for CRISPR system activity in eukaryotic cells.
**Figure 11A-B** illustrates a graphical representation of the results of SURVEYOR assays for CRISPR system activity in eukaryotic cells.
**Figure 12** illustrates predicted secondary structures for exemplary chimeric RNAs comprising a guide sequence, tracr mate sequence, and tracr sequence.
**Figure 13** is a phylogenetic tree of Cas genes

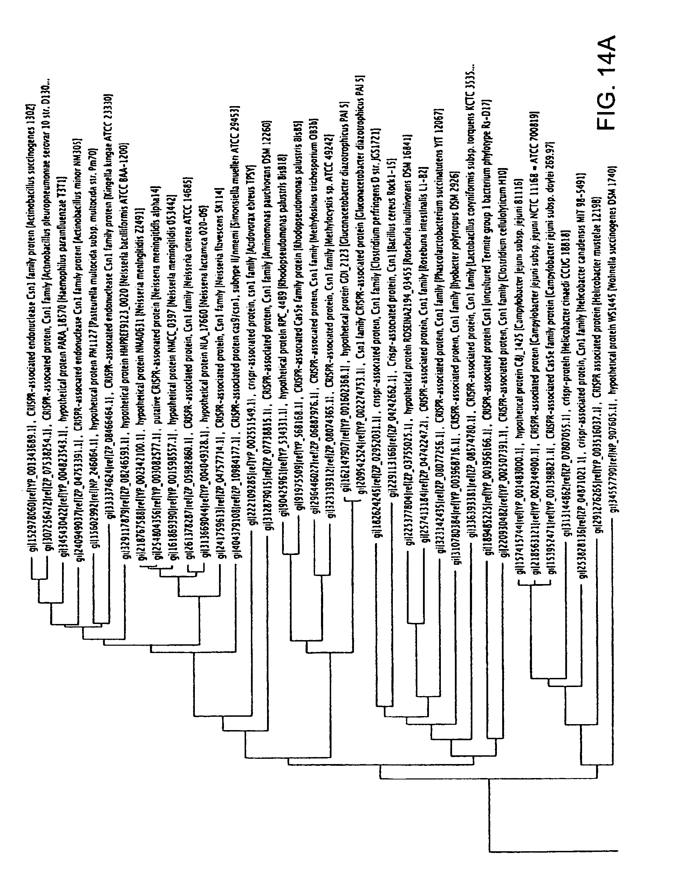

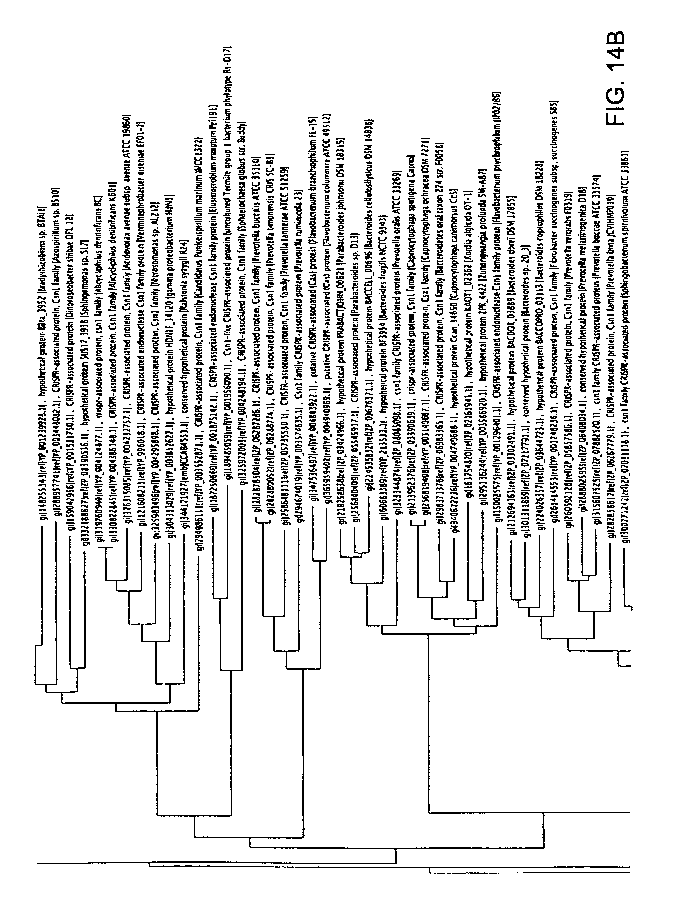

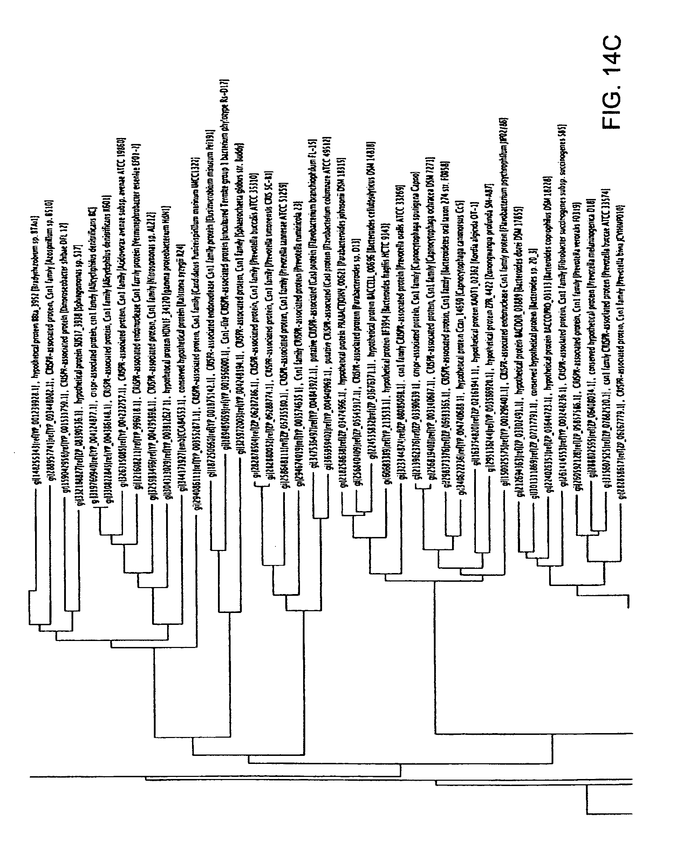

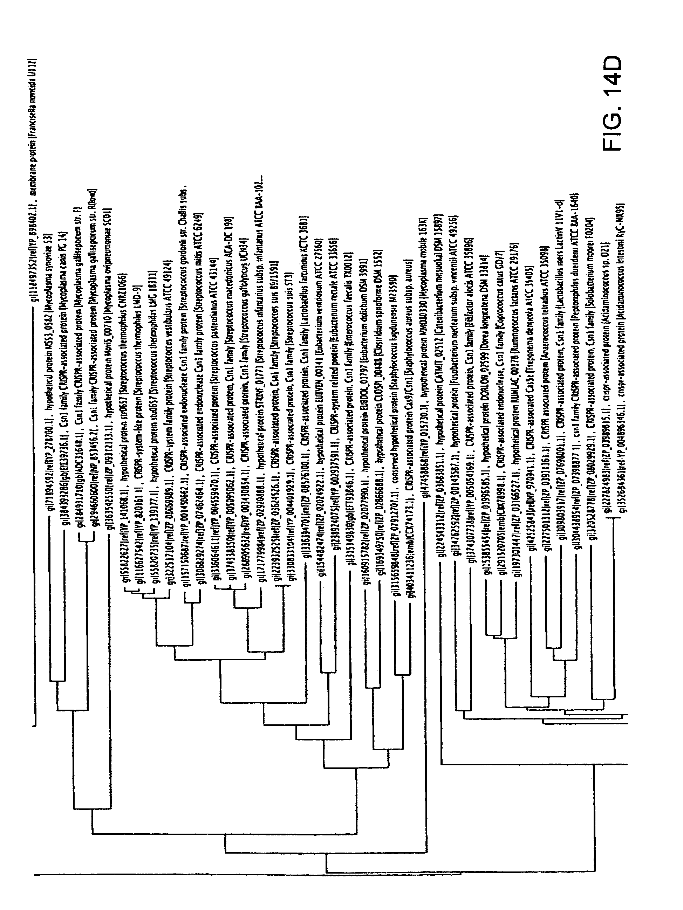

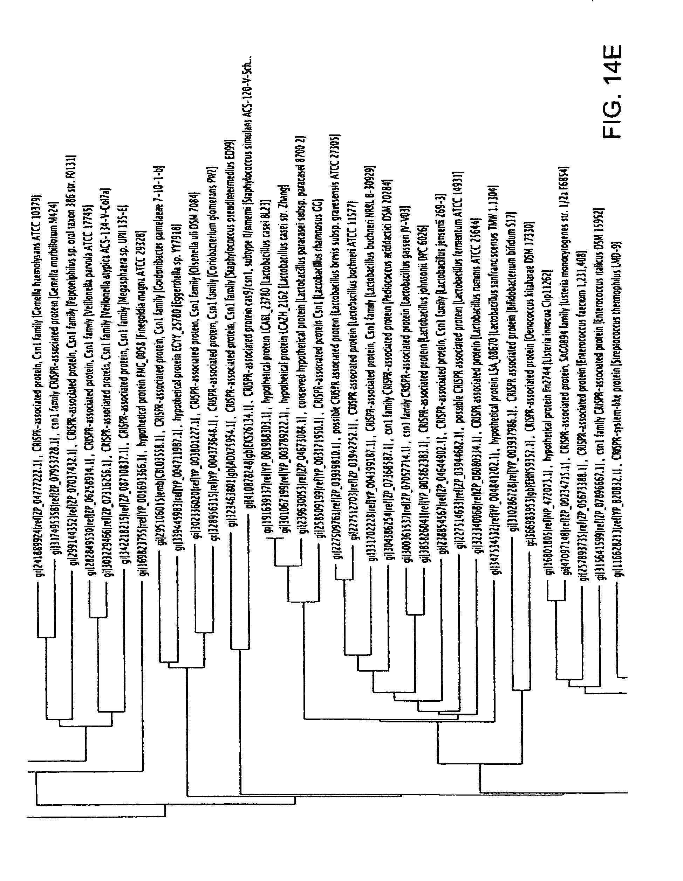

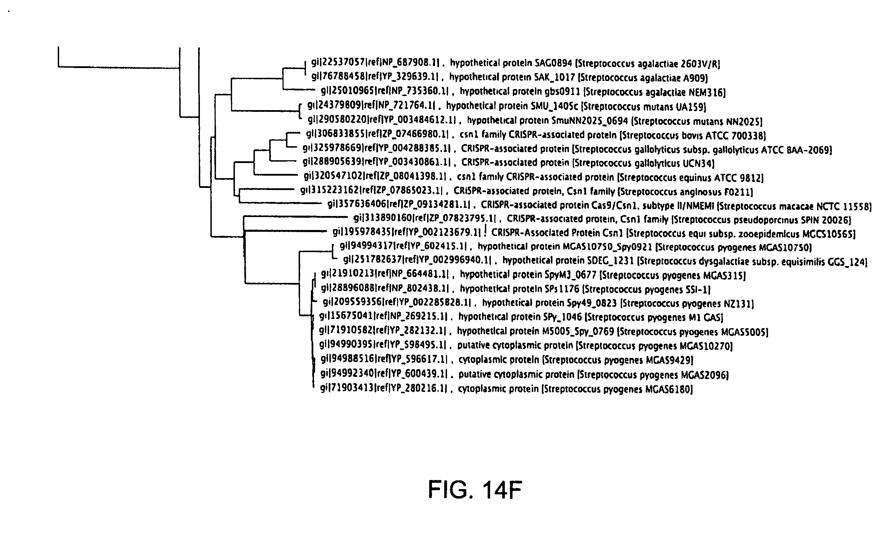

**Figure 14A-F** shows the phylogenetic analysis revealing five families of Cas9s, including three groups of large Cas9s (~1400 amino acids) and two of small Cas9s (~1100 amino acids).

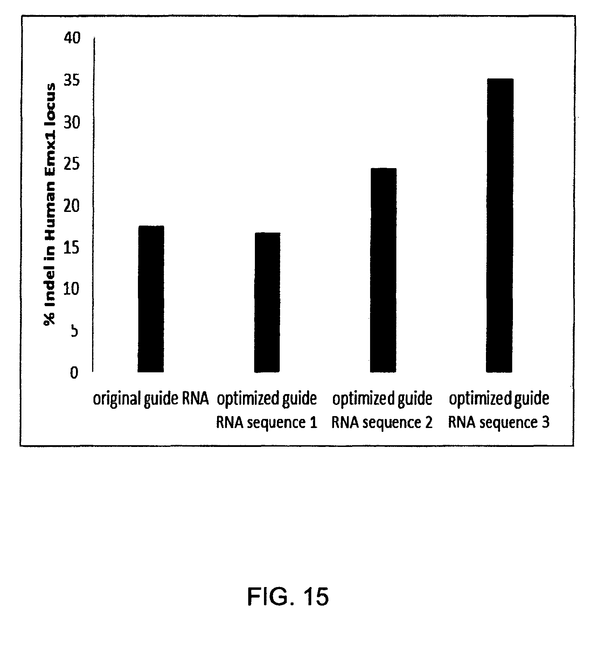

**Figure 15** shows a graph depicting the function of different optimized guide RNAs.

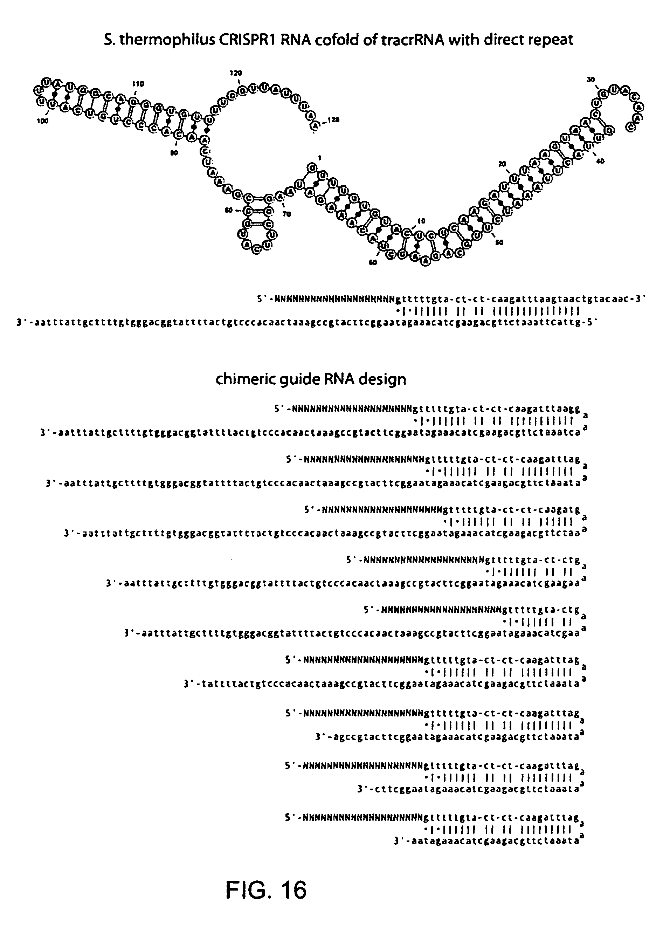

**Figure 16** shows the sequence and structure of different guide chimeric RNAs.

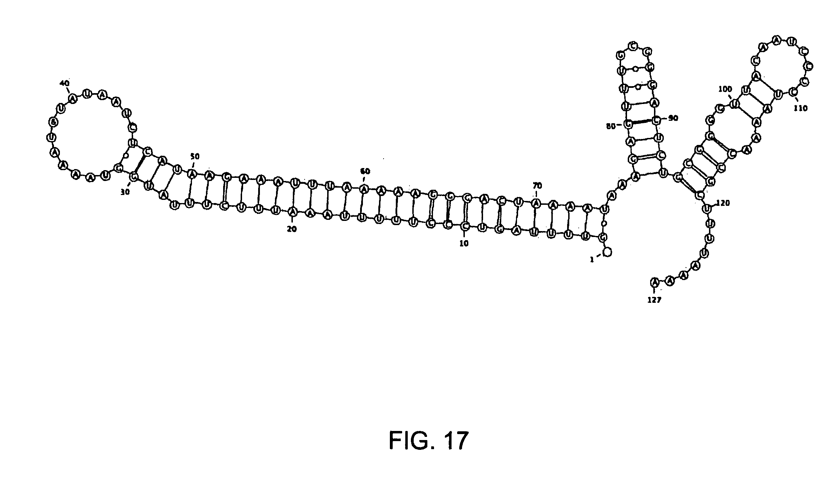

**Figure 17** shows the co-fold structure of the tracrRNA and direct repeat.
**Figure 18** A and B shows data from the St1Cas9 chimeric guide RNA optimization *in vitro.*

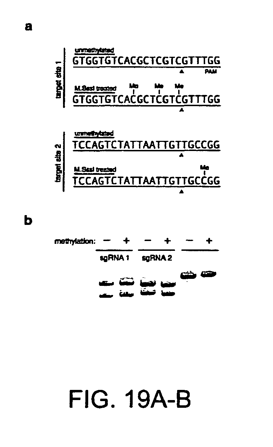

**Figure 19A-B** shows cleavage of either unmethylated or methylated targets by SpCas9 cell lysate.

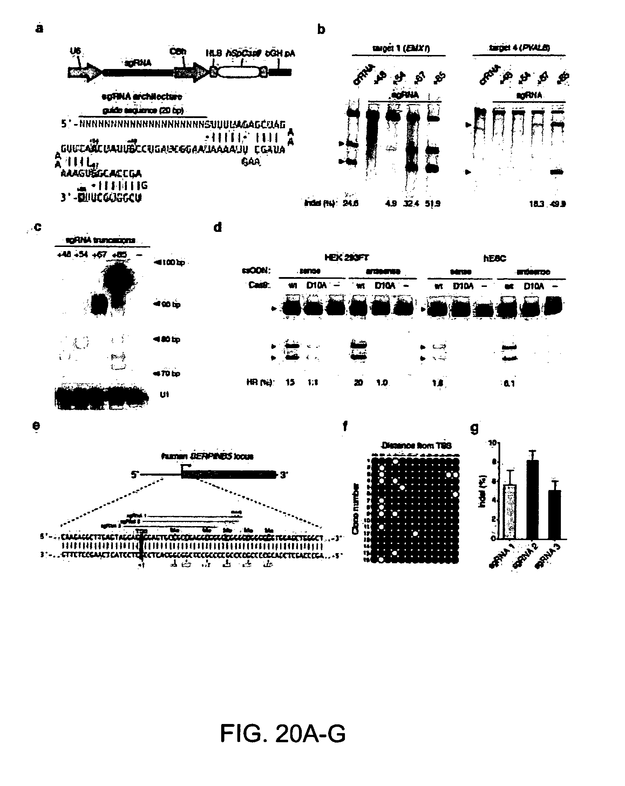

**Figure 20A-G** shows the optimization of guide RNA architecture for SpCas9-mediated mammalian genome editing. (a) Schematic of bicistronic expression vector (PX330) for U6 promoter-driven single guide RNA (sgRNA) and CBh promoter-driven human codon-optimized *Streptococcus pyogenes* Cas9 (*hSpCas9*) used for all subsequent experiments. The sgRNA consists of a 20-nt guide sequence (blue) and scaffold (red), truncated at various positions as indicated. (b) SURVEYOR assay for SpCas9-mediated indels at the human *EMX1* and *PVALB* loci. Arrows indicate the expected SURVEYOR fragments (*n* = 3). (c) Northern blot analysis for the four sgRNA truncation architectures, with U1 as loading control. (d) Both wildtype (wt) or nickase mutant (D10A) of SpCas9 promoted insertion of a *Hin*dIII site into the human *EMX1* gene. Single stranded oligonucleotides (ssODNs), oriented in either the sense or antisense direction relative to genome sequence, were used as homologous recombination templates. (e) Schematic of the human *SERPINB5* locus. sgRNAs and PAMs are indicated by colored bars above sequence; methylcytosine (Me) are highlighted (pink) and numbered relative to the transcriptional start site (TSS, +1). (f) Methylation status of *SERPINB5* assayed by bisulfite sequencing of 16 clones. Filled circles, methylated CpG; open circles, unmethylated CpG. (g) Modification efficiency by three sgRNAs targeting the methylated region of *SERPINB5*, assayed by deep sequencing (*n* = 2). Error bars indicate Wilson intervals (Online Methods).

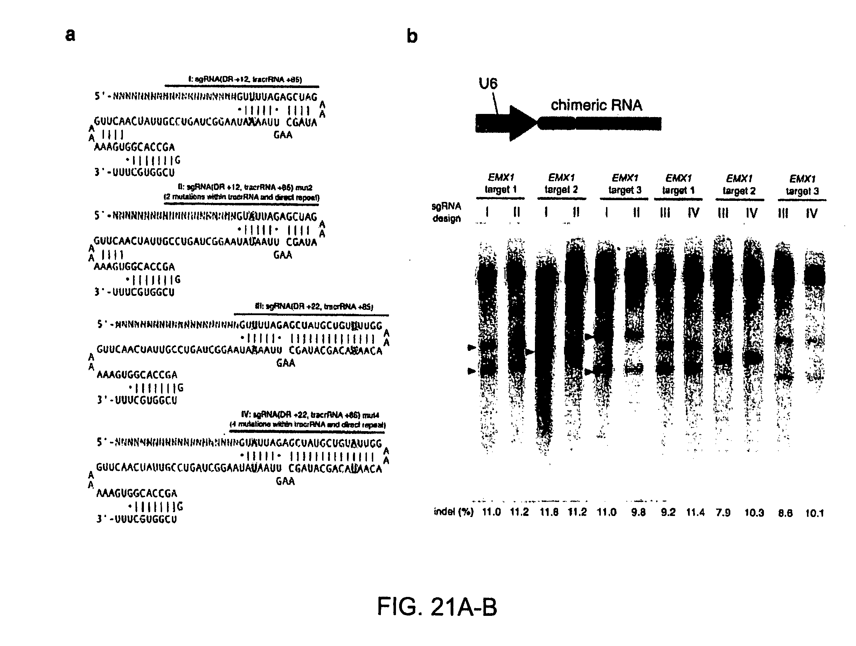

**Figure 21A-B** shows the further optimization of CRISPR-Cas sgRNA architecture. (a) Schematic of four additional sgRNA architectures, I-IV. Each consists of a 20-nt guide sequence (blue) joined to the direct repeat (DR, grey), which hybridizes to the tracrRNA (red). The DR-tracrRNA hybrid is truncated at +12 or +22, as indicated, with an artificial GAAA stem loop. tracrRNA truncation positions are numbered according to the previously reported transcription start site for tracrRNA. sgRNA architectures II and IV carry mutations within their poly-U tracts, which could serve as premature transcriptional terminators. (b) SURVEYOR assay for SpCas9-mediated indels at the human *EMX1* locus for target sites 1-3. Arrows indicate the expected SURVEYOR fragments (*n* = 3).
**Figure 22** illustrates visualization of some target sites in the human genome.
**Figure 23A-B** shows (A) a schematic of the sgRNA and (B) the SURVEYOR analysis of five sgRNA variants for SaCas9 for an optimal truncated architecture with highest cleavage efficiency

The figures herein are for illustrative purposes only and are not necessarily drawn to scale.

### DETAILED DESCRIPTION OF THE INVENTION

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non nucleotide components. A polynucleotide may be further modified after polymerization, such as by conjugation with a labeling component.

In aspects of the invention the terms "chimeric RNA", "chimeric guide RNA", "guide RNA", "single guide RNA" and "synthetic guide RNA" are used interchangeably and refer to the polynucleotide sequence comprising the guide sequence, the tracr sequence and the tracr mate sequence. The term "guide sequence" refers to the about 20bp sequence within the guide RNA that specifies the target site and may be used interchangeably with the terms "guide" or "spacer". The term "tracr mate sequence" may also be used interchangeably with the term "direct repeat(s)".

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base-pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, "stabilization" or "increasing stability" with respect to components of the CRISPR system relate to securing or steadying the structure of the molecule. This may be accomplished by introduction of one or mutations, including single or multiple base pair changes, increasing the number of hair pins, cross linking, breaking up particular stretches of nucleotides and other modifications. Modifications may include inclusion of at least one non naturally occurring nucleotide, or a modified nucleotide, or analogs thereof Modified nucleotides may be modified at the ribose, phosphate, and/or base moiety. Modified nucleotides may include 2'-O-methyl analogs, 2'-deoxy analogs, or 2'-fluoro analogs. The nucleic acid backbone may be modified, for example, a phosphorothioate backbone may be used. The use of locked nucleic acids (LNA) or bridged nucleic acids (BNA) may also be possible. Further examples of modified bases include, but are not limited to, 2-aminopurine, 5-bromo-uridine, pseudouridine, inosine, 7-methylguanosine. These modifications may apply to any component of the CRSIPR system. In a preferred embodiment these modifications are made to the RNA components, e.g. the guide RNA or chimeric polynucleotide sequence.

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed. In some embodiments, a subject may be an invertebrate animal, for example, an insect or a nematode; while in others, a subject may be a plant or a fungus.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

Several aspects of the invention relate to vector systems comprising one or more vectors, or vectors as such. Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as *Escherichia coli,* insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego; Calif. (1990). Alternatively, the recombinant expression vector can be transcribed and translated in vitro, for example using T7 promoter regulatory sequences and T7 polymerase.

Vectors may be introduced and propagated in a prokaryote. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calf. (1990) 60-89).

In some embodiments, a vector is a yeast expression vector. Examples of vectors for expression in yeast *Saccharomyces cerivisae* include pYepSec1 (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the α-fetoprotein promoter (Campes and Tilghman, 1989. Genes Dev. 3: 537-546).

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in *E. coli* (Ishino et al., J. Bacteriol., 169:5429-5433 [1987]; and Nakata et al., J. Bacteriol., 171:3553-3556 [1989]), and associated genes. Similar interspersed SSRs have been identified in *Haloferax mediterranei, Streptococcus pyogenes, Anabaena,* and *Mycobacterium tuberculosis* (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to *Aeropyrum, Pyrobaculum, Sulfolobus, Archaeoglobus. Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella. Acinetobacter, Erwinia, Escherichia, Legionella, Methylococcus, Pasteurella, Photobacterium, Salmonella, Xanthomonas, Yersinia, Treponema,* and *Thermotoga.*

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In some embodiments, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridisation and promote formation of a CRISPR complex. A target sequence may comprise any polynucleotide, such as DNA or RNA polynucleotides. In some embodiments, a target sequence is located in the nucleus or cytoplasm of a cell. In some embodiments, the target sequence may be within an organelle of a eukaryotic cell, for example, mitochondrion or chloroplast. A sequence or template that may be used for recombination into the the targeted locus comprising the target sequences is refered to as an "editing template" or "editing polynucleotide" or "editing sequence". In aspects of the invention, an exogenous template polynucleotide may be referred to as an editing template. In an aspect of the invention the recombination is homologous recombination.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, the tracr sequence has sufficient complementarity to a tracr mate sequence to hybridise and participate in formation of a CRISPR complex. As with the target sequence, it is believed that complete complementarity is not needed, provided there is sufficient to be functional. In some embodiments, the tracr sequence has at least 50%, 60%, 70%, 80%, 90%, 95% or 99% of sequence complementarity along the length of the tracr mate sequence when optimally aligned. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of *S*. *pyogenes* Cas9 protein may be found in the SwissProt database under accession number Q99ZW2. In some embodiments, the unmodified CRISPR enzyme has DNA cleavage activity, such as Cas9. In some embodiments the CRISPR enzyme is Cas9, and may be Cas9 from *S*. *pyogenes* or *S*. *pneumoniae.* In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from *S*. *pyogenes* converts Cas9 from a nuclease that cleaves both strands to a nickase (cleaves a single strand). Other examples of mutations that render Cas9 a nickase include, without limitation, H840A, N854A, and N863A. In some embodiments, a Cas9 nickase may be used in combination with guide sequenc(es), e.g., two guide sequences, which target respectively sense and antisense strands of the DNA target. This combination allows both strands to be nicked and used to induce NHEJ. Applicants have demonstrated (data not shown) the efficacy of two nickase targets (i.e., sgRNAs targeted at the same location but to different strands of DNA) in inducing mutagenic NHEJ. A single nickase (Cas9-D10A with a single sgRNA) is unable to induce NHEJ and create indels but Applicants have shown that double nickase (Cas9-D10A and two sgRNAs targeted to different strands at the same location) can do so in human embryonic stem cells (hESCs). The efficiency is about 50% of nuclease (i.e., regular Cas9 without D10 mutation) in hESCs.

As a further example, two or more catalytic domains of Cas9 (RuvC I, RuvC II, and RuvC III) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form. Other mutations may be useful; where the Cas9 or other CRISPR enzyme is from a species other than *S. pyogenes,* mutations in corresponding amino acids may be made to achieve similar effects.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the Nor C-terminus. Typically, an NLS consists of one or more short sequences of positively charged lysines or arginines exposed on the protein surface, but other types of NLS are known. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Examples of detectable markers include fluorescent proteins (such as Green fluorescent proteins, or GFP; RFP; CFP), and epitope tags (HA tag, flag tag, SNAP tag). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. For example, for the *S*. *pyogenes* Cas9, a unique target sequence in a genome may include a Cas9 target site of the form MMMMMMMMNNNNNNNNNNNNXGG where NNNNNNNNNNNNXGG (N is A, G, T, or C; and X can be anything) has a single occurrence in the genome. A unique target sequence in a genome may include an *S*. *pyogenes* Cas9 target site of the form MMMMMMMMMNNNNNNNNNNNXGG where NNNNNNNNNNNXGG (N is A, G, T, or C; and X can be anything) has a single occurrence in the genome. For the *S*. *thermophilus* CRISPR1 Cas9, a unique target sequence in a genome may include a Cas9 target site of the form MMMMMMMMNNNNNNNNNNNNXXAGAA W where NNNNNNNNNNNNXXAGAAW (N is A, G, T, or C; X can be anything; and W is A or T) has a single occurrence in the genome. A unique target sequence in a genome may include an *S*. *thermophilus* CRISPR1 Cas9 target site of the form MMMMMMMMMNNNNNNNNNNNXXAGAAW where NNNNNNNNNNNXXAGAAW (N is A, G, T, or C; X can be anything; and W is A or T) has a single occurrence in the genome. For the *S*. *pyogenes* Cas9, a unique target sequence in a genome may include a Cas9 target site of the form MMMMMMMMNNNNNNNNNNNNXGGXG where NNNNNNNNNNNNXGGXG (N is A, G, T, or C; and X can be anything) has a single occurrence in the genome. A unique target sequence in a genome may include an *S*. *pyogenes* Cas9 target site of the form MMMMMMMMNMNNNNNNNNNNXGGXG where NNNNNNNNNNNXGGXG (N is A, G, T, or C; and X can be anything) has a single occurrence in the genome. In each of these sequences "M" may be A, G, T, or C, and need not be considered in identifying a sequence as unique.

In some embodiments, a guide sequence is selected to reduce the degree of secondary structure within the guide sequence. Secondary structure may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

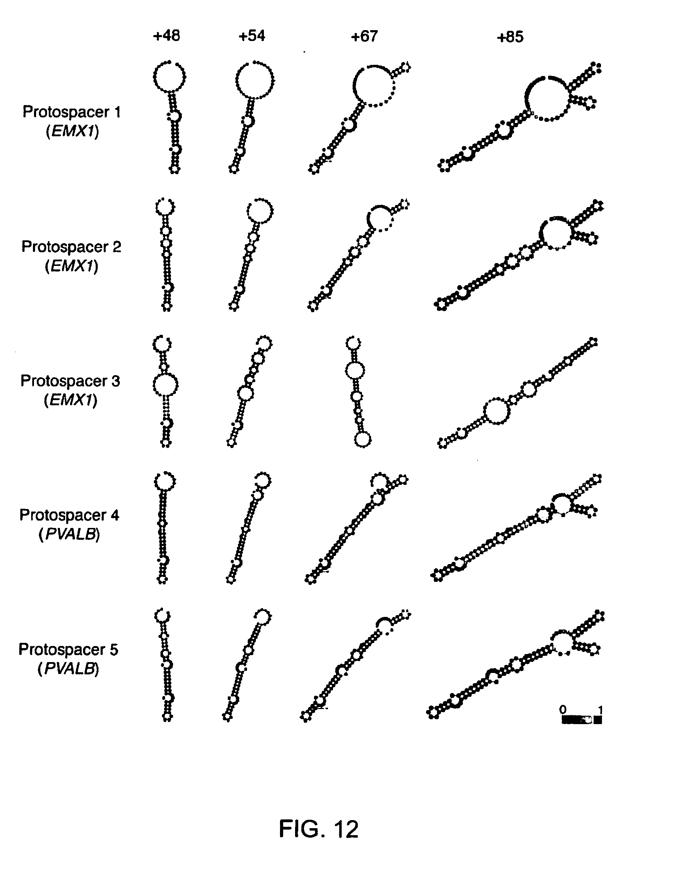

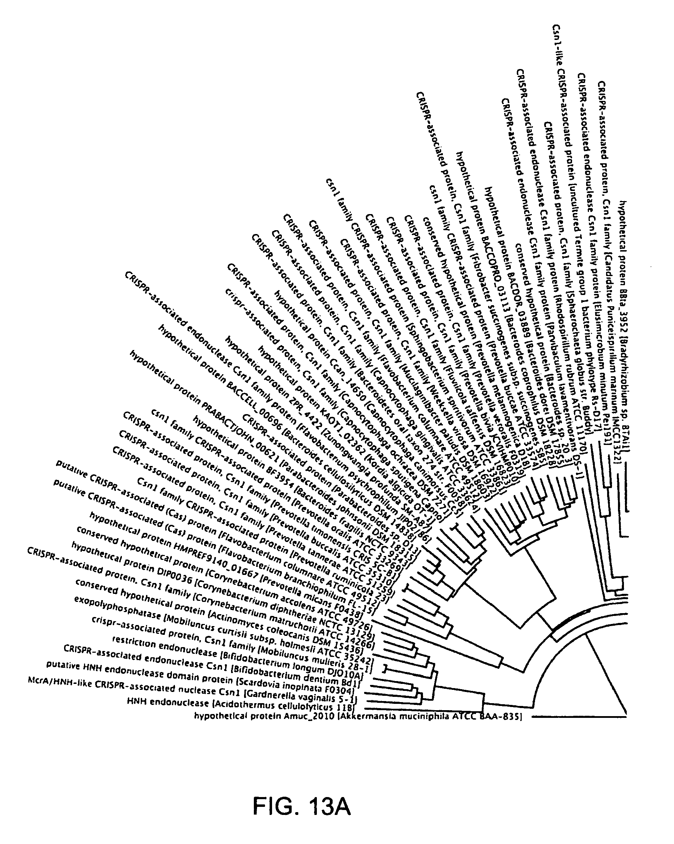

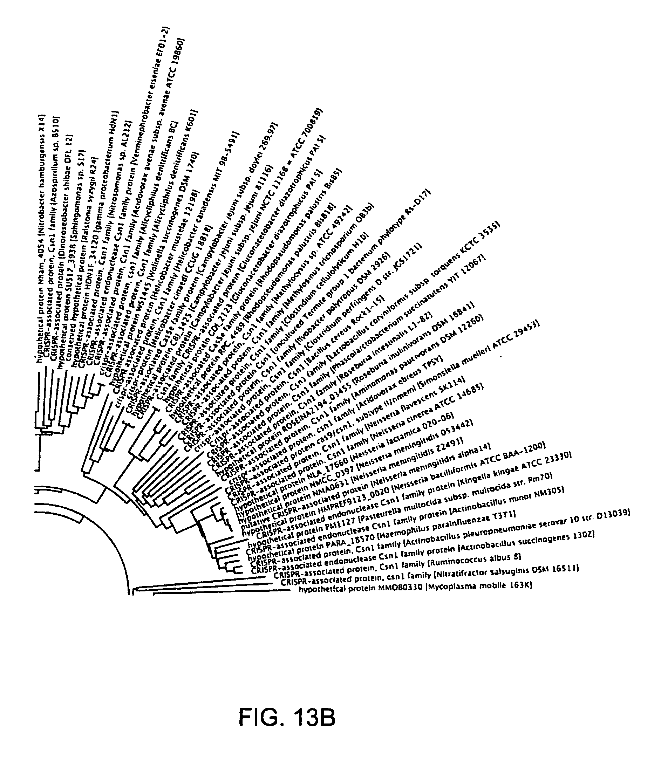

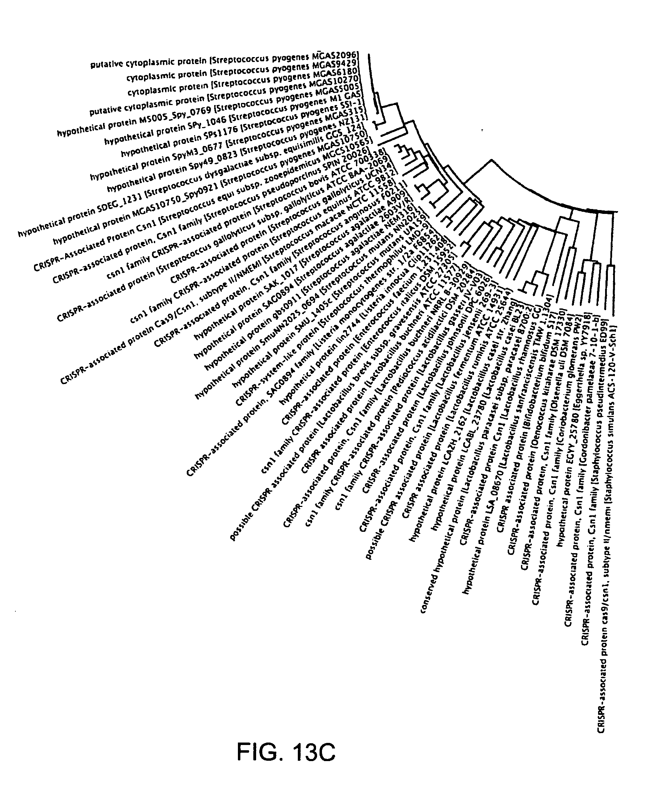

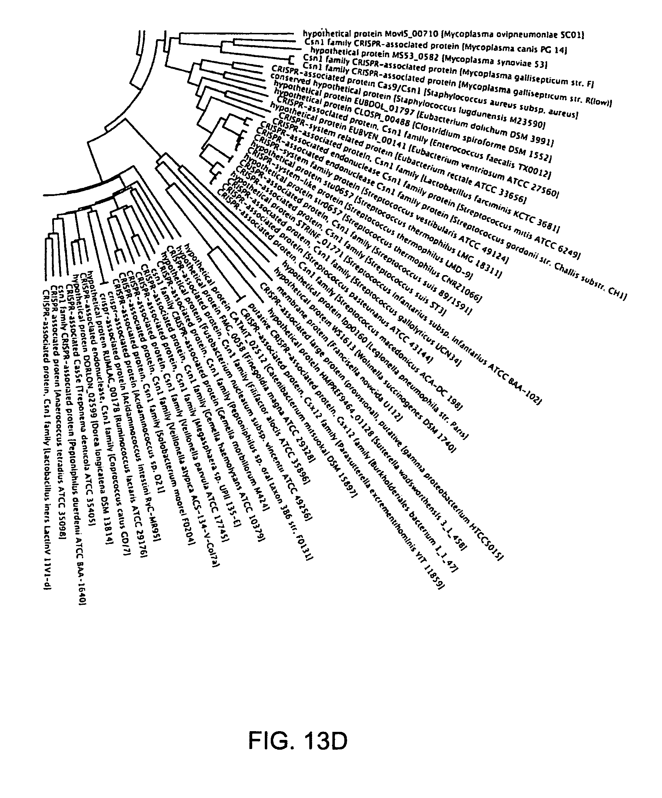

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. Example illustrations of optimal alignment between a tracr sequence and a tracr mate sequence are provided in Figures 12B and 13B. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. Preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In an embodiment, the transcript or transcribed polynucleotide sequence has at least two or more hairpins. In preferred embodiments, the transcript has two, three, four or five hairpins. In a further embodiment, the transcript has at most five hairpins. In some embodiments, the single transcript further includes a transcription termination sequence; preferably this is a polyT sequence, for example six T nucleotides. An example illustration of such a hairpin structure is provided in the lower portion of Figure 13B, where the portion of the sequence 5' of the final "N" and upstream of the loop corresponds to the tracr mate sequence, and the portion of the sequence 3' of the loop corresponds to the tracr sequence. Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) gtttttgtactctcaagatttaGAAAtaaatcttgcagaagctacaaagataaggctt catgccgaaatcaacaccctgtcattttatggcagggtgttttcgttatttaaTTTTTT; (2) gtttttgtactctcaGAAAtgcagaagctacaaagataaggcttcatgccgaaatca acaccctgtcattttatggcagggtgttttcgttatttaaTTTTTT; (3) NNNNNNNNNNNNNNNNNNNNgtttttgtactctcaGAAAtgcagaagctacaaagataaggcttcatgccgaaatea acaccctgtcattttatggcagggtgtTTTTTT; (4) NNNNNNNNNNNNNNNNNNNNgttttagagctaGAAAtagcaagttaaaataaggctagtccgttatcaacttgaaaa agtggcaccgagtcggtgcTTTTTT; (5) NNNNNNNNNNNNNNNNNNNNgttttagagetaGAAATAGcaagttaaaataaggetagtecgttatcaacttgaa aaagtgTTTTTTT; and (6) NNNNNNNNNNNNNNNNNNNNgttttagagetagAAATAGcaagttaaaataaggetagtccgttatcaTTTTT TTT. In some embodiments, sequences (1) to (3) are used in combination with Cas9 from *S*. *thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence (such as illustrated in the top portion of Figure 13B).

In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, 25, 30, 35, 40, 45, 50, 60, 70, 80, 90, 100 or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502. In some embodiments, a tagged CRISPR enzyme is used to identify the location of a target sequence.

In some aspects, the disclosure provides methods comprising delivering one or more polynucleotides, such as or one or more vectors as described herein, one or more transcripts thereof, and/or one or proteins transcribed therefrom, to a host cell. In some aspects, the disclosure further provides cells produced by such methods, and organisms (such as animals, plants, or fungi) comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids include lipofection, nucleofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids takes advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producing a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Additional methods for the delivery of nucleic acids to cells are known to those skilled in the art. See, for example, US20030087817.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepa1c1c7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant. In some embodiments, the transgenic animal is a mammal, such as a mouse, rat, or rabbit. In certain embodiments, the organism or subject is a plant. In certain embodiments, the organism or subject or plant is algae. Methods for producing transgenic plants and animals are known in the art, and generally begin with a method of cell transfection, such as described herein. Transgenic animals are also provided, as are transgenic plants, especially crops and algae. The transgenic animal or plant may be useful in applications outside of providing a disease model. These may include food or feed production through expression of, for instance, higher protein, carbohydrate, nutrient or vitamins levels than would normally be seen in the wildtype. In this regard, transgenic plants, especially pulses and tubers, and animals, especially mammals such as livestock (cows, sheep, goats and pigs), but also poultry and edible insects, are preferred.

Transgenic algae or other plants such as rape may be particularly useful in the production of vegetable oils or biofuels such as alcohols (especially methanol and ethanol), for instance. These may be engineered to express or overexpress high levels of oil or alcohols for use in the oil or biofuel industries.

In one aspect, the disclosure provides for methods of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, the disclosure provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence.

With recent advances in crop genomics, the ability to use CRISPR-Cas systems to perform efficient and cost effective gene editing and manipulation will allow the rapid selection and comparison of single and and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. Morrell et al "Crop genomics:advances and applications" Nat Rev Genet. 2011 Dec 29;13(2):85-96. In an advantageous embodiment of the invention, the CRISPR/Cas9 system is used to engineer microalgae (Example 14). Accordingly, reference herein to animal cells may also apply, mutatis mutandis, to plant cells unless otherwise apparent.

In one aspect, the disclosure provides for methods of modifying a target polynucleotide in a eukaryotic cell, which may be in vivo, ex vivo or in vitro. In some embodiments, the method comprises sampling a cell or population of cells from a human or non-human animal or plant (including micro-algae), and modifying the cell or cells. Culturing may occur at any stage ex vivo. The cell or cells may even be re-introduced into the non-human animal or plant (including micro-algae).

In one aspect, the disclosure provides kits containing any one or more of the elements disclosed in the above methods and compositions. In some embodiments, the kit comprises a vector system and instructions for using the kit. In some embodiments, the vector system comprises (a) a first regulatory element operably linked to a tracr mate sequence and one or more insertion sites for inserting a guide sequence upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. Elements may provide individually or in combinations, and may provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, the disclosure provides methods for using one or more elements of a CRISPR system. The CRISPR complex of the invention provides an effective means for modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex of the invention has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, it is believed that the target sequence should be associated with a PAM (protospacer adjacent motif); that is, a short sequence recognised by the CRISPR complex. The precise sequence and length requirements for the PAM differ depending on the CRISPR enzyme used, but PAMs are typically 2-5 base pair sequences adjacent the protospacer (that is, the target sequence) Examples of PAM sequences are given in the examples section below, and the skilled person will be able to identify further PAM sequences for use with a given CRISPR enzyme.

The target polynucleotide of a CRISPR complex may include a number of disease-associated genes and polynucleotides as well as signaling biochemical pathway-associated genes and polynucleotides.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are available from McKusick-Nathans Institute of Genetic Medicine, Johns Hopkins University (Baltimore, Md.) and National Center for Biotechnology Information, National Library of Medicine (Bethesda, Md.), available on the World Wide Web.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Such genes, proteins and pathways may be the target polynucleotide of a CRISPR complex.

**Table A**

| DISEASE/DISORDERS | GENE(S) |
|---|---|
| Neoplasia | PTEN; ATM; ATR; EGFR; ERBB2; ERBB3; ERBB4; |
| | Notch1; Notch2; Notch3; Notch4; AKT; AKT2; AKT3; HIF; |
| | HIF1a; HIF3a; Met; HRG; Bcl2; PPAR alpha; PPAR |
| | gamma; WT1 (Wilms Tumor); FGF Receptor Family |
| | members (5 members: 1, 2, 3, 4, 5); CDKN2a; APC; RB |
| | (retinoblastoma); MEN1; VHL; BRCA1; BRCA2; AR |
| | (Androgen Receptor); TSG101; IGF; IGF Receptor; Igf1 (4 |
| | variants); Igf2 (3 variants); Igf 1 Receptor; Igf 2 Receptor; |
| | Bax; Bcl2; caspases family (9 members: |
| | 1, 2, 3, 4, 6, 7, 8, 9, 12); Kras; Apc |
| Age-related Macular | Abcr; Cc12; Cc2; cp (ceruloplasmin); Timp3; cathepsinD; |
| Degeneration | Vldlr; Ccr2 |
| Schizophrenia | Neuregulin1 (Nrg1); Erb4 (receptor for Neuregulin); |
| | Complexin1 (Cplx1); Tph1 Tryptophan hydroxylase; Tph2 |
| | Tryptophan hydroxylase 2; Neurexin 1; GSK3; GSK3a; |
| | GSK3b |
| Disorders | 5-HTT (Slc6a4); COMT; DRD (Drd1a); SLC6A3; DAOA; |
| | DTNBP1; Dao (Dao1) |
| Trinucleotide Repeat | HTT (Huntington's Dx); SBMA/SMAX1/AR (Kennedy's |
| Disorders | Dx); FXN/X25 (Friedrich's Ataxia); ATX3 (Machado- |
| | Joseph's Dx); ATXN1 and ATXN2 (spinocerebellar |
| | ataxias); DMPK (myotonic dystrophy); Atrophin-1 and Atn1 |
| | (DRPLA Dx); CBP (Creb-BP - global instability); VLDLR |
| | (Alzheimer's); Atxn7; Atxn10 |
| Fragile X Syndrome | FMR2; FXR1; FXR2; mGLUR5 |
| Secretase Related | APH-1 (alpha and beta); Presenilin (Psen1); nicastrin |
| Disorders | (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; |
| | VEGF-b; VEGF-c) |
| Drug addiction | Prkce (alcohol); Drd2; Drd4; ABAT (alcohol); GRIA2; |
| | Grm5; Grin1; Htr1b; Grin2a; Drd3; Pdyn; Gria1 (alcohol) |
| Autism | Mecp2; BZRAP1; MDGA2; Sema5A; Neurexin 1; Fragile X |
| | (FMR2 (AFF2); FXR1; FXR2; Mglur5) |
| Alzheimer's Disease | E1; CHIP; UCH; UBB; Tau; LRP; PICALM.; Clusterin; PS1; |
| | SORL1; CR1; Vldlr; Uba1; Uba3; CHIP28 (Aqp1, |
| | Aquaporin 1); Uchl1; Uchl3; APP |
| Inflammation | IL-10; IL-1 (IL-1a; IL-1b); IL-13; IL-17 (IL-17a (CTLA8); IL- |
| | 17b; IL-17c; IL-17d; IL-17f); II-23; Cx3cr1; ptpn22; TNFa; |
| | NOD2/CARD15 for IBD; IL-6; IL-12 (IL-12a; IL-12b); |
| | CTLA4; Cx3cl1 |
| Parkinson's Disease | x-Synuclein; DJ-1; LRRK2; Parkin; PINK1 |

**Table B:**

| | |
|---|---|
| Blood and coagulation diseases and disorders | Anemia (CDAN1, CDA1, RPS19, DBA, PKLR, PK1, NT5C3, BUMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
| | |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF 145, PLZF, PML, MYL, STAT5B, AF 10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), II-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, C1RH1A, NAIC, TEX292, K1AA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD 1, PKD2, PKD4, PKDTS, PRKCSH, G19P1, PCLD, SEC63). |
| | |
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD21, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
| | |
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drd1a), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Nat1, Nat2); Trinucleotide Repeat Disorders (HTT (Huntington's Dx), SBMA/SMAX1/AR (Kennedy's Dx), FXN/X25 (Friedrich's Ataxia), ATX3 (Machado- Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
| | |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; |
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKCA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor | RAC1; TAF4B; EP300; SMAD2; TRAF6; PCAF; ELK1; |
| Signaling | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPKB; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |
| | CFL1; GNAQ; PIK3CB; CXCL 12; PIK3C3; WNT11; |
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAK1; |
| | PRKAA2; EIF2AK2; RAC I; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton | ACTN4; PRKCE; ITGAM; ROCK1; ITGA5; IRAK1; |
| Signaling | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; PIK3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPKB; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |
| | BIRC4; GRK6; MAPK1; CAPNS1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGRI; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; TTGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response | IRAK1; SOD2; MYD88; TRAF6; ELK1; MAPK1; PTPN11; |
| Signaling | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L 11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor | HSPB1; EP300; FASN; TGM2; RXRA; MAPK1; NQO1; |
| Signaling | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN I A; NCOA2; APAF1; NFKB1; CCND1; ATM; ESR1; |
| | CDKN2A; MYC; JUN; ESR2; BAX; IL6; CYP1B1; |
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAK1; PRKAA2; EIF2AK2; RAC I ; ELK1; |
| | GRK6; MAPK1; GADD45A; RAC2; PLK1; AKT2; PIK3CA; |
| | FADD; CDK8; PIK3CB; PIK3C3; MAPK8; R1PK1; |
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB 1; |
| | TGFBR1; SMAD4; JUN; IL1R1; PRKCA; IL6; HSP90AA1; ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF 1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; 1GF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |
| | PRKCA |
| Inositol Phosphate | PRKCE; IRAK1; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOXO1; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and | PRKCE; IRAK1; PRKAA2; EIF2AK2; GRK6; MAPK1; |
| Nicotinamide | |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; FLAN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAK1; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAK1; MYD88; TRAF6; PPARA; RXRA; ABCA1; |
| ofRXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS 1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCKS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |
| | PKM2; ENTPD1; RAD51; RRM2B; TJP2; RAD51C; |
| | NT5E; POLD1; NME1 |
| cAMP-mediated Signaling | RAP1A; MAPK1; GNAS; CREB1; CAMK2A; MAPK3 ; |
| | SRC; RAF1; MAP2K2; STAT3; MAP2K1; BRAF; ATF4 |
| Mitochondrial Dysfunction | SOD2; MAPK8; CASP8; MAPK10; MAPK9; CASP9; |
| | PARK7; PSEN1; PARK2; APP; CASP3 |
| Notch Signaling | HES1; JAG1; NUMB; NOTCH4; ADAM 17; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD1; RRM2B; |
| | NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism | |
| Phospholipid Degradation | PRDX6; PLD1; GRN; YWHAZ; SPHK1; SPHK2 |
| Cryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway | |
| Starch and Sucrose | UCHL1; HK2; GCK; GPI; HK1 |
| Metabolism | |
| Aminosugars Metabolism | NQO1; HK2; GCK; HK1 |
| Arachidonic Acid | PRDX6; GRN; YWHAZ; CYP1B1 |
| Metabolism | |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling | |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline | ALDH1A1; NOS3; NOS2A |
| Metabolism | |
| Eicosanoid Signaling | PRDX6; GRN; YWHAZ |
| Fructose and Mannose | HK2; GCK; HK I |
| Metabolism | |
| Galactose Metabolism | HK2; GCK; HK1 |
| Stilbene, Coumarine and | PRDX6; PRDX1; TYR |
| Lignin Biosynthesis | |
| Antigen Presentation | CALR; B2M |
| Pathway | |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid | PRDX6; CHKA |
| Metabolism | |
| Histidine Metabolism | PRMT5; ALDH1A1 |
| Inositol Metabolism | ERO1L; APEX1 |
| Metabolism of Xenobiotics | GSTP1; CYP1B1 |
| by Cytochrome p450 | |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid | PRMT5; AHCY |
| Metabolism | |
| Sphingolipid Metabolism | SPHK1; SPHK2 |
| Aminophosphonate | PRMT5 |
| Metabolism | |
| Androgen and Estrogen | PRMT5 |
| Metabolism | |
| Ascorbate and Aldarate | ALDH1A1 |
| Metabolism | |
| Bile Acid Biosynthesis | ALDH1A1 |
| Cysteine Metabolism | LDHA |
| Fatty Acid Biosynthesis | FASN |
| Glutamate Receptor | GNB2L1 |
| Signaling | |
| NRF2-mediated Oxidative | PRDX1 |
| Stress Response | |
| Pentose Phosphate | GPI |
| Pathway | |
| Pentose and Glucuronate | UCHL1 |
| Interconversions | |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and | ALDH1A1 |
| Isoleucine Degradation | |
| Glycine, Serine and | CHKA |
| Threonine Metabolism | |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Reln |

Embodiments also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011-Medical). Specific aspects of tandem repeat sequences have been found to be responsible for more than twenty human diseases (New insights into repeat instability: role of RNA·DNA hybrids. McIvor EI, Polak U, Napierala M. RNA Biol. 2010 Sep-Oct;7(5):551-8). The CRISPR-Cas system may be harnessed to correct these defects of genomic instability.

A further aspect of the disclosure relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas sytem and the underlying genetics is further described in Genetics of Epilepsy and Genetic Epilepsies, edited by Giuliano Avanzini, Jeffrey L. Noebels, Mariani Foundation Paediatric Neurology:20; 2009).

In yet another aspect, the CRISPR-Cas system may beCl used to correct ocular defects that arise from several genetic mutations further described in Genetic Diseases of the Eye, Second Edition, edited by Elias I. Traboulsi, Oxford University Press, 2012.

Several further aspects relate to correcting defects associated with a wide range of genetic diseases which are further described on the website of the National Institutes of Health under the topic subsection Genetic Disorders. The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN asn so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcer1g gene, for example.

Examples of cardiovascular diseases associated proteins may include IL1B (interleukin 1, beta), XDH (xanthine dehydrogenase), TP53 (tumor protein p53), PTGIS (prostaglandin 12 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated with Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated with Macular Degeneration may include the ATP-binding cassette, sub-family A (ABC 1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated with Schizophrenia may include NRG1, ErbB4, CPLX1, TPH1, TPH2, NRXN1, GSK3A, BDNF, DISC1, GSK3B, and combinations thereof

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-1D adrenergic receptor for Alpha-1D adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type V1-Related Disorders; Usher Syndrome Type I; Congenital Muscular Dystrophy; Wolf-Hirschhorn Syndrome; Lysosomal Acid Lipase Deficiency; and Xeroderma Pigmentosum.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity (Fangri S, et al. ("Rationally engineered therapeutic proteins with reduced immunogenicity" J Immunol. 2005 Mar 15;174(6):3187-96.) The immunogenicity of the CRISPR enzyme in particular may be reduced following the approach first set out in Tangri et al with respect to erythropoietin and subsequently developed. Accordingly, directed evolution or rational design may be used to reduce the immunogenicity of the CRISPR enzyme (for instance a Cas9) in the host species (human or other species).

In plants, pathogens are often host-specific. For example, *Fusarium oxysporum* f. sp. *lycopersici* causes tomato wilt but attacks only tomato, and *F. oxysporum f. dianthii Puccinia graminis* f sp. *tritici* attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present disclosure, plant breeders are provided with a new tool to induce mutations. Accordingly, one skilled in the art can analyze the genome of sources of resistance genes, and in Varieties having desired characteristics or traits employ the present invention to induce the rise of resistance genes, with more precision than previous mutagenic agents and hence accelerate and improve plant breeding programs.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention.

### Example 1: CRISPR Complex Activity in the Nucleus of a Eukaryotic Cell

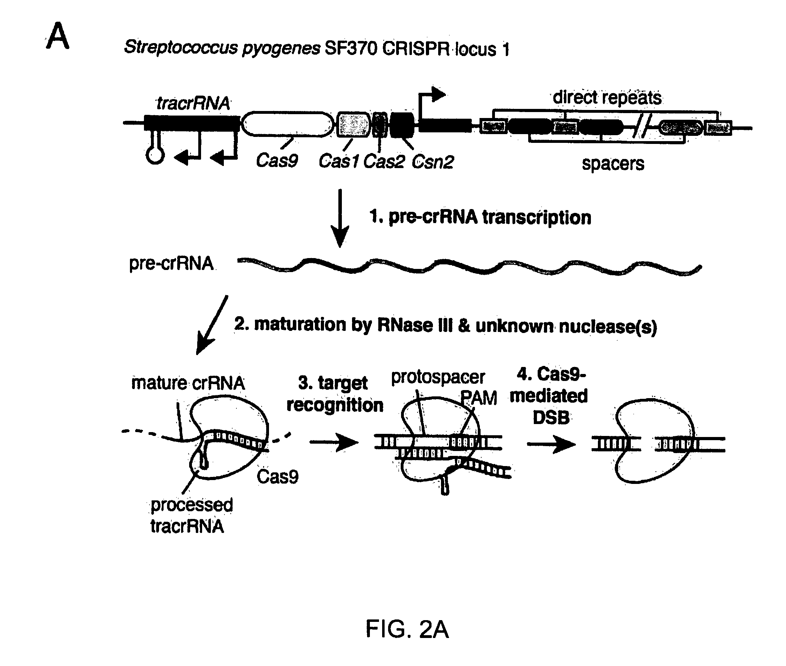

An example type II CRISPR system is the type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Figure 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Figure 2A). This example describes an example process for adapting this RNA-programmable nuclease system to direct CRISPR complex activity in the nuclei of eukaryotic cells.

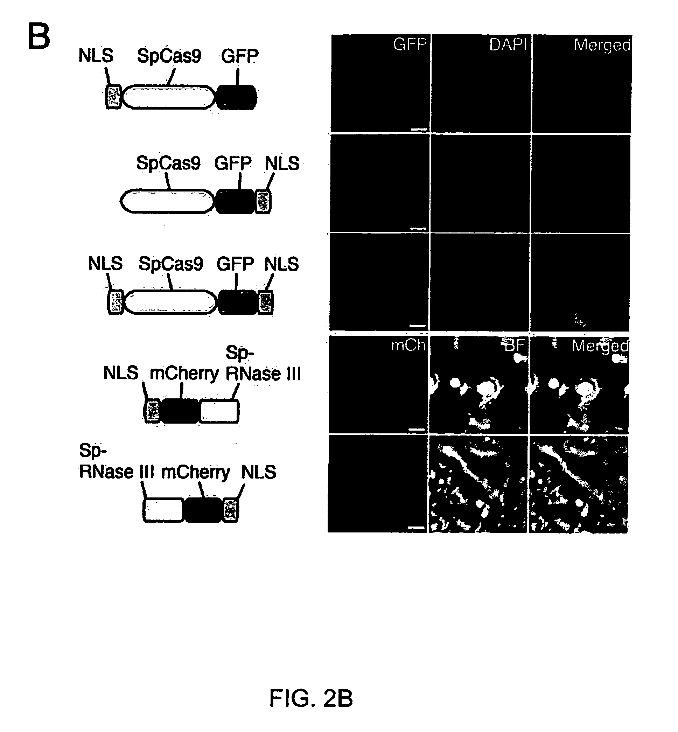

To improve expression of CRISPR components in mammalian cells, two genes from the SF370 locus 1 of *Streptococcus pyogenes* (*S. pyogenes*) were codon-optimized, *Cas9 (SpCas9)* and *RNase III* (*SpRNase III*). To facilitate nuclear localization, a nuclear localization signal (NLS) was included at the amino (N)- or carboxyl (C)-termini of both SpCas9 and SpRNase III (Figure 2B). To facilitate visualization of protein expression, a fluorescent protein marker was also included at the N- or C-termini of both proteins (Figure 2B). A version of SpCas9 with an NLS attached to both N- and C-termini (2xNLS-SpCas9) was also generated. Constructs containing NLS-fused SpCas9 and SpRNase III were transfected into 293FT human embryonic kidney (HEK) cells, and the relative positioning of the NLS to SpCas9 and SpRNase III was found to affect their nuclear localization efficiency. Whereas the C-terminal NLS was sufficient to target SpRNase III to the nucleus, attachment of a single copy of these particular NLS's to either the N- or C-terminus of SpCas9 was unable to achieve adequate nuclear localization in this system. In this example, the C-terminal NLS was that of nucleoplasmin (KRPAATKKAGQAKKKK), and the C-terminal NLS was that of the SV40 large T-antigen (PKKKRKV). Of the versions of SpCas9 tested, only 2xNLS-SpCas9 exhibited nuclear localization (Figure 2B).

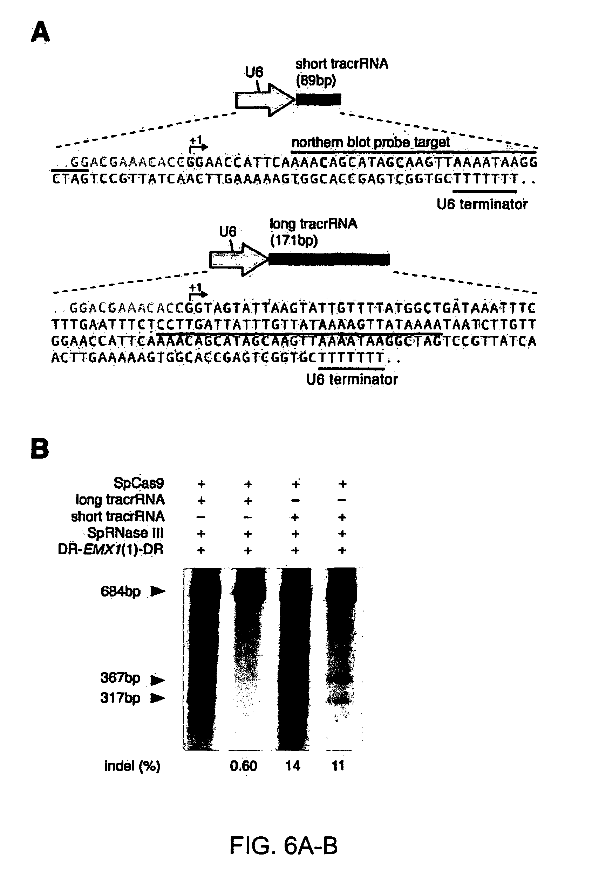

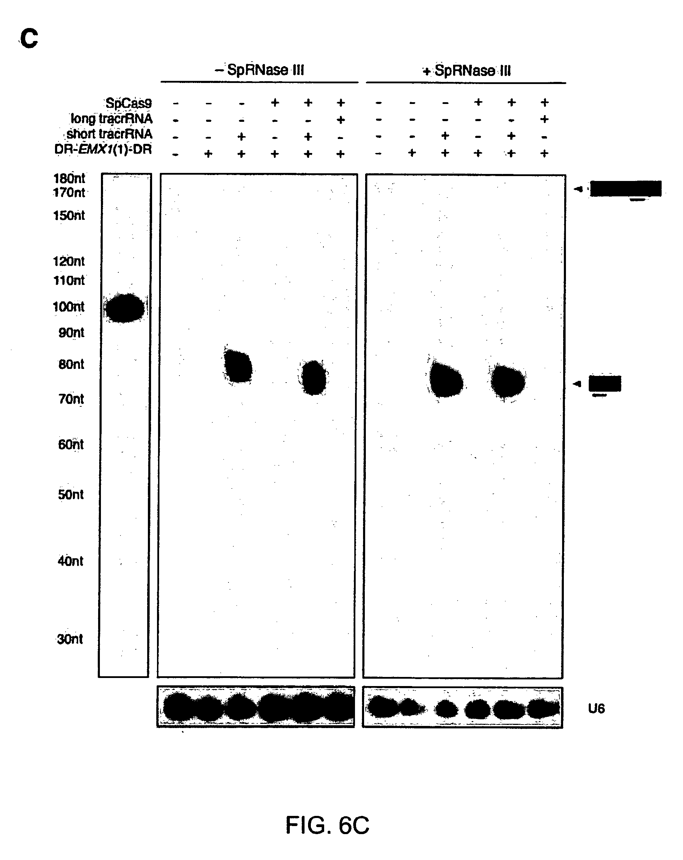

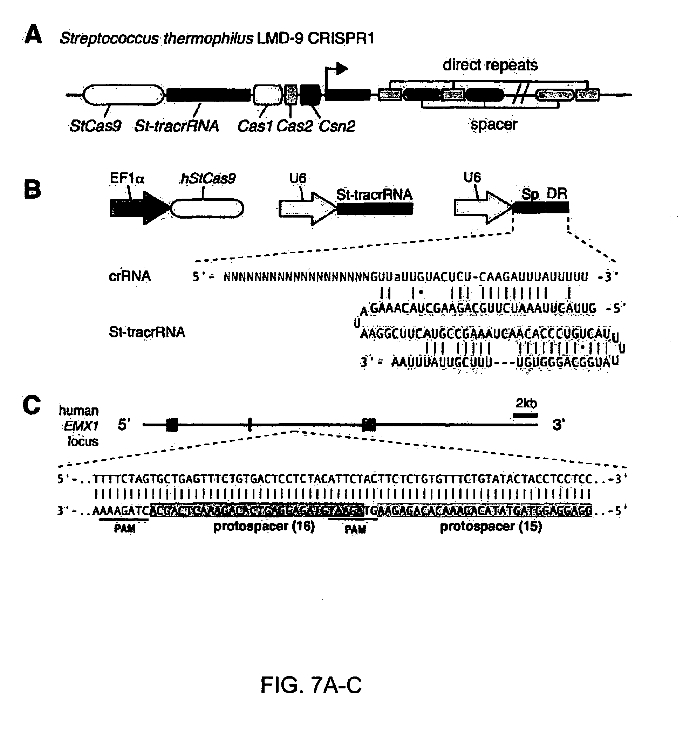

The tracrRNA from the CRISPR locus of *S. pyogenes* SF370 has two transcriptional start sites, giving rise to two transcripts of 89-nucleotides (nt) and 171 nt that are subsequently processed into identical 75nt mature tracrRNAs. The shorter 89nt tracrRNA was selected for expression in mammalian cells (expression constructs illustrated in Figure 6, with functionality as determined by results of Surveryor assay shown in Figure 6B). Transcription start sites are marked as +1, and transcription terminator and the sequence probed by northern blot are also indicated. Expression of processed tracrRNA was also confirmed by Northern blot. Figure 7C shows results of a Northern blot analysis of total RNA extracted from 293FT cells transfected with U6 expression constructs carrying long or short tracrRNA, as well as SpCas9 and DR-EMX1(1)-DR. Left and right panels are from 293FT cells transfected without or with SpRNase III, respectively. U6 indicate loading control blotted with a probe targeting human U6 snRNA. Transfection of the short tracrRNA expression construct led to abundant levels of the processed form of tracrRNA (~75bp). Very low amounts of long tracrRNA are detected on the Northern blot.

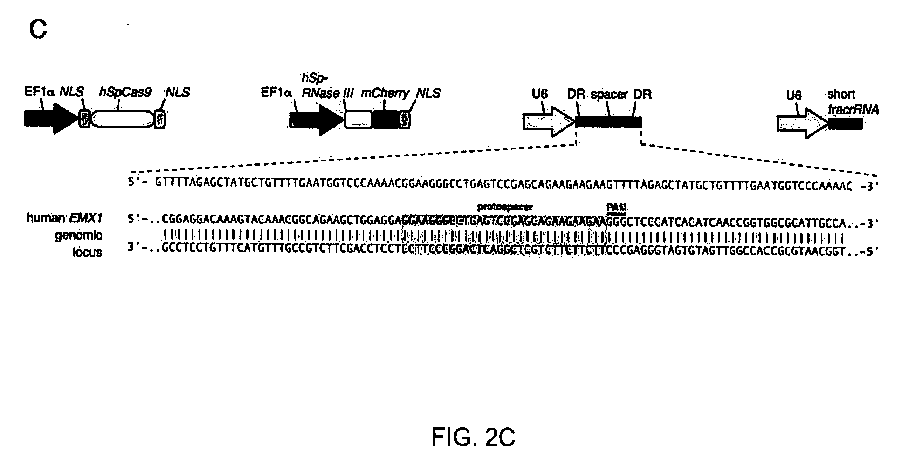

To promote precise transcriptional initiation, the RNA polymerase III-based U6 promoter was selected to drive the expression of tracrRNA (Figure 2C). Similarly, a U6 promoter-based construct was developed to express a pre-crRNA array consisting of a single spacer flanked by two direct repeats (DRs, also encompassed by the term "tracr-mate sequences"; Figure 2C). The initial spacer was designed to target a 33-base-pair (bp) target site (30-bp protospacer plus a 3-bp CRISPR motif (PAM) sequence satisfying the NGG recognition motif of Cas9) in the human *EMX1* locus (Figure 2C), a key gene in the development of the cerebral cortex.

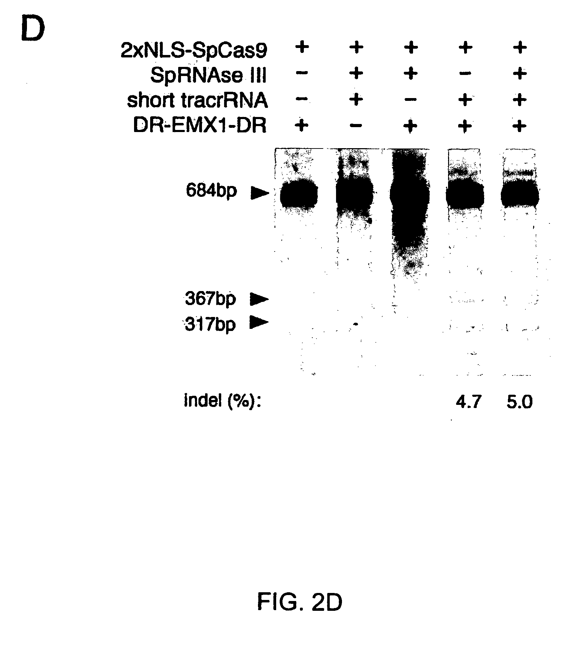

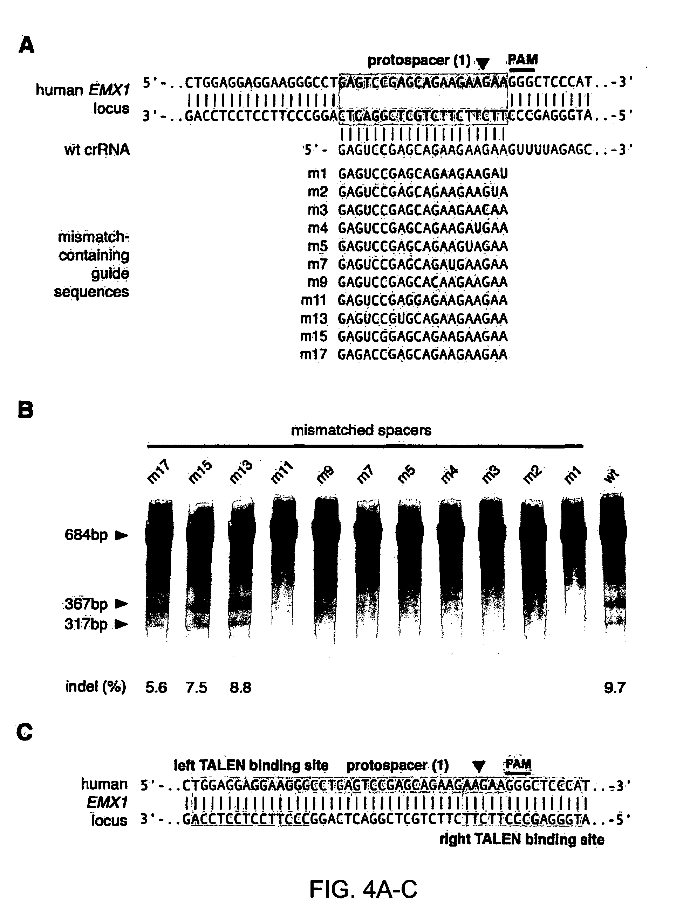

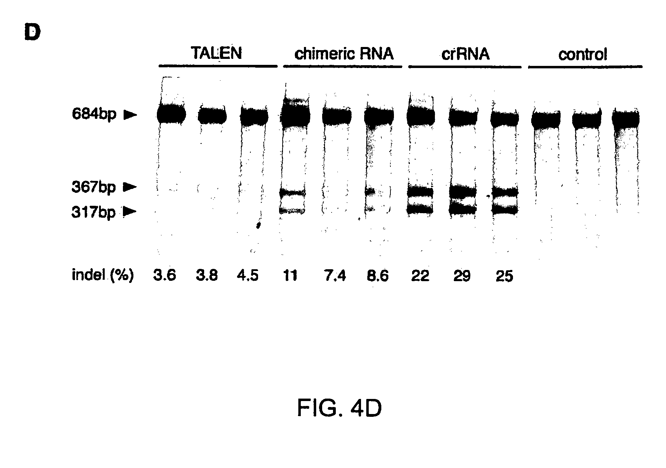

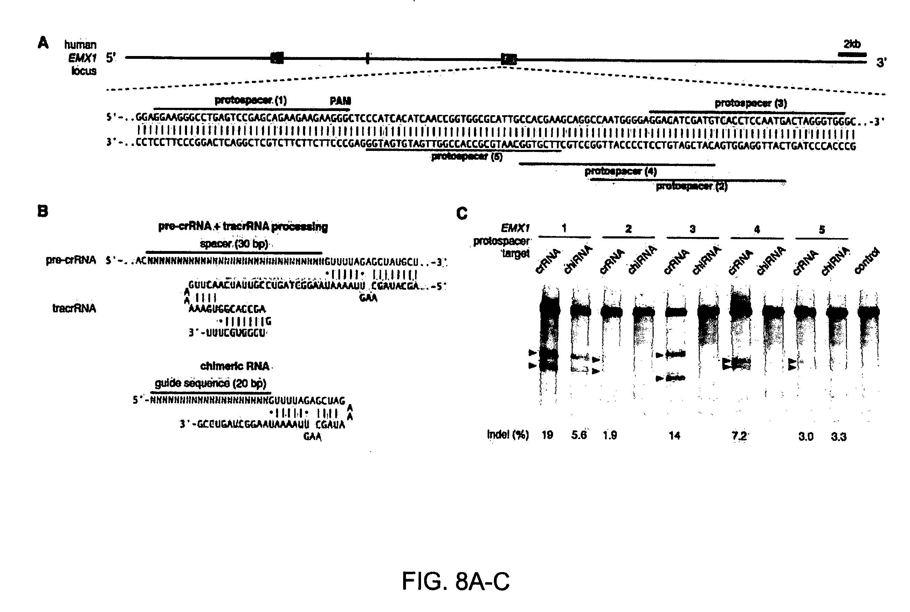

To test whether heterologous expression of the CRISPR system (SpCas9, SpRNase III, tracrRNA, and pre-crRNA) in mammalian cells can achieve targeted cleavage of mammalian chromosomes, HEK 293FT cells were transfected with combinations of CRISPR components. Since DSBs in mammalian nuclei are partially repaired by the non-homologous end joining (NHEJ) pathway, which leads to the formation of indels, the Surveyor assay was used to detect potential cleavage activity at the target *EMX1* locus (see e.g. Guschin *et al.,* 2010, Methods Mol Biol 649: 247). Co-transfection of all four CRISPR components was able to induce up to 5.0% cleavage in the protospacer (see Figure 2D). Co-transfection of all CRISPR components minus SpRNase III also induced up to 4.7% indel in the protospacer, suggesting that there may be endogenous mammalian RNases that are capable of assisting with crRNA maturation, such as for example the related Dicer and Drosha enzymes. Removing any of the remaining three components abolished the genome cleavage activity of the CRISPR system (Figure 2D). Sanger sequencing of amplicons containing the target locus verified the cleavage activity: in 43 sequenced clones, 5 mutated alleles (11.6%) were found. Similar experiments using a variety of guide sequences produced indel percentages as high as 29% (see Figures 4-8, 10 and 11). These results define a three-component system for efficient CRISPR-mediated genome modification in mammalian cells.

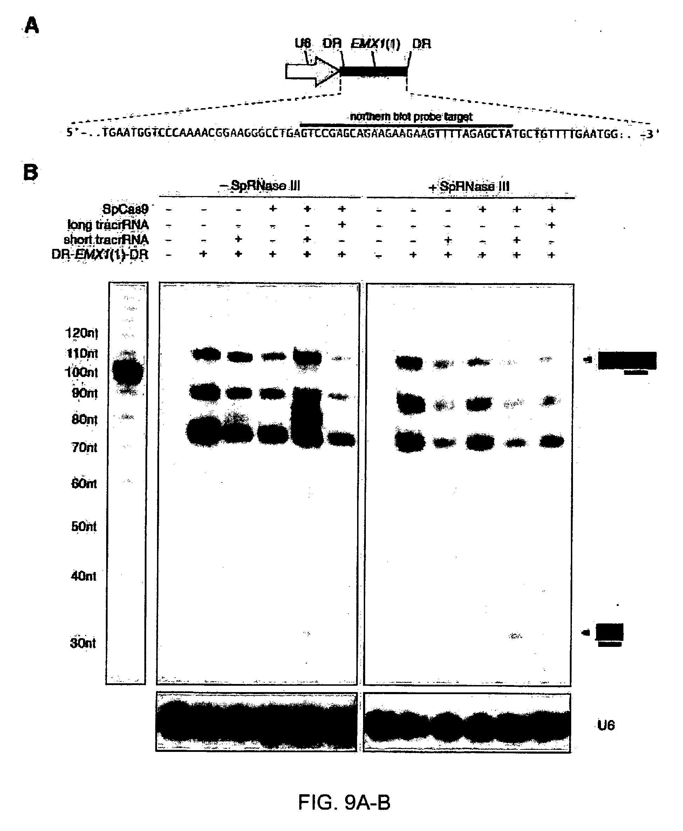

To optimize the cleavage efficiency, Applicants also tested whether different isoforms of tracrRNA affected the cleavage efficiency and found that, in this example system, only the short (89-bp) transcript form was able to mediate cleavage of the human *EMX1* genomic locus. Figure 9 provides an additional Northern blot analysis of crRNA processing in mammalian cells. Figure 9A illustrates a schematic showing the expression vector for a single spacer flanked by two direct repeats (DR-EMX1(1)-DR). The 30bp spacer targeting the human EMX1 locus protospacer 1 and the direct repeat sequences are shown in the sequence beneath Figure 9A. The line indicates the region whose reverse-complement sequence was used to generate Northern blot probes for EMX1(1) crRNA detection. Figure 9B shows a Northern blot analysis of total RNA extracted from 293FT cells transfected with U6 expression constructs carrying DR-EMX1(1)-DR. Left and right panels are from 293FT cells transfected without or with SpRNase III respectively. DR-EMX1(1)-DR was processed into mature crRNAs only in the presence of SpCas9 and short tracrRNA and was not dependent on the presence of SpRNase III. The mature crRNA detected from transfected 293FT total RNA is ~33bp and is shorter than the 39-42bp mature crRNA from *S. pyogenes.* These results demonstrate that a CRISPR system can be transplanted into eukaryotic cells and reprogrammed to facilitate cleavage of endogenous mammalian target polynucleotides.

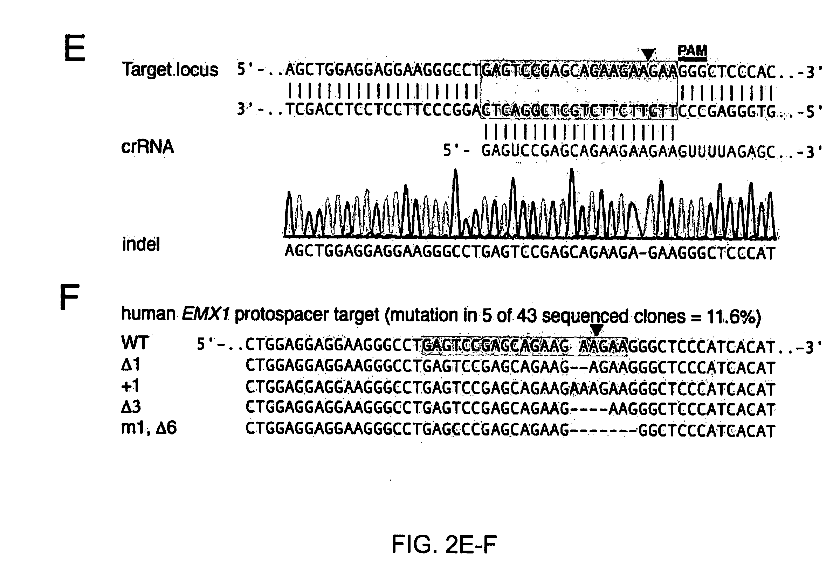

Figure 2 illustrates the bacterial CRISPR system described in this example. Figure 2A illustrates *a Schematic* showing the CRISPR locus 1 from *Streptococcus pyogenes* SF370 and a proposed mechanism of CRISPR-mediated DNA cleavage by this system. Mature crRNA processed from the direct repeat-spacer array directs Cas9 to genomic targets consisting of complimentary protospacers and a protospacer-adjacent motif (PAM). Upon target-spacer base pairing, Cas9 mediates a double-strand break in the target DNA. Figure 2B illustrates engineering of *S. pyogenes* Cas9 (SpCas9) and RNase III (SpRNase III) with nuclear localization signals (NLSs) to enable import into the mammalian nucleus. Figure 2C illustrates mammalian expression of SpCas9 and SpRNase III driven by the constitutive EF1a promoter and tracrRNA and pre-crRNA array (DR-Spacer-DR) driven by the RNA Pol3 promoter U6 to promote precise transcription initiation and termination. A protospacer from the human *EMX1* locus with a satisfactory PAM sequence is used as the spacer in the pre-crRNA array. Figure 2D illustrates surveyor nuclease assay for SpCas9-mediated minor insertions and deletions. SpCas9 was expressed with and without SpRNase III, tracrRNA, and a pre-crRNA array carrying the *EMX1 -* target spacer. Figure 2E illustrates a schematic representation of base pairing between target locus and *EMX1*-targeting crRNA, as well as an example chromatogram showing a micro deletion adjacent to the SpCas9 cleavage site. Figure 2F illustrates mutated alleles identified from sequencing analysis of 43 clonal amplicons showing a variety of micro insertions and deletions. Dashes indicate deleted bases, and non-aligned or mismatched bases indicate insertions or mutations. Scale bar = 10µm.

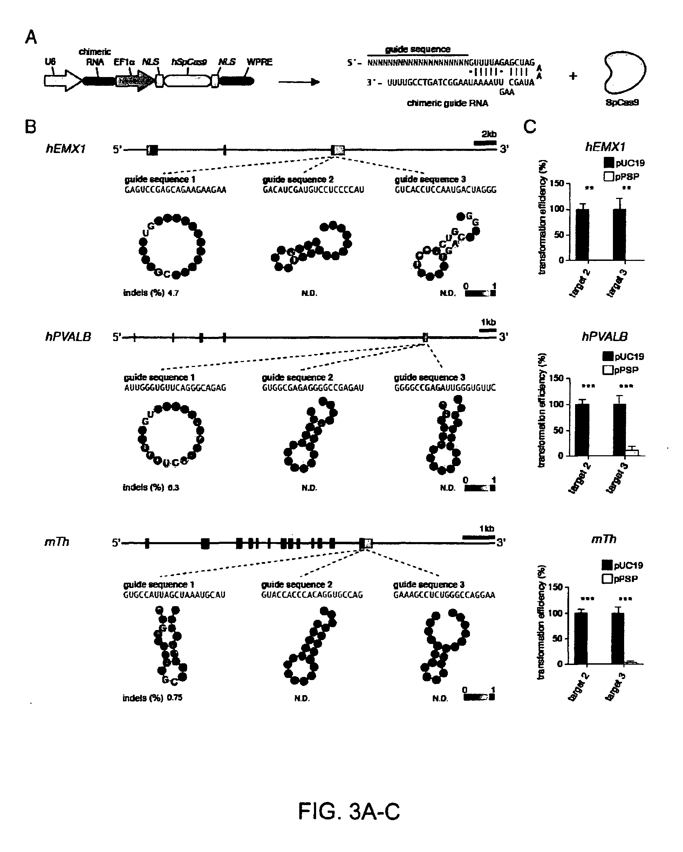

To further simplify the three-component system, a chimeric crRNA-tracrRNA hybrid design was adapted, where a mature crRNA (comprising a guide sequence) is fused to a partial tracrRNA via a stem-loop to mimic the natural crRNA:tracrRNA duplex (Figure 3A).

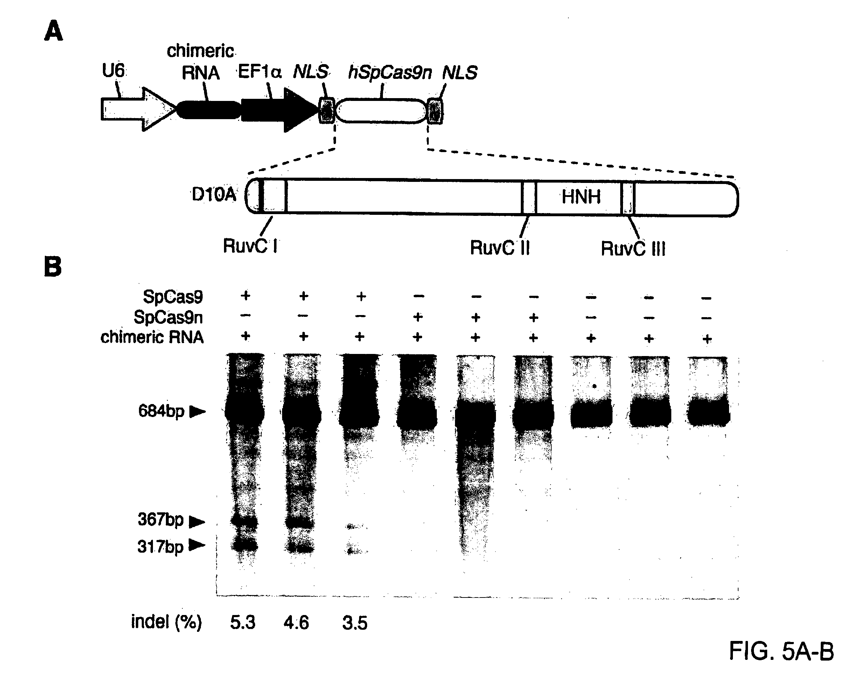

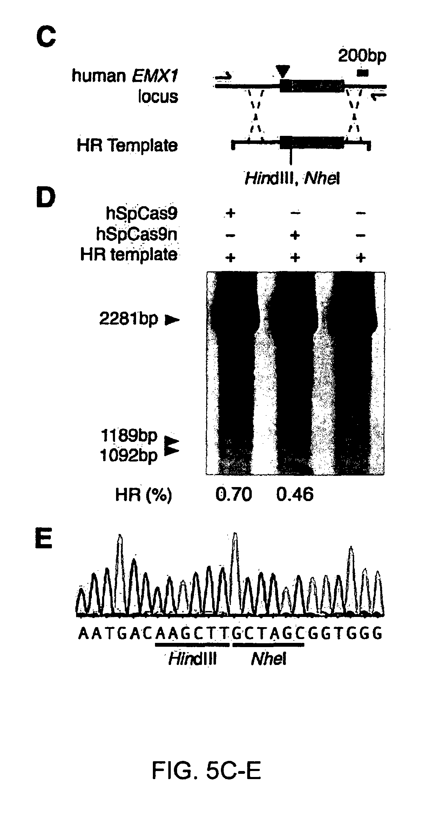

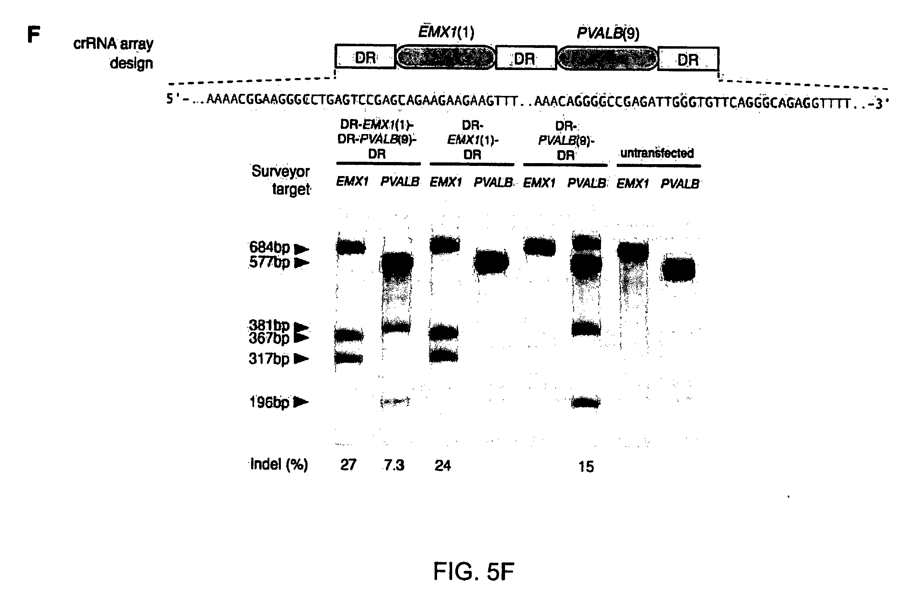

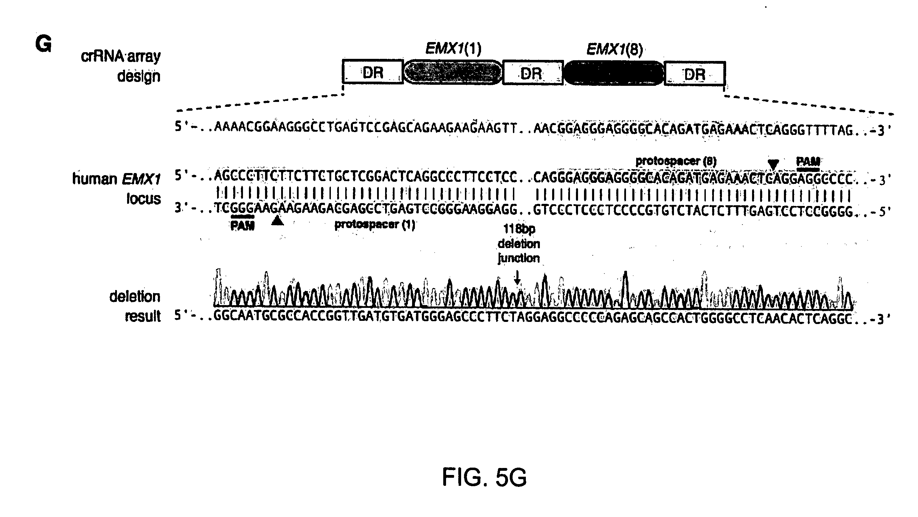

Guide sequences can be inserted between BbsI sites using annealed oligonucleotides. Protospacers on the sense and anti-sense strands are indicated above and below the DNA sequences, respectively. A modification rate of 6.3% and 0.75% was achieved for the human *PVALB* and mouse *Th* loci respectively, demonstrating the broad applicability of the CRISPR system in modifying different loci across multiple organisms While cleavage was only detected with one out of three spacers for each locus using the chimeric constructs, all target sequences were cleaved with efficiency of indel production reaching 27% when using the co-expressed pre-crRNA arrangement (Figures 4 and 5).

Figure 5 provides a further illustration that SpCas9 can be reprogrammed to target multiple genomic loci in mammalian cells. Figure 5A provides a schematic of the human *EMX1* locus showing the location of five protospacers, indicated by the underlined sequences. Figure 5B provides a schematic of the pre-crRNA/trcrRNA complex showing hybridization between the direct repeat region of the pre-crRNA and tracrRNA (top), and a schematic of a chimeric RNA design comprising a 20bp guide sequence, and tracr mate and tracr sequences consisting of partial direct repeat and tracrRNA sequences hybridized in a hairpin structure (bottom). Results of a Surveyor assay comparing the efficacy of Cas9-mediated cleavage at five protospacers in the human *EMX1* locus is illustrated in Figure 5C. Each protospacer is targeted using either processed pre-crRNA/tracrRNA complex (crRNA) or chimeric RNA (chiRNA).

Since the secondary structure of RNA can be crucial for intermolecular interactions, a structure prediction algorithm based on minimum free energy and Boltzmann-weighted structure ensemble was used to compare the putative secondary structure of all guide sequences used in our genome targeting experiment (Figure 3B) (see e.g. Gruber et al., 2008, Nucleic Acids Research, 36: W70). Analysis revealed that in most cases, the effective guide sequences in the chimeric crRNA context were substantially free of secondary structure motifs, whereas the ineffective guide sequences were more likely to form internal secondary structures that could prevent base pairing with the target protospacer DNA. It is thus possible that variability in the spacer secondary structure might impact the efficiency of CRISPR-mediated interference when using a chimeric crRNA.

Figure 3 illustrates example expression vectors. Figure 3A provides a schematic of a bi-cistronic vector for driving the expression of a synthetic crRNA-tracrRNA chimera (chimeric RNA) as well as SpCas9. The chimeric guide RNA contains a 20-bp guide sequence corresponding to the protospacer in the genomic target site. Figure 3B provides a schematic showing guide sequences targeting the human *EMX1, PVALB,* and mouse *Th* loci, as well as their predicted secondary structures. The modification efficiency at each target site is indicated below the RNA secondary structure drawing (*EMX1, n* = 216 amplicon sequencing reads; *PVALB, n* = 224 reads; *Th, n* = 265 reads). The folding algorithm produced an output with each base colored according to its probability of assuming the predicted secondary structure, as indicated by a rainbow scale that is reproduced in Figure 3B in gray scale. Further vector designs for SpCas9 are shown in Figure 3A, including single expression vectors incorporating a U6 promoter linked to an insertion site for a guide oligo, and a Cbh promoter linked to SpCas9 coding sequence.

To test whether spacers containing secondary structures are able to function in prokaryotic cells where CRISPRs naturally operate, transformation interference of protospacer-bearing plasmids were tested in an *E. coli* strain heterologously expressing the *S. pyogenes* SF370 CRISPR locus 1 (Figure 3C). The CRISPR locus was cloned into a low-copy *E. coli* expression vector and the crRNA array was replaced with a single spacer flanked by a pair of DRs (pCRISPR). *E. coli* strains harboring different pCRISPR plasmids were transformed with challenge plasmids containing the corresponding protospacer and PAM sequences (Figure 3C). In the bacterial assay, all spacers facilitated efficient CRISPR interference (Figure 3C). These results suggest that there may be additional factors affecting the efficiency of CRISPR activity in mammalian cells.

To investigate the specificity of CRISPR-mediated cleavage, the effect of single-nucleotide mutations in the guide sequence on protospacer cleavage in the mammalian genome was analyzed using a series of *EMX1*-targeting chimeric crRNAs with single point mutations (Figure 4A). Figure 4B illustrates results of a Surveyor nuclease assay comparing the cleavage efficiency of Cas9 when paired with different mutant chimeric RNAs. Single-base mismatch up to 12-bp 5' of the PAM substantially abrogated genomic cleavage by SpCas9, whereas spacers with mutations at farther upstream positions retained activity against the original protospacer target (Figure 4B). In addition to the PAM, SpCas9 has single-base specificity within the last 12-bp of the spacer. Furthermore, CRISPR is able to mediate genomic cleavage as efficiently as a pair of TALE nucleases (TALEN) targeting the same *EMX1* protospacer. Figure 4C provides a schematic showing the design of TALENs targeting *EMX1*, and Figure 4D shows a Surveyor gel comparing the efficiency of TALEN and Cas9 (n=3).

Having established a set of components for achieving CRISPR-mediated gene editing in mammalian cells through the error-prone NHEJ mechanism, the ability of CRISPR to stimulate homologous recombination (HR), a high fidelity gene repair pathway for making precise edits in the genome, was tested. The wild type SpCas9 is able to mediate site-specific DSBs, which can be repaired through both NHEJ and HR. In addition, an aspartate-to-alanine substitution (D10A) in the RuvC I catalytic domain of SpCas9 was engineered to convert the nuclease into a nickase (SpCas9n; illustrated in Figure 5A) (see e.g. Sapranausaks et al., 2011, Cucleic Acis Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the *EMX1* protospacer target. As illustrated in Figure 5B, co-expression of *EMX1*-targeting chimeric crRNA with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting *EMX1*, hSpCas9 or hSpCas9n, as well as a HR template to introduce a pair of restriction sites (*Hin*dIII and *Nhe*I) near the protospacer. Figure 5C provides a schematic illustration of the HR strategy, with relative locations of recombination points and primer annealing sequences (arrows). SpCas9 and SpCas9n indeed catalyzed integration of the HR template into the *EMX1* locus. PCR amplification of the target region followed by restriction digest with *Hin*dIII revealed cleavage products corresponding to expected fragment sizes (arrows in restriction fragment length polymorphism gel analysis shown in Figure 5D), with SpCas9 and SpCas9n mediating similar levels of HR efficiencies. Applicants further verified HR using Sanger sequencing of genomic amplicons (Figure 5E). These results demonstrate the utility of CR1SPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway.

Expression constructs mimicking the natural architecture of CRISPR loci with arrayed spacers (Figure 2A) were constructed to test the possibility of multiplexed sequence targeting. Using a single CRISPR array encoding a pair of *EMX1* - and *PVALB*-targeting spacers, efficient cleavage at both loci was detected (Figure 4F, showing both a schematic design of the crRNA array and a Surveyor blot showing efficient mediation of cleavage). Targeted deletion of larger genomic regions through concurrent DSBs using spacers against two targets within *EMX1* spaced by 119bp was also tested, and a 1.6% deletion efficacy (3 out of 182 amplicons; Figure 5G) was detected. This demonstrates that the CRISPR system can mediate multiplexed editing within a single genome.

### Example 2: CRISPR system modifications and alternatives

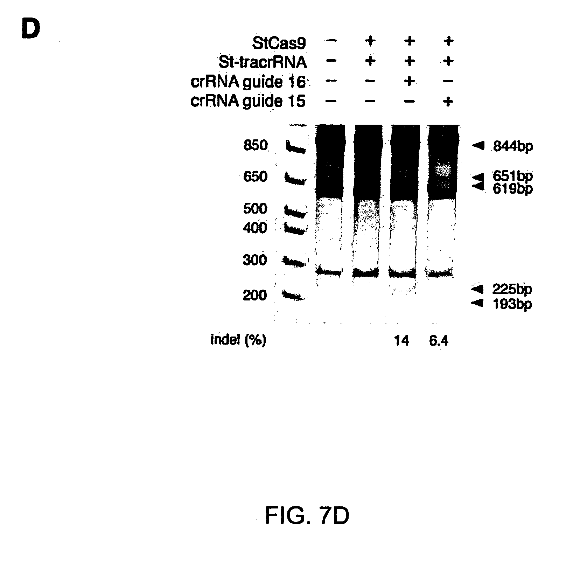

The ability to use RNA to program sequence-specific DNA cleavage defines a new class of genome engineering tools for a variety of research and industrial applications. Several aspects of the CRISPR system can be further improved to increase the efficiency and versatility of CRISPR targeting. Optimal Cas9 activity may depend on the availability of free Mg²⁺ at levels higher than that present in the mammalian nucleus (see e.g. Jinek et al., 2012, Science, 337:816), and the preference for an NGG motif immediately downstream of the protospacer restricts the ability to target on average every 12-bp in the human genome. Some of these constraints can be overcome by exploring the diversity of CRISPR loci across the microbial metagenome (see e.g. Makarova et al., 2011, Nat Rev Microbiol, 9:467). Other CRISPR loci may be transplanted into the mammalian cellular milieu by a process similar to that described in Example 1. The modification efficiency at each target site is indicated below the RNA secondary structures. The algorithm generating the structures colors each base according to its probability of assuming the predicted secondary structure. RNA guide spacers 1 and 2 induced 14% and 6.4%, respectively. Statistical analysis of cleavage activity across biological replica at these two protospacer sites is also provided in Figure 7.

### Example 3: Sample target sequence selection algorithm

A software program is designed to identify candidate CRISPR target sequences on both strands of an input DNA sequence based on desired guide sequence length and a CRISPR motif sequence (PAM) for a specified CRISPR enzyme. For example, target sites for Cas9 from *S. pyogenes,* with PAM sequences NGG, may be identified by searching for 5'-Nₓ-NGG-3' both on the input sequence and on the reverse-complement of the input. Likewise, target sites for Cas9 of *S*. *thermophilus* CRISPR1, with PAM sequence NNAGAAW, may be identified by searching for 5'-Nₓ-NNAGAAW-3' both on the input sequence and on the reverse-complement of the input. Likewise, target sites for Cas9 of *S*. *thermophilus* CRISPR3, with PAM sequence NGGNG, may be identified by searching for 5'-Nₓ-NGGNG-3' both on the input sequence and on the reverse-complement of the input. The value "x" in Nₓ may be fixed by the program or specified by the user, such as 20.

Since multiple occurrences in the genome of the DNA target site may lead to nonspecific genome editing, after identifying all potential sites, the program filters out sequences based on the number of times they appear in the relevant reference genome. For those CRISPR enzymes for which sequence specificity is determined by a 'seed' sequence, such as the 11-12bp 5' from the PAM sequence, including the PAM sequence itself, the filtering step may be based on the seed sequence. Thus, to avoid editing at additional genomic loci, results are filtered based on the number of occurrences of the seed:PAM sequence in the relevant genome. The user may be allowed to choose the length of the seed sequence. The user may also be allowed to specify the number of occurrences of the seed:PAM sequence in a genome for purposes of passing the filter. The default is to screen for unique sequences. Filtration level is altered by changing both the length of the seed sequence and the number of occurrences of the sequence in the genome. The program may in addition or alternatively provide the sequence of a guide sequence complementary to the reported target sequence(s) by providing the reverse complement of the identified target sequence(s).

### Example 4: Evaluation of multiple chimeric crRNA-tracrRNA hybrids

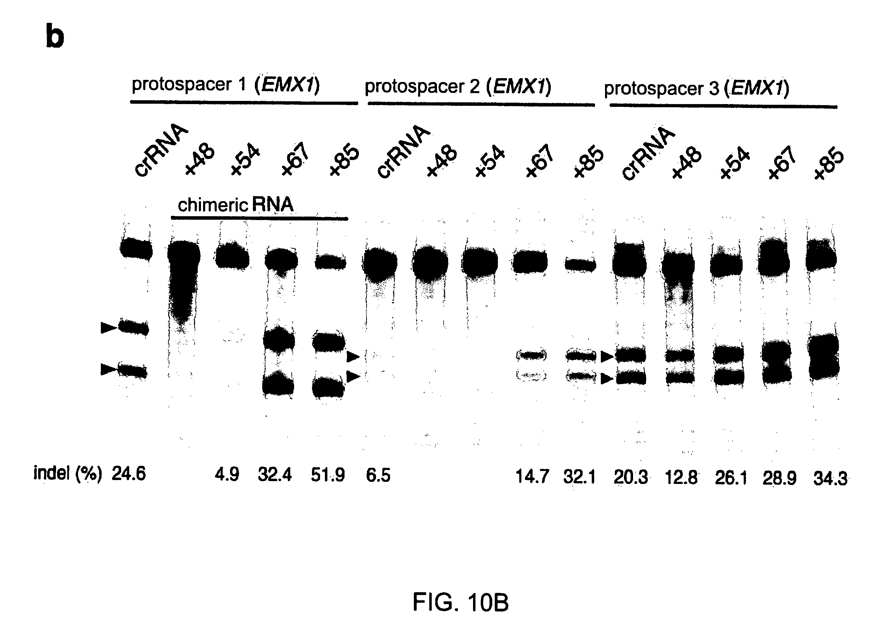

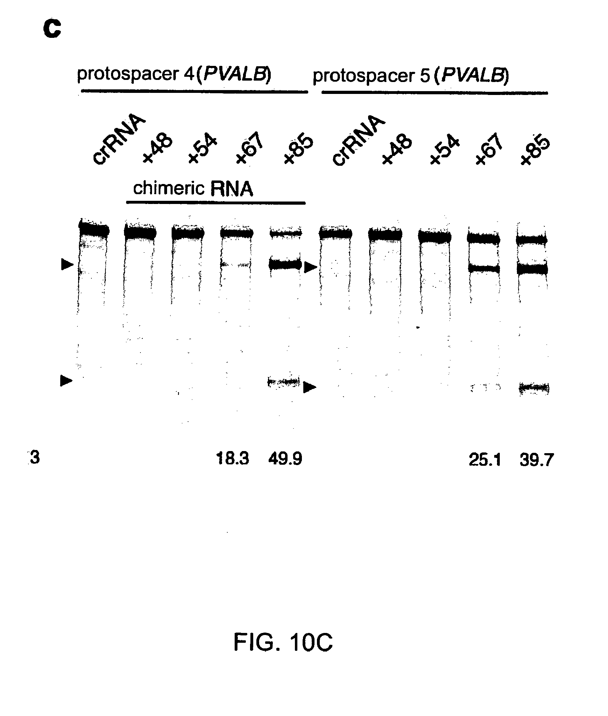

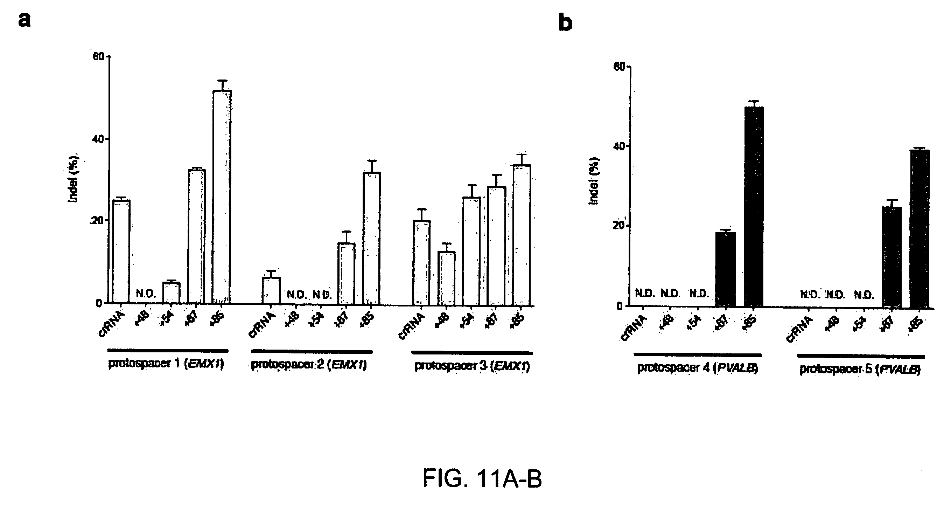

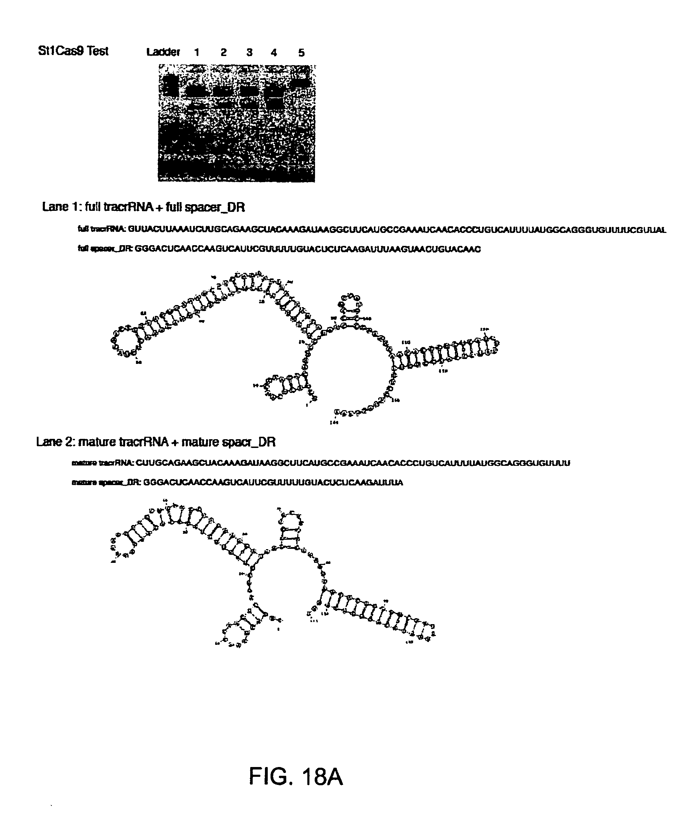

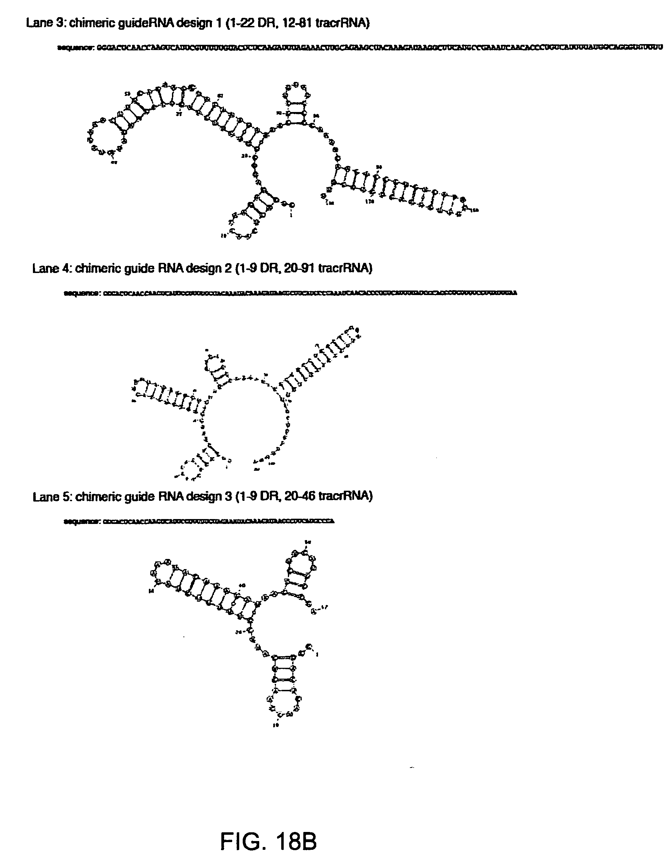

This example describes results obtained for chimeric RNAs (chiRNAs; comprising a guide sequence, a tracr mate sequence, and a tracr sequence in a single transcript) having tracr sequences that incorporate different lengths of wild-type tracrRNA sequence. Figure 18a illustrates a schematic of a bicistronic expression vector for chimeric RNA and Cas9. Cas9 is driven by the CBh promoter and the chimeric RNA is driven by a U6 promoter. The chimeric guide RNA consists of a 20bp guide sequence (Ns) joined to the tracr sequence (running from the first "U" of the lower strand to the end of the transcript), which is truncated at various positions as indicated. The guide and tracr sequences are separated by the tracr-mate sequence GUUUUAGAGCUA followed by the loop sequence GAAA. Results of SURVEYOR assays for Cas9-mediated indels at the human *EMX1* and *PVALB* loci are illustrated in Figure 18b and 18c, respectively. Arrows indicate the expected SURVEYOR fragments. ChiRNAs are indicated by their "+n" designation, and crRNA refers to a hybrid RNA where guide and tracr sequences are expressed as separate transcripts. Quantification of these results, performed in triplicate, are illustrated by histogram in Figures 11a and 11b, corresponding to Figures 10b and 10c, respectively ("N.D." indicates no indels detected). Protospacer IDs and their corresponding genomic target, protospacer sequence, PAM sequence, and strand location are provided in Table D. Guide sequences were designed to be complementary to the entire protospacer sequence in the case of separate transcripts in the hybrid system, or only to the underlined portion in the case of chimeric RNAs.

**Table D:**

| **protospace r ID** | **genomic target** | **protospacer sequence (5' to 3')** | **PAM** | **Strand** |
|---|---|---|---|---|
| 1 | *EMX1* | | TGG | + |
| 2 | *EMX1* | | TGG | - |
| 3 | *EMX1* | | GGG | + |
| 4 | *PVALB* | | AGG | + |
| 5 | *PVALB* | | TGG | + |

### Cell culture and transfection

Human embryonic kidney (HEK) cell line 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100µg/mL streptomycin at 37°C with 5% CO₂ incubation. 293FT cells were seeded onto 24-well plates (Coming) 24 hours prior to transfection at a density of 150,000 cells per well. Cells were transfected using Lipofectamine 2000 (Life Technologies) following the manufacturer's recommended protocol. For each well of a 24-well plate, a total of 500ng plasmid was used.

### SURVEYOR assay for genome modification

293FT cells were transfected with plasmid DNA as described above. Cells were incubated at 37°C for 72 hours post-transfection prior to genomic DNA extraction. Genomic DNA was extracted using the QuickExtract DNA Extraction Solution (Epicentre) following the manufacturer's protocol. Briefly, pelleted cells were resuspended in QuickExtract solution and incubated at 65°C for 15 minutes and 98°C for 10 minutes. The genomic region flanking the CRISPR target site for each gene was PCR amplified (primers listed in Table E), and products were purified using QiaQuick Spin Column (Qiagen) following the manufacturer's protocol. 400ng total of the purified PCR products were mixed with 2µl 10X Taq DNA Polymerase PCR buffer (Enzymatics) and ultrapure water to a final volume of 20µl, and subjected to a re-annealing process to enable heteroduplex formation: 95°C for 10min, 95°C to 85°C ramping at-2°C/s, 85°C to 25°C at - 0.25°C/s, and 25°C hold for 1 minute. After re-annealing, products were treated with SURVEYOR nuclease and SURVEYOR enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities.

**Table E:**

| **primer name** | **genomic target** | **primer sequence (5' to 3')** |
|---|---|---|
| Sp-EMX1-F | *EMX1* | AAAACCACCCTTCTCTCTGGC |
| Sp-EMX1-R | *EMX1* | |
| Sp-PVALB-F | *PVALB* | CTGGAAAGCCAATGCCTGAC |
| Sp-PVALB-R | *PVALB* | GGCAGCAAACTCCTTGTCCT |

### Computational identification of unique CRISPR target sites

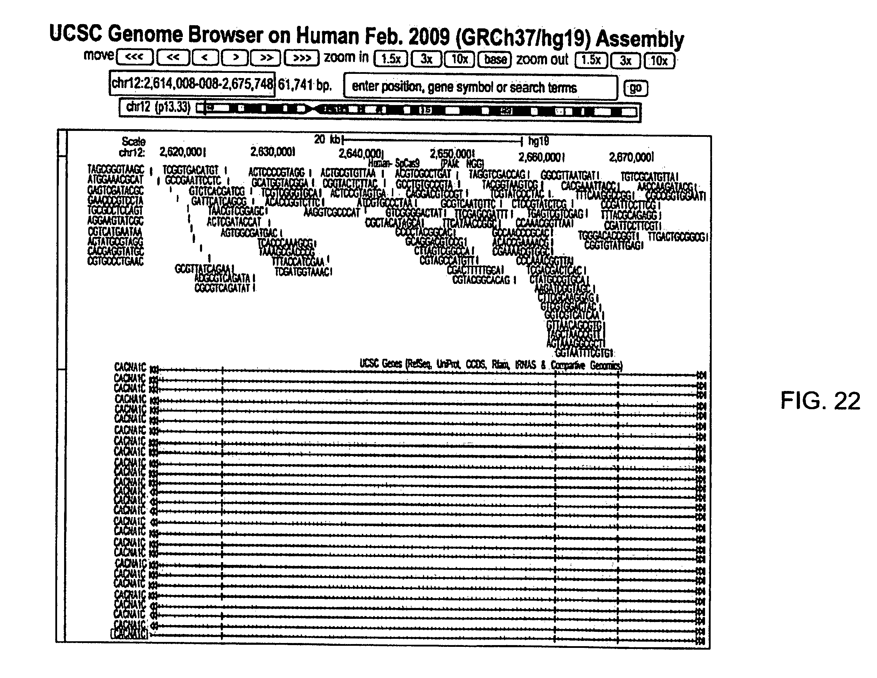

To identify unique target sites for the *S*. *pyogenes* SF370 Cas9 (SpCas9) enzyme in the human, mouse, rat, zebrafish, fruit fly, and *C*. *elegans* genome, we developed a software package to scan both strands of a DNA sequence and identify all possible SpCas9 target sites. For this example, each SpCas9 target site was operationally defined as a 20bp sequence followed by an NGG protospacer adjacent motif (PAM) sequence, and we identified all sequences satisfying this 5'-N₂₀-NGG-3' definition on all chromosomes. To prevent non-specific genome editing, after identifying all potential sites, all target sites were filtered based on the number of times they appear in the relevant reference genome. To take advantage of sequence specificity of Cas9 activity conferred by a 'seed' sequence, which can be, for example, approximately 11-12bp sequence 5' from the PAM sequence, 5'-NNNNNNNNNN-NGG-3' sequences were selected to be unique in the relevant genome. All genomic sequences were downloaded from the UCSC Genome Browser (Human genome hg19, Mouse genome mm9, Rat genome m5, Zebrafish genome danRer7, *D. melanogaster* genome dm4 and *C*. *elegans* genome ce10). The full search results are available to browse using UCSC Genome Browser information. An example visualization of some target sites in the human genome is provided in Figure 22.

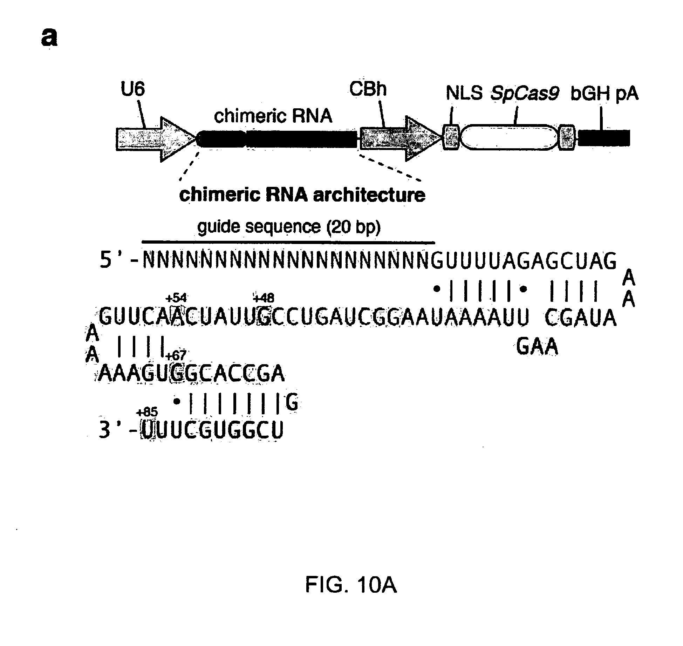

Initially, three sites within the *EMX1* locus in human HEK 293FT cells were targeted. Genome modification efficiency of each chiRNA was assessed using the SURVEYOR nuclease assay, which detects mutations resulting from DNA double-strand breaks (DSBs) and their subsequent repair by the non-homologous end joining (NHEJ) DNA damage repair pathway. Constructs designated chiRNA(+n) indicate that up to the +n nucleotide of wild-type tracrRNA is included in the chimeric RNA construct, with values of 48, 54, 67, and 85 used for n. Chimeric RNAs containing longer fragments of wild-type tracrRNA (chiRNA(+67) and chiRNA(+85)) mediated DNA cleavage at all three *EMX1* target sites, with chiRNA(+85) in particular demonstrating significantly higher levels of DNA cleavage than the corresponding crRNA/tracrRNA hybrids that expressed guide and tracr sequences in separate transcripts (Figures 10b and 10a). Two sites in the *PVALB* locus that yielded no detectable cleavage using the hybrid system (guide sequence and tracr sequence expressed as separate transcripts) were also targeted using chiRNAs. chiRNA(+67) and chiRNA(+85) were able to mediate significant cleavage at the two *PVALB* protospacers (Figures 10c and 10b).

For all five targets in the *EMX1* and *PVALB* loci, a consistent increase in genome modification efficiency with increasing tracr sequence length was observed. Without wishing to be bound by any theory, the secondary structure formed by the 3' end of the tracrRNA may play a role in enhancing the rate of CRISPR complex formation. An illustration of predicted secondary structures for each of the chimeric RNAs used in this example is provided in Figure 21. The secondary structure was predicted using RNAfold (http://rna.tbi.univie.ac.at/cgi-bin/RNAfold.cgi) using minimum free energy and partition function algorithm. Pseudocolor for each based (reproduced in grayscale) indicates the probability of pairing. Because chiRNAs with longer tracr sequences were able to cleave targets that were not cleaved by native CRISPR crRNA/tracrRNA hybrids, it is possible that chimeric RNA may be loaded onto Cas9 more efficiently than its native hybrid counterpart. To facilitate the application of Cas9 for site-specific genome editing in eukaryotic cells and organisms, all predicted unique target sites for the *S*. *pyogenes* Cas9 were computationally identified in the human, mouse, rat, zebra fish, *C*. *elegans,* and *D. melanogaster* genomes. Chimeric RNAs can be designed for Cas9 enzymes from other microbes to expand the target space of CRISPR RNA-programmable nucleases.

Figures 11 and 21 illustrate exemplary bicistronic expression vectors for expression of chimeric RNA including up to the +85 nucleotide of wild-type tracr RNA sequence, and SpCas9 with nuclear localization sequences. SpCas9 is expressed from a CBh promoter and terminated with the bGH polyA signal (bGH pA). The expanded sequence illustrated immediately below the schematic corresponds to the region surrounding the guide sequence insertion site, and includes, from 5' to 3', 3'-portion of the U6 promoter (first shaded region), BbsI cleavage sites (arrows), partial direct repeat (tracr mate sequence GTTTTAGAGCTA, underlined), loop sequence GAAA, and +85 tracr sequence (underlined sequence following loop sequence). An exemplary guide sequence insert is illustrated below the guide sequence insertion site, with nucleotides of the guide sequence for a selected target represented by an "N".

Sequences described in the above examples are as follows (polynucleotide sequences are 5' to 3'):
U6-short tracrRNA (*Streptococcus pyogenes* SF370):
U6-long tracrRNA (*Streptococcus pyogenes* SF370):
U6-DR-BbsI backbone-DR (*Streptococcus pyogenes* SF370):
U6-chimeric RNA-BbsI backbone (Streptococcus pyogenes SF370)
NLS-SpCas9-EGFP:
SpCas9-EGFP-NLS:
NLS-SpCas9-EGFP-NLS:
NLS-SpCas9-NLS:
NLS-mCherry-SpRNase3:
SpRNase3-mCherry-NLS:
NLS-SpCas9n-NLS (the D10A nickase mutation is lowercase):
hEMX1-HR Template-HindII-NheI:
NLS-StCsn1-NLS:
U6-St_tracrRNA(7-97):
U6-DR-spacer-DR (*S*. *pyogenes* SF370) (lowercase underline = direct repeat; N = guide sequence; bold = terminator)
Chimeric RNA containing +48 tracr RNA *(S. pyogenes* SF370)
   gagggcctatttcccatgattccttcatatttgcatatacgatacaaggctgttagagagataattggaattaatttgactgtaaacacaaagatat tagtacaaaatacgtgacgtagaaagtaataatttcttgggtagtttgcagttttaaaattatgttttaaaatggactatcatatgcttaccgtaactt gaaagtatttcgafftettggctttatatatcttgtggaaaggacgaaacaccNNNNNNNNNNNNNNNNNNNNgttttaga gctagaaatagcaagttaaaataaggctagtccg**TTTTTTT** (N = guide sequence; first underline = tracr mate sequence; second underline = tracr sequence; bold = terminator)
Chimeric RNA containing +54 tracr RNA (*S*. *pyogenes* SF370)
   gagggcctatttcccatgattccttcatatttgcatatacgatacaaggctgttagagagataattggaattaatttgactgtaaacacaaagatat tagtacaaaatacgtgacgtagaaagtaataatttcttgggtagtttgcagttttaaaattatgttttaaaatggactatcatatgcttaccgtaactt gaaagtatttcgatttcttggctttatatatcttgtggaaaggacgaaacaccNNNNNNNNNNNNNNNNNNNNgttttaga gctagaaatagcaagttaaaataaggctagtccgttatca**TTTTTTTT** (N = guide sequence; first underline = tracr mate sequence; second underline = tracr sequence; bold = terminator)
Chimeric RNA containing +67 tracr RNA *(S. pyogenes* SF370)
   gagggcctatttcccatgattccttcatatttgcatatacgatacaaggctgttagagagataattggaattaatttgactgtaaacacaaagatat tagtacaaaatacgtgacgtagaaagtaataatttcttgggtagtttgcagttttaaaattatgttttaaaatggactatcatatgcttaccgtaactt gaaagtatttcgatttcttggctttatatatcttgtggaaaggacgaaacaccNNNNNNNNNNNNNNNNNNNNgttttaga gctagaaatagcaagttaaaataaggctagtccgttatcaacttgaaaaagtg**TTTTTTT** (N = guide sequence; first underline = tracr mate sequence; second underline = tracr sequence; bold = terminator)
Chimeric RNA containing +85 tracr RNA (*S. pyogenes* SF370)
   gagggcctatttcccatgattccttcatatttgcatatacgatacaaggctgttagagagataattggaattaatttgactgtaaacacaaagatat tagtacaaaatacgtgacgtagaaagtaataatttcttgggtagtttgcagttttaaaattatgttttaaaatggactatcatatgcttaccgtaactt gaaagtatttcgatttcttggctttatatatcttgtggaaaggacgaaacaccNNNNNNNNNNNNNNNNNNNNgttttaga gctagaaatagcaagttaaaataaggctagtccgttatcaacttgaaaaagtggcaccgagtcggtgc**TTTTTTT** (N = guide sequence; first underline = tracr mate sequence; second underline = tracr sequence; bold = terminator)
CBh-NLS-SpCas9-NLS
Example chimeric RNA for *S*. *thermophilus* LMD-9 CRISPR1 Cas9 (with PAM of NNAGAAW)
   NNNNNNNNNNNNNNNNNNNNgtttttgtactctcaagatttaGAAAtaaatcttgcagaagctacaaagataaggctt catgccgaaatcaacaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgtttttgtactctcaGAAAtgcagaagctacaaagataaggcttcatgccgaaatca acaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgtttttgtactctcaGAAAtgcagaagctacaaagataaggcttcatgccgaaatca acaccctgtcattttatggcagggtgt**TTTTTT** (N = guide sequence; first underline = tracr mate sequence; second underline = tracr sequence; bold = terminator)
Example chimeric RNA for S. *thermophilus* LMD-9 CRISPR1 Cas9 (with PAM of NNAGAAW)
   NNNNNNNNNNNNNNNNNNNNgttattgtactctcaagatttaGAAAtaaatcttgcagaagctacaaagataaggctt catgccgaaatcaacaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgttattgtactctcaGAAAtgcagaaectacaaagataaggcttcatgccgaaatc aacaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgttattgtactctcaGAAAtgcagaagctacaaagataaggcttcatgccgaaatc aacaccctgtcattttatggcagggtgt**TTTTTT** (N = guide sequence; first underline = tracr mate sequence; second underline = tracr sequence; bold = terminator)
Example chimeric RNA for *S. thermophilus* LMD-9 CRISPR1 Cas9 (with PAM of NNAGAAW)
   NNNNNNNNNNNNNNNNNNNNgttattgtactctcaagatttaGAAAtaaatcttgcagaagctacaatgataaggctt catgccgaaatcaacaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgttattgtactctcaGAAAtgcagaagctacaatgataaggcttcatgccgaaatca acaccctgtcattttatggcagggtgttttcgttatttaa**TTTTTT** (N = guide sequence; first underline = tracr mate sequence; second underline = tracr sequence; bold = terminator)
Example chimeric RNA for *S*. *thermophilus* LMD-9 CRISPR1 Cas9 (with PAM of NNAGAAW)
   NNNNNNNNNNNNNNNNNNNNgttattgtactctcaGAAAtgcagaagctacaatgataasscttcatgccgaaatca acaccctgtcattttatggcagggtgt**TTTTTT** (N = guide sequence; first underline = tracr mate sequence; second underline = tracr sequence; bold = terminator)
Example chimeric RNA for *S*. *thermophilus* LMD-9 CRISPR3 Cas9 (with PAM of NGGNG)
   NNNNNNNNNNNNNNNNNNNNgttttagagctgtgGAAAcacagcgagttaaaataaggcttagtccgtactcaactt gaaaaggtggcaccgattcggtgt**TTTTTT** (N = guide sequence; first underline = tracr mate sequence; second underline = tracr sequence; bold = terminator)
Codon-optimized version of Cas9 from *S*. *thermophilus* LMD-9 CRISPR3 locus (with an NLS at both 5' and 3' ends)

### Example 5: Optimization of the guide RNA for Streptococcus pyogenes Cas9 (referred to as SpCas9).

Applicants mutated the tracrRNA and direct repeat sequences, or mutated the chimeric guide RNA to enhance the RNAs in cells.

The optimization is based on the observation that there were stretches of thymines (Ts) in the tracrRNA and guide RNA, which might lead to early transcription termination by the pol 3 promoter. Therefore Applicants generated the following optimized sequences. Optimized tracrRNA and corresponding optimized direct repeat are presented in pairs.

Optimized tracrRNA 1 (mutation underlined):

Optimized direct repeat 1 (mutation underlined):
GTTaTAGAGCTATGCTGTTaTGAATGGTCCCAAAAC

Optimized tracrRNA 2 (mutation underlined):

Optimized direct repeat 2 (mutation underlined):
GTaTTAGAGCTATGCTGTaTTGAATGGTCCCAAAAC

Applicants also optimized the chimeric guideRNA for optimal activity in eukaryotic cells.

Original guide RNA:

Optimized chimeric guide RNA sequence 1:

Optimized chimeric guide RNA sequence 2:

Optimized chimeric guide RNA sequence 3:

Applicants showed that optimized chimeric guide RNA works better as indicated in Figure 9. The experiment was conducted by co-transfecting 293FT cells with Cas9 and a U6-guide RNA DNA cassette to express one of the four RNA forms shown above. The target of the guide RNA is the same target site in the human Emx1 locus: "GTCACCTCCAATGACTAGGG"

### Example 6: Optimization of Streptococcus thermophilus LMD-9 CRISPR1 Cas9 (referred to as St1Cas9).

Applicants designed guide chimeric RNAs as shown in Figure 12.

The St1Cas9 guide RNAs can under go the same type of optimization as for SpCas9 guide RNAs, by breaking the stretches of poly thymines (Ts).

### Example 7: Improvement of the Cas9 system for in vivo application

Applicants conducted a Metagenomic search for a Cas9 with small molecular weight. Most Cas9 homologs are fairly large. For example the SpCas9 is around 1368aa long, which is too large to be easily packaged into viral vectors for delivery. Some of the sequences may have been mis-annotated and therefore the exact frequency for each length may not necessarily be accurate. Nevertheless it provides a glimpse at distribution of Cas9 proteins and suggest that there are shorter Cas9 homologs.

Through computational analysis, Applicants found that in the bacterial strain *Campylobacter,* there are two Cas9 proteins with less than 1000 amino acids. The sequence for one Cas9 from *Campylobacter jejuni* is presented below. At this length, CjCas9 can be easily packaged into AAV, lentiviruses, Adenoviruses, and other viral vectors for robust delivery into primary cells and *in vivo* in animal models.

>Campylobacter jejuni Cas9 (CjCas9)

The putative tracrRNA element for this CjCas9 is:

The Direct Repeat sequence is:

### ATTTTACCATAAAGAAATTTAAAAAGGGACTAAAAC

The co-fold structure of the tracrRNA and direct repeat is provided in Figure 6.

An example of a chimeric guideRNA for CjCas9 is:

Applicants have also optimized Cas9 guide RNA using *in vitro* methods. Figure 18 shows data from the St1Cas9 chimeric guide RNA optimization *in vitro*.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 8: Sa sgRNA Optimization

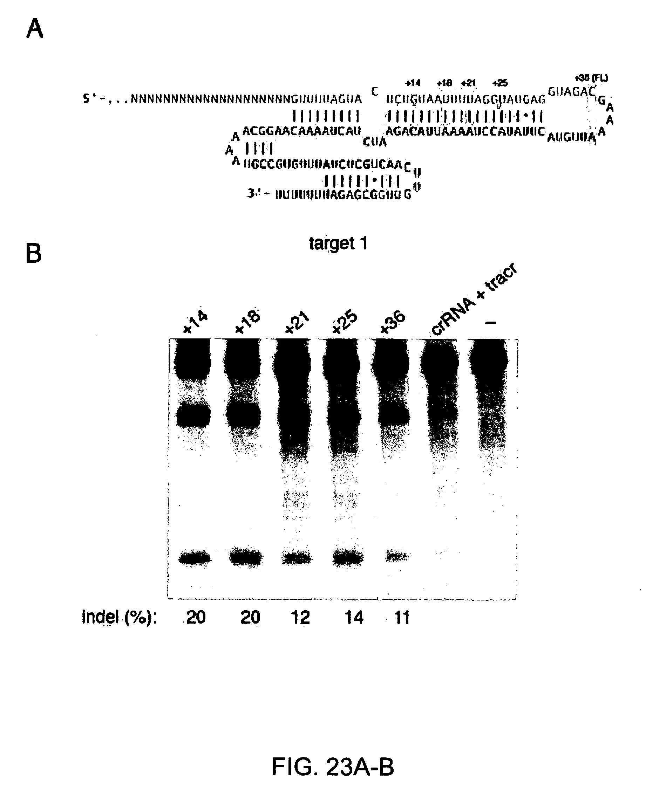

Applicants designed five sgRNA variants for SaCas9 for an optimal truncated architecture with highest cleavage efficiency. In addition, the native direct repeat:tracr duplex system was tested alongside sgRNAs. Guides with indicated lengths were co-transfected with SaCas9 and tested in HEK 293FT cells for activity. A total of 100ng sgRNA U6-PCR amplicon (or 50ng of direct repeat and 50ng of tracrRNA) and 400ng of SaCas9 plasmid were co-transfected into 200,000 Hepa1-6 mouse hepatocytes, and DNA was harvested 72-hours post-transfection for SURVEYOR analysis. The results are shown in Fig. 23.

### References:

1. Urnov, F.D., Rebar, E.J., Holmes, M.C., Zhang, H.S. & Gregory, P.D. Genome editing with engineered zinc finger nucleases. Nat. Rev. Genet. 11, 636-646 (2010).
2. Bogdanove, A.J. & Voytas, D.F. TAL effectors: customizable proteins for DNA targeting. Science 333, 1843-1846 (2011).
3. Stoddard, B.L. Homing endonuclease structure and function. Q. Rev. Biophys. 38, 49-95 (2005).
4. Bae, T. & Schneewind, O. Allelic replacement in Staphylococcus aureus with inducible counter-selection. Plasmid 55, 58-63 (2006).
5. Sung, C.K., Li, H., Claverys, J.P. & Morrison, D.A. An rpsL cassette, janus, for gene replacement through negative selection in Streptococcus pneumoniae. Appl. Environ. Microbiol. 67, 5190-5196 (2001).
6. Sharan, S.K., Thomason, L.C., Kuznetsov, S.G. & Court, D.L. Recombineering: a homologous recombination-based method of genetic engineering. Nat. Protoc. 4, 206-223 (2009).
7. Jinek, M. et al. A programmable dual-RNA-guided DNA endonuclease in adaptive bacterial immunity. Science 337, 816-821 (2012).
8. Deveau, H., Garneau, J.E. & Moineau, S. CRISPR-Cas system and its role in phage-bacteria interactions. Annu. Rev. Microbiol. 64, 475-493 (2010).
9. Horvath, P. & Barrangou, R. CRISPR-Cas, the immune system of bacteria and archaea. Science 327, 167-170 (2010).
10. Terns, M.P. & Terns, R.M. CRISPR-based adaptive immune systems. Curr. Opin. Microbiol. 14, 321-327 (2011).
11. van der Oost, J., Jore, M.M., Westra, E.R., Lundgren, M. & Brouns, S.J. CRISPR-based adaptive and heritable immunity in prokaryotes. Trends. Biochem. Sci. 34, 401-407 (2009).
12. Brouns, S.J. et al. Small CRISPR RNAs guide antiviral defense in prokaryotes. Science 321, 960-964 (2008).
13. Carte, J., Wang, R., Li, H., Terns, R.M. & Terns, M.P. Cas6 is an endoribonuclease that generates guide RNAs for invader defense in prokaryotes. Genes Dev. 22, 3489-3496 (2008).
14. Deltcheva, E. et al. CRISPR RNA maturation by trans-encoded small RNA and host factor RNase III. Nature 471, 602-607 (2011).
15. Hatoum-Aslan, A., Maniv, I. & Marraffini, L.A. Mature clustered, regularly interspaced, short palindromic repeats RNA (crRNA) length is measured by a ruler mechanism anchored at the precursor processing site. Proc. Natl. Acad. Sci. U.S.A. 108, 21218-21222 (2011).
16. Haurwitz, R.E., Jinek, M., Wiedenheft, B., Zhou, K. & Doudna, J.A. Sequence- and structure-specific RNA processing by a CRISPR endonuclease. Science 329, 1355-1358 (2010).
17. Deveau, H. et al. Phage response to CRISPR-encoded resistance in Streptococcus thermophilus. J. Bacteriol. 190, 1390-1400 (2008).
18. Gasiunas, G., Barrangou, R., Horvath, P. & Siksnys, V. Cas9-crRNA ribonucleoprotein complex mediates specific DNA cleavage for adaptive immunity in bacteria. Proc. Natl. Acad. Sci. U.S.A. (2012).
19. Makarova, K.S., Aravind, L., Wolf, Y.I. & Koonin, E.V. Unification of Cas protein families and a simple scenario for the origin and evolution of CRISPR-Cas systems. Biol. Direct. 6, 38 (2011).
20. Barrangou, R. RNA-mediated programmable DNA cleavage. Nat. Biotechnol. 30, 836-838 (2012).
21. Brouns, S.J. Molecular biology. A Swiss army knife of immunity. Science 337, 808-809 (2012).
22. Carroll, D. A CRISPR Approach to Gene Targeting. Mol. Ther. 20, 1658-1660 (2012).
23. Bikard, D., Hatoum-Aslan, A., Mucida, D. & Marraffini, L.A. CRISPR interference can prevent natural transformation and virulence acquisition during in vivo bacterial infection. Cell Host Microbe 12, 177-186 (2012).
24. Sapranauskas, R. et al. The Streptococcus thermophilus CRISPR-Cas system provides immunity in Escherichia coli. Nucleic Acids Res. (2011).
25. Semenova, E. et al. Interference by clustered regularly interspaced short palindromic repeat (CRISPR) RNA is governed by a seed sequence. Proc. Natl. Acad. Sci. U.S.A. (2011).
26. Wiedenheft, B. et al. RNA-guided complex from a bacterial immune system enhances target recognition through seed sequence interactions. Proc. Natl. Acad. Sci. U.S.A. (2011).
27. Zahner, D. & Hakenbeck, R. The Streptococcus pneumoniae beta-galactosidase is a surface protein. J. Bacteriol. 182, 5919-5921 (2000).
28. Marraffini, L.A., Dedent, A.C. & Schneewind, O. Sortases and the art of anchoring proteins to the envelopes of gram-positive bacteria. Microbiol. Mol. Biol. Rev. 70, 192-221 (2006).
29. Motamedi, M.R., Szigety, S.K. & Rosenberg, S.M. Double-strand-break repair recombination in Escherichia coli: physical evidence for a DNA replication mechanism in vivo. Genes Dev. 13, 2889-2903 (1999).
30. Hosaka, T. et al. The novel mutation K87E in ribosomal protein S 12 enhances protein synthesis activity during the late growth phase in Escherichia coli. Mol. Genet. Genomics 271, 317-324 (2004).
31. Costantino, N. & Court, D.L. Enhanced levels of lambda Red-mediated recombinants in mismatch repair mutants. Proc. Natl. Acad. Sci. U.S.A. 100, 15748-15753 (2003).
32. Edgar, R. & Qimron, U. The Escherichia coli CRISPR system protects from lambda lysogenization, lysogens, and prophage induction. J. Bacteriol. 192, 6291-6294 (2010).
33. Marraffini, L.A. & Sontheimer, E.J. Self versus non-self discrimination during CRISPR RNA-directed immunity. Nature 463, 568-571 (2010).
34. Fischer, S. et al. An archaeal immune system can detect multiple Protospacer Adjacent Motifs (PAMs) to target invader DNA. J. Biol. Chem. 287, 33351-33363 (2012).
35. Gudbergsdottir, S. et al. Dynamic properties of the Sulfolobus CRISPR-Cas and CRISPR/Cmr systems when challenged with vector-bome viral and plasmid genes and protospacers. Mol. Microbiol. 79, 35-49 (2011).
36. Wang, H.H. et al. Genome-scale promoter engineering by coselection MAGE. Nat Methods 9, 591-593 (2012).
37. Cong, L. et al. Multiplex Genome Engineering Using CRISPR-Cas Systems. Science In press (2013).
38. Mali, P. et al. RNA-Guided Human Genome Engineering via Cas9. Science In press (2013).
39. Hoskins, J. et al. Genome of the bacterium Streptococcus pneumoniae strain R6. J. Bacteriol. 183, 5709-5717 (2001).
40. Havarstein, L.S., Coomaraswamy, G. & Morrison, D.A. An unmodified heptadecapeptide pheromone induces competence for genetic transformation in Streptococcus pneumoniae. Proc. Natl. Acad. Sci. U.S.A. 92, 11140-11144 (1995).
41. Horinouchi, S. & Weisblum, B. Nucleotide sequence and functional map of pC194, a plasmid that specifies inducible chloramphenicol resistance. J Bacteriol. 150, 815-825 (1982).
42. Horton, R.M. In Vitro Recombination and Mutagenesis of DNA : SOEing Together Tailor-Made Genes. Methods Mol. Biol. 15, 251-261 (1993).
43. Podbielski, A., Spellerberg, B., Woischnik, M., Pohl, B. & Lutticken, R. Novel series of plasmid vectors for gene inactivation and expression analysis in group A streptococci (GAS). Gene 177, 137-147 (1996).
44. Husmann, L.K., Scott, J.R., Lindahl, G. & Stenberg, L. Expression of the Arp protein, a member of the M protein family, is not sufficient to inhibit phagocytosis of Streptococcus pyogenes. Infection and immunity 63, 345-348 (1995).
45. Gibson, D.G. et al. Enzymatic assembly of DNA molecules up to several hundred kilobases. Nat Methods 6, 343-345 (2009).
46. Tangri S, et al. ("Rationally engineered therapeutic proteins with reduced immunogenicity" J Immunol. 2005 Mar 15; 174(6):3187-96.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

## Claims

1. An engineered, non-naturally occurring Clustered Regularly Interspersed Short Palindromic Repeats (CRISPR)-CRISPR associated (Cas) (CRISPR-Cas) vector system comprising one or more vectors comprising:
a) a first regulatory element operably linked to one or more nucleotide sequences encoding one or more CRISPR-Cas system polynucleotide sequences comprising a guide sequence, a tracr RNA, and a tracr mate sequence, wherein the guide sequence hybridizes with one or more target sequences in polynucleotide loci in a eukaryotic cell ,
b) a second regulatory element operably linked to a nucleotide sequence encoding a Type II Cas9 protein,
wherein components (a) and (b) are located on same or different vectors of the system,
wherein the CRISPR-Cas system comprises two or more nuclear localization signals (NLSs) expressed with the nucleotide sequence encoding the Cas9 protein,
whereby the one or more guide sequences target the one or more polynucleotide loci in a eukaryotic cell and the Cas9 protein cleaves the one or more polynucleotide loci, whereby the sequence of the one or more polynucleotide loci is modified.

2. An engineered, non-naturally occurring Type II CRISPR-Cas vector system according to claim 1,
wherein the Cas9 protein is mutated with respect to a corresponding wild type Cas9 protein such that the mutated protein is a nickase that lacks the ability to cleave one strand of a target polynucleotide,
whereby the one or more guide sequences target the one or more polynucleotide loci in a eukaryotic cell and the Cas9 protein cleaves only one strand of the polynucleotide loci, whereby the sequence of the one or more polynucleotide loci is modified.

3. The system of claim 1 or 2, wherein the vectors are viral vectors.

4. The system of claim 3, wherein the viral vectors are retroviral, lentiviral, adenoviral, adeno-associated or herpes simplex viral vectors.

5. The system of any of claims 2-4 wherein the Cas9 protein comprises one or more mutations in the RuvC I, RuvC II or RuvC III catalytic domains.

6. The system of any of claims 2-4 wherein the Cas9 protein comprises a mutation selected from the group consisting of D10A, H840A, N854A and N863A with reference to the position numbering of a *Streptococcus pyogenes* Cas9 (SpCas9) protein.

7. The system of any preceding claim, wherein at least one NLS is at or near amino-terminus of the Cas9 protein and/or at least one NLS is at or near carboxy terminus of the Cas9 protein.

8. The system of claim 7, wherein at least one NLS is at or near amino-terminus of the Cas9 protein and at least one NLS is at or near carboxy terminus of the Cas9 protein.

9. The system of any preceding claim, wherein the one or more CRISPR-Cas system polynucleotide sequences comprise a guide sequence fused to a trans-activating cr (tracr) sequence.

10. The system of any preceding claim, wherein the CRISPR-Cas system polynucleotide sequence is a chimeric RNA comprising the guide sequence, the tracr sequence, and a tracr mate sequence.

11. The system of any preceding claim, wherein the eukaryotic cell is a mammalian cell or a human cell.

12. The system of any preceding claim, wherein the Cas9 protein is codon optimized for expression in a eukaryotic cell.

13. Use of the system of any of claims 1 to 12 for genome engineering, provided that the use does not comprise a process for modifying the germ line genetic identity of human beings, and provided that said use is not a method for treatment of the human or animal body by surgery or therapy.

14. The use of claim 13 wherein the genome engineering comprises modifying a target polynucleotide in a eukaryotic cell, modifying expression of a polynucleotide in a eukaryotic cell, generating a model eukaryotic cell comprising a mutated disease gene, or knocking out a gene.

15. The use of claim 13 wherein the use further comprises repairing said cleaved target polynucleotide by inserting an exogenous template polynucleotide, wherein said repair results in a mutation comprising an insertion, deletion, or substitution of one or more nucleotides of said target polynucleotide.

16. The use of claim 13 wherein the use further comprises editing said cleaved target polynucleotide by inserting an exogenous template polynucleotide, wherein said edit results in a mutation comprising an insertion, deletion, or substitution of one or more nucleotides of said target polynucleotide.

17. The use of claim 15 or 16 wherein the inserting is by homologous recombination.

18. Use of the system of any of claims 1 to 12 in the production of a non-human transgenic animal or transgenic plant

## Patentansprüche

1. Hergestelltes, nicht natürlich vorkommendes CRISPR (Clustered Regularly Interspersed Short Palindromic Repeats)-Cas (CRISPR-assoziiert)-Vektorsystem, umfassend einen oder mehrere Vektor(en), umfassend:
a) ein erstes regulatorisches Element, das funktionell verbunden ist mit einer oder mehreren Nukleotidsequenz(en), die eine oder mehrere CRISPR-Cas-System-Polynukleotidsequenz(en) codiert bzw. codieren, umfassend eine Führungssequenz, eine tracrRNA und eine tracr-Mate-Sequenz, wobei die Führungssequenz mit einer oder mehreren Zielsequenz(en) in Polynukleotidloki in einer eukaryotischen Zelle hybridisiert,
b) ein zweites regulatorisches Element, das funktionell verbunden ist mit einer Nukleotidsequenz, die ein Typ-II-Cas9-Protein codiert,
wobei sich Komponenten (a) und (b) auf dem bzw. den gleichen oder auf verschiedenen Vektor(en) des Systems befinden,
wobei das CRISPR-Cas-System zwei oder mehr Kemlokalisierungssignale (Nuclear Localization Signals, NLSs), exprimiert mit der das Cas9-Protein codierenden Nukleotidsequenz, umfasst,
wodurch die eine oder mehreren Führungssequenz(en) auf den einen Polynukleotidlokus oder die mehreren Polynukleotidloki in einer eukaryotischen Zelle gerichtet ist bzw. sind und das Cas9-Protein den einen Polynukleotidlokus oder die mehreren Polynukleotidloki spaltet, wodurch die Sequenz des einen Polynukleotidlokus oder der mehreren Polynukleotidloki modifiziert wird.

2. Hergestelltes, nicht natürlich vorkommendes Typ-II-CRISPR-Cas-Vektorsystem nach Anspruch 1,
wobei das Cas9-Protein in Bezug auf ein entsprechendes Wildtyp-Cas9-Protein so mutiert ist, dass das mutierte Protein eine Nickase ist, der die Fähigkeit fehlt, einen Strang eines Zielpolynukleotids zu spalten,
wodurch die eine oder mehreren Führungssequenz(en) auf den einen Polynukleotidlokus oder die mehreren Polynukleotidloki in einer eukaryotischen Zelle gerichtet ist bzw. sind und das Cas9-Protein lediglich einen Strang der Polynukleotidloki spaltet, wodurch die Sequenz des einen Polynukleotidlokus oder der mehreren Polynukleotidloki modifiziert wird.

3. System nach Anspruch 1 oder 2, wobei die Vektoren virale Vektoren sind.

4. System nach Anspruch 3, wobei die viralen Vektoren retrovirale, lentivirale, adenovirale, adeno-assoziierte virale Vektoren oder virale Herpes-simplex-Vektoren sind.

5. System nach einem der Ansprüche 2-4, wobei das Cas9-Protein eine oder mehrere Mutation(en) in den katalytischen Domänen RuvC I, RuvC II oder RuvC III umfasst.

6. System nach einem der Ansprüche 2-4, wobei das Cas9-Protein eine Mutation umfasst, die aus der Gruppe gewählt ist, die besteht aus D10A, H840A, N854A und N863A in Bezug auf die Positionsnummerierung eines *Streptococcus-pyogenes*-Cas9-(SpCas-) Proteins.

7. System nach einem der vorstehenden Ansprüche, wobei zumindest ein NLS an oder nahe dem Amino-Terminus des Cas9-Proteins ist und/oder zumindest ein NLS an oder nahe dem Carboxy-Terminus des Cas9-Proteins ist.

8. System nach Anspruch 7, wobei zumindest ein NLS an oder nahe dem Amino-Terminus des Cas9-Proteins ist und zumindest ein NLS an oder nahe dem Carboxy-Terminus des Cas9-Proteins ist.

9. System nach einem der vorstehenden Ansprüche, wobei die eine oder mehreren CRISPR-Cas-System-Polynukleotidsequenz(en) eine Führungssequenz umfasst bzw. umfassen, die an eine trans-activating cr- (tracr-) Sequenz fusioniert ist.

10. System nach einem der vorstehenden Ansprüche, wobei die CRISPR-Cas-System-Polynukleotidsequenz eine chimäre RNA ist, die die Führungssequenz, die tracr-Sequenz und eine tracr-Mate-Sequenz umfasst.

11. System nach einem der vorstehenden Ansprüche, wobei die eukaryotische Zelle eine Säugerzelle oder eine menschliche Zelle ist.

12. System nach einem der vorstehenden Ansprüche, wobei das Cas9-Protein Codonoptimiert für Expression in einer eukaryotischen Zelle ist.

13. Verwendung des Systems nach einem der Ansprüche 1 bis 12 für Genom-Engineering, mit der Maßgabe, dass die Verwendung keinen Prozess zur Modifizierung der genetischen Identität der Keimbahn von Menschen umfasst, und mit der Maßgabe, dass die Verwendung kein Verfahren zur Behandlung des menschlichen oder tierischen Körpers durch operativen Eingriff oder Therapie ist.

14. Verwendung nach Anspruch 13, wobei das Genom-Engineering Modifizieren eines Zielpolynukleotids in einer eukaryotischen Zelle, Modifizieren der Expression eines Polynukleotids in einer eukaryotischen Zelle, Erzeugen einer eukaryotischen Modellzelle, umfassend ein mutiertes Krankheitsgen, oder Knocking-out eines Gens umfasst.

15. Verwendung nach Anspruch 13, wobei die Verwendung weiterhin Reparieren des gespaltenen Zielpolynukleotids durch Einbringen eines exogenen Template-Polynukleotids umfasst, wobei das Reparieren in einer Mutation resultiert, die eine Insertion, Deletion oder Substitution eines Nukleotids oder mehrerer Nukleotide des Zielpolynukleotids umfasst.

16. Verwendung nach Anspruch 13, wobei die Verwendung weiterhin Editieren des gespaltenen Zielpolynukleotids durch Einbringen eines exogenen Template-Polynukleotids umfasst, wobei das Editieren in einer Mutation resultiert, die eine Insertion, Deletion oder Substitution eines Nukleotids oder mehrerer Nukleotide des Zielpolynukleotids umfasst.

17. Verwendung nach Anspruch 15 oder 16, wobei das Einbringen durch homologe Rekombination erfolgt.

18. Verwendung des Systems nach einem der Ansprüche 1 bis 12 bei der Herstellung eines nicht menschlichen transgenen Lebewesens oder einer transgenen Pflanze.

## Revendications

1. Système de vecteurs associé à CRISPR (Cas) (CRISPR-Cas) (CRISPR: répétitions palindromiques courtes régulièrement intercalées regroupées) fabriqué, d'origine non naturelle comprenant un ou plusieurs vecteurs comprenant:
a) un premier élément régulateur lié de manière fonctionnelle à une ou plusieurs séquences de nucléotides codant pour une ou plusieurs séquences polynucléotidiques d'un système CRISPR-Cas comprenant une séquence de guide, un ARN tracr et une séquence complément de tracr, où la séquence de guide s'hybride avec une ou plusieurs séquences cibles dans des loci polynucléotiques dans une cellule eucaryote,
b) un deuxième élément régulateur lié de manière fonctionnelle à une séquence de nucléotides codant pour une protéine Cas9 de Type II,
dans lequel les composants (a) et (b) sont localisés sur le même vecteur ou des vecteurs différents du système,
dans lequel le système CRISPR-Cas comprend deux signaux de localisation nucléaire (NLSs) ou plus exprimés avec la séquence de nucléotides codant pour la protéine Cas9,
en conséquence de quoi les une ou plusieurs séquences de guide ciblent les un ou plusieurs loci polynucléotiques dans une cellule eucaryote et la protéine Cas9 clive les un ou plusieurs loci polynucléotiques, en conséquence de quoi la séquence des un ou plusieurs loci polynucléotiques est modifiée.

2. Système de vecteurs CRISPR-Cas de Type II fabriqué, d'origine non naturelle selon la revendication 1,
dans lequel la protéine Cas9 est mutée par rapport à une protéine Cas9 de type sauvage correspondante de sorte que la protéine mutée est une nickase qui est dénuée de la capacité de cliver un brin d'un polynucléotide cible,
en conséquence de quoi les une ou plusieurs séquences de guide ciblent les un ou plusieurs loci polynucléotiques dans une cellule eucaryote et la protéine Cas9 clive uniquement un brin des loci polynucléotiques, en conséquence de quoi la séquence des un ou plusieurs loci polynucléotiques est modifiée.

3. Système selon la revendication 1 ou 2, dans lequel les vecteurs sont des vecteurs viraux.

4. Système selon la revendication 3, dans lequel les vecteurs viraux sont des vecteurs rétroviraux, lentiviraux, adénoviraux, d'un virus adéno-associé ou du virus de l'herpès simplex.

5. Système selon l'une quelconque des revendications 2-4, dans lequel la protéine Cas9 comprend une ou plusieurs mutations dans les domaines catalytiques RuvC I, RuvC II ou RuvC III.

6. Système selon l'une quelconque des revendications 2-4, dans lequel la protéine Cas9 comprend une mutation choisie dans le groupe constitué de D10A, H840A, N854A et N863A en faisant référence à la numérotation de position d'une protéine Cas9 de *Streptococcus pyogenes* (SpCas9).

7. Système selon l'une quelconque des revendications précédentes, dans lequel au moins un NLS est à ou à proximité de la terminaison amino de la protéine Cas9 et/ou au moins un NLS est à ou à proximité de la terminaison carboxy de la protéine Cas9.

8. Système selon la revendication 7, dans lequel au moins un NLS est à ou à proximité de la terminaison amino de la protéine Cas9 et au moins un NLS est à ou à proximité de la terminaison carboxy de la protéine Cas9.

9. Système selon l'une quelconque des revendications précédentes, dans lequel les une ou plusieurs séquences polynucléotidiques du système CRISPR-Cas comprennent une séquence de guide fusionnée à une séquence cr trans-activante (tracr).

10. Système selon l'une quelconque des revendications précédentes, dans lequel la séquence polynucléotidique du système CRISPR-Cas est un ARN chimérique comprenant la séquence de guide, la séquence tracr et une séquence complément de tracr.

11. Système selon l'une quelconque des revendications précédentes, dans lequel la cellule eucaryote est une cellule mammifère ou une cellule humaine.

12. Système selon l'une quelconque des revendications précédentes, dans lequel la protéine Cas9 est à codons optimisés pour une expression dans une cellule eucaryote.

13. Utilisation du système selon l'une quelconque des revendications 1 à 12 pour une manipulation de génome, à condition que l'utilisation ne comprenne pas un procédé pour la modification de l'identité génétique germinale d'êtres humains et à condition que ladite utilisation ne soit pas une méthode pour le traitement du corps humain ou animal par une intervention chirurgicale ou une thérapie.

14. Utilisation selon la revendication 13, dans laquelle la manipulation de génome comprend la modification d'un polynucléotide cible dans une cellule eucaryote, la modification de l'expression d'un polynucléotide dans une cellule eucaryote, la génération d'une cellule eucaryote modèle comprenant un gène pathologique muté ou l'inactivation d'un gène.

15. Utilisation selon la revendication 13, où l'utilisation comprend en outre la réparation dudit polynucléotide cible clivé par l'insertion d'un polynucléotide matrice exogène, dans laquelle ladite réparation conduit à une mutation comprenant une insertion, une délétion ou une substitution d'un ou de plusieurs nucléotides dudit polynucléotide cible.

16. Utilisation selon la revendication 13, où l'utilisation comprend en outre l'édition dudit polynucléotide cible clivé par l'insertion d'un polynucléotide matrice exogène, dans laquelle ladite édition conduit à une mutation comprenant une insertion, une délétion ou une substitution d'un ou de plusieurs nucléotides dudit polynucléotide cible.

17. Utilisation selon la revendication 15 ou 16, dans laquelle l'insertion se fait par une recombinaison homologue.

18. Utilisation du système selon l'une quelconque des revendications 1 à 12 dans la production d'un animal transgénique non humain ou d'une plante transgénique.


### SEQUENCE LISTING

<110> THE BROAD INSTITUTE, INC. MASSACHUSETTS INSTITUTE OF TECHNOLOGY
<120> ENGINEERING OF SYSTEMS, METHODS AND OPTIMIZED GUIDE COMPOSITIONS FOR SEQUENCE MANIPULATION
<130> PC927381EPA
<140> PCT/US2013/074819
   <141> 2013-12-12
<150> 61/836,127
   <151> 2013-06-17
<150> 61/835,931
   <151> 2013-06-17
<150> 61/828,130
   <151> 2013-05-28
<150> 61/819,803
   <151> 2013-05-06
<150> 61/814,263
   <151> 2013-04-20
<150> 61/806,375
   <151> 2013-03-28
<150> 61/802,174
   <151> 2013-03-15
<150> 61/791,409
   <151> 2013-03-15
<150> 61/769,046
   <151> 2013-02-25
<150> 61/758,468
   <151> 2013-01-30
<150> 61/748,427
   <151> 2013-01-02
<150> 61/736,527
   <151> 2012-12-12
<160> 264
<170> PatentIn version 3.5
<210> 1
   <211> 15
   <212> DNA
   <213> Artificial Sequence
<220>
   <221> source
   <223> /note="Description of Artificial Sequence: Synthetic oligonucleotide"
<400> 1
   aggacgaagt cctaa 15
<210> 2
   <211> 7
   <212> PRT
   <213> Simian virus 40
<400> 2
   Pro Lys Lys Lys Arg Lys Val
   1 5
<210> 3
   <211> 16
   <212> PRT
   <213> Unknown
<220>
   <221> source
   <223> /note="Description of Unknown: Nucleoplasmin bipartite NLS sequence"
<400> 3
   Lys Arg Pro Ala Ala Thr Lys Lys Ala Gly Gln Ala Lys Lys Lys Lys
   1 5 10 15
<210> 4
   <211> 9
   <212> PRT
   <213> Unknown
<220>
   <221> source
   <223> /note="Description of Unknown: C-myc NLS sequence"
<400> 4
   Pro Ala Ala Lys Arg Val Lys Leu Asp
   1 5
<210> 5
   <211> 11
   <212> PRT
   <213> Unknown
<220>
   <221> source
   <223> /note="Description of Unknown: C-myc NLS sequence"
<400> 5
   Arg Gln Arg Arg Asn Glu Leu Lys Arg Ser Pro
   1 5 10
<210> 6
   <211> 38
   <212> PRT
   <213> Homo sapiens
<400> 6
   Asn Gln Ser Ser Asn Phe Gly Pro Met Lys Gly Gly Asn Phe Gly Gly
   1 5 10 15
   Arg Ser Ser Gly Pro Tyr Gly Gly Gly Gly Gln Tyr Phe Ala Lys Pro
   20 25 30
   Arg Asn Gln Gly Gly Tyr
   35
<210> 7
   <211> 42
   <212> PRT
   <213> Unknown
<220>
   <221> source
   <223> /note="Description of Unknown: IBB domain from importin-alpha sequence"
<400> 7
   Arg Met Arg Ile Glx Phe Lys Asn Lys Gly Lys Asp Thr Ala Glu Leu
   1 5 10 15
   Arg Arg Arg Arg Val Glu Val Ser Val Glu Leu Arg Lys Ala Lys Lys
   20 25 30
   Asp Glu Gln Ile Leu Lys Arg Arg Asn Val
   35 40
<210> 8
   <211> 8
   <212> PRT
   <213> Unknown
<220>
   <221> source
   <223> /note="Description of Unknown: Myoma T protein sequence"
<400> 8
   Val Ser Arg Lys Arg Pro Arg Pro
   1 5
<210> 9
   <211> 8
   <212> PRT
   <213> Unknown
<220>
   <221> source
   <223> /note="Description of Unknown: Myoma T protein sequence"
<400> 9
   Pro Pro Lys Lys Ala Arg Glu Asp
   1 5
<210> 10
   <211> 8
   <212> PRT
   <213> Homo sapiens
<400> 10
   Pro Gln Pro Lys Lys Lys Pro Leu
   1 5
<210> 11
   <211> 12
   <212> PRT
   <213> Mus musculus
<400> 11
   Ser Ala Leu Ile Lys Lys Lys Lys Lys Met Ala Pro
   1 5 10
<210> 12
   <211> 5
   <212> PRT
   <213> Influenza virus
<400> 12
   Asp Arg Leu Arg Arg
   1 5
<210> 13
   <211> 7
   <212> PRT
   <213> Influenza virus
<400> 13
   Pro Lys Gln Lys Lys Arg Lys
   1 5
<210> 14
   <211> 10
   <212> PRT
   <213> Hepatitus delta virus
<400> 14
   Arg Lys Leu Lys Lys Lys Ile Lys Lys Leu
   1 5 10
<210> 15
   <211> 10
   <212> PRT
   <213> Mus musculus
<400> 15
   Arg Glu Lys Lys Lys Phe Leu Lys Arg Arg
   1               5                   10
<210> 16
   <211> 20
   <212> PRT
   <213> Homo sapiens
<400> 16
   Lys Arg Lys Gly Asp Glu Val Asp Gly Val Asp Glu Val Ala Lys Lys
   1               5                   10                  15
   Lys Ser Lys Lys
               20
<210> 17
   <211> 17
   <212> PRT
   <213> Homo sapiens
<400> 17
   Arg Lys Cys Leu Gln Ala Gly Met Asn Leu Glu Ala Arg Lys Thr Lys
   1               5                   10                  15
   Lys
<210> 18
   <211> 27
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t or g
<220>
   <221> modified_base
   <222> (21)..(22)
   <223> a, c, t, g, unknown or other
<400> 18
   nnnnnnnnnn nnnnnnnnnn nnagaaw 27
<210> 19
   <211> 19
   <212> DNA
   <213> Artificial Sequence
<220>
   <221> source
   <223> /note="Description of Artificial Sequence: Synthetic oligonucleotide"
<220>
   <221> modified_base
   <222> (1)..(12)
   <223> a, c, t or g
<220>
   <221> modified_base
   <222> (13)..(14)
   <223> a, c, t, g, unknown or other
<400> 19
   nnnnnnnnnn nnnnagaaw 19
<210> 20
   <211> 27
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t or g
<220>
   <221> modified_base
   <222> (21)..(22)
   <223> a, c, t, g, unknown or other
<400> 20
   nnnnnnnnnn nnnnnnnnnn nnagaaw 27
<210> 21
   <211> 18
   <212> DNA
   <213> Artificial Sequence
<220>
   <221> source
   <223> /note="Description of Artificial Sequence: Synthetic oligonucleotide"
<220>
   <221> modified_base
   <222> (1)..(11)
   <223> a, c, t or g
<220>
   <221> modified_base
   <222> (12)..(13)
   <223> a, c, t, g, unknown or other
<400> 21
   nnnnnnnnnn nnnagaaw 18
<210> 22
   <211> 137
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 22
   nnnnnnnnnn nnnnnnnnnn gtttttgtac tctcaagatt tagaaataaa tcttgcagaa 60
   gctacaaaga taaggcttca tgccgaaatc aacaccctgt cattttatgg cagggtgttt 120
   tcgttattta atttttt 137
<210> 23
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 23
   nnnnnnnnnn nnnnnnnnnn gtttttgtac tctcagaaat gcagaagcta caaagataag 60
   gcttcatgcc gaaatcaaca ccctgtcatt ttatggcagg gtgttttcgt tatttaattt 120
   ttt 123
<210> 24
   <211> 110
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 24
   nnnnnnnnnn nnnnnnnnnn gtttttgtac tctcagaaat gcagaagcta caaagataag 60
   gcttcatgcc gaaatcaaca ccctgtcatt ttatggcagg gtgttttttt 110
<210> 25
   <211> 102
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 25
   nnnnnnnnnn nnnnnnnnnn gttttagagc tagaaatagc aagttaaaat aaggctagtc 60
   cgttatcaac ttgaaaaagt ggcaccgagt cggtgctttt tt 102
<210> 26
   <211> 88
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t, g, unknown or other
<400> 26
   nnnnnnnnnn nnnnnnnnnn gttttagagc tagaaatagc aagttaaaat aaggctagtc 60
   cgttatcaac ttgaaaaagt gttttttt 88
<210> 27
   <211> 76
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t, g, unknown or other
<400> 27
   nnnnnnnnnn nnnnnnnnnn gttttagagc tagaaatagc aagttaaaat aaggctagtc 60
   cgttatcatt tttttt 76
<210> 28
   <211> 12
   <212> RNA
   <213> Artificial Sequence
<220>
   <221> source
   <223> /note="Description of Artificial Sequence: Synthetic oligonucleotide"
<400> 28
   guuuuagagc ua 12
<210> 29
   <211> 33
   <212> DNA
   <213> Homo sapiens
<400> 29
   ggacatcgat gtcacctcca atgactaggg tgg 33
<210> 30
   <211> 33
   <212> DNA
   <213> Homo sapiens
<400> 30
   cattggaggt gacatcgatg tcctccccat tgg 33
<210> 31
   <211> 33
   <212> DNA
   <213> Homo sapiens
<400> 31
   ggaagggcct gagtccgagc agaagaagaa ggg 33
<210> 32
   <211> 33
   <212> DNA
   <213> Homo sapiens
<400> 32
   ggtggcgaga ggggccgaga ttgggtgttc agg 33
<210> 33
   <211> 33
   <212> DNA
   <213> Homo sapiens
<400> 33
   atgcaggagg gtggcgagag gggccgagat tgg 33
<210> 34
   <211> 21
   <212> DNA
   <213> Artificial Sequence
<220>
   <221> source
   <223> /note="Description of Artificial Sequence: Synthetic primer"
<400> 34
   aaaaccaccc ttctctctgg c 21
<210> 35
   <211> 21
   <212> DNA
   <213> Artificial Sequence
<220>
   <221> source
   <223> /note="Description of Artificial Sequence: Synthetic primer"
<400> 35
   ggagattgga gacacggaga g 21
<210> 36
   <211> 20
   <212> DNA
   <213> Artificial Sequence
<220>
   <221> source
   <223> /note="Description of Artificial Sequence: Synthetic primer"
<400> 36
   ctggaaagcc aatgcctgac 20
<210> 37
   <211> 20
   <212> DNA
   <213> Artificial Sequence
<220>
   <221> source
   <223> /note="Description of Artificial Sequence: Synthetic primer"
<400> 37
   ggcagcaaac tccttgtcct 20
<210> 38
   <211> 12
   <212> DNA
   <213> Artificial Sequence
<220>
   <221> source
   <223> /note="Description of Artificial Sequence: Synthetic oligonucleotide"
<400> 38
   gttttagagc ta 12
<210> 39
   <211> 335
   <212> DNA
   <213> Artificial Sequence
<220>
   <221> source
   <223> /note="Description of Artificial Sequence: Synthetic polynucleotide"
<400> 39
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg gaaccattca aaacagcata gcaagttaaa ataaggctag tccgttatca 300
   acttgaaaaa gtggcaccga gtcggtgctt ttttt 335
<210> 40
   <211> 423
   <212> DNA
   <213> Artificial Sequence
<220>
   <221> source
   <223> /note="Description of Artificial Sequence: Synthetic polynucleotide"
<400> 40
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg gtagtattaa gtattgtttt atggctgata aatttctttg aatttctcct 300
   tgattatttg ttataaaagt tataaaataa tcttgttgga accattcaaa acagcatagc 360
   aagttaaaat aaggctagtc cgttatcaac ttgaaaaagt ggcaccgagt cggtgctttt 420
   ttt 423
<210> 41
   <211> 339
   <212> DNA
   <213> Artificial Sequence
<220>
   <221> source
   <223> /note="Description of Artificial Sequence: Synthetic polynucleotide"
<400> 41
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ggttttagag ctatgctgtt ttgaatggtc ccaaaacggg tcttcgagaa 300
   gacgttttag agctatgctg ttttgaatgg tcccaaaac 339
<210> 42
   <211> 309
   <212> DNA
   <213> Artificial Sequence
<220>
   <221> source
   <223> /note="Description of Artificial Sequence: Synthetic polynucleotide"
<400> 42
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ggtcttcgag aagacctgtt ttagagctag aaatagcaag ttaaaataag 300
   gctagtccg 309
<210> 43
   <211> 1648
   <212> PRT
   <213> Artificial Sequence
<220>
   <221> source
   <223> /note="Description of Artificial Sequence: Synthetic polypeptide"
<400> 43
   Met Asp Tyr Lys Asp His Asp Gly Asp Tyr Lys Asp His Asp Ile Asp
   1 5 10 15
   Tyr Lys Asp Asp Asp Asp Lys Met Ala Pro Lys Lys Lys Arg Lys Val
   20 25 30
   Gly Ile His Gly Val Pro Ala Ala Asp Lys Lys Tyr Ser Ile Gly Leu
   35 40 45
   Asp Ile Gly Thr Asn Ser Val Gly Trp Ala Val Ile Thr Asp Glu Tyr
   50 55 60
   Lys Val Pro Ser Lys Lys Phe Lys Val Leu Gly Asn Thr Asp Arg His
   65 70 75 80
   Ser Ile Lys Lys Asn Leu Ile Gly Ala Leu Leu Phe Asp Ser Gly Glu
   85 90 95
   Thr Ala Glu Ala Thr Arg Leu Lys Arg Thr Ala Arg Arg Arg Tyr Thr
   100 105 110
   Arg Arg Lys Asn Arg Ile Cys Tyr Leu Gln Glu Ile Phe Ser Asn Glu
   115 120 125
   Met Ala Lys Val Asp Asp Ser Phe Phe His Arg Leu Glu Glu Ser Phe
   130 135 140
   Leu Val Glu Glu Asp Lys Lys His Glu Arg His Pro Ile Phe Gly Asn
   145 150 155 160
   Ile Val Asp Glu Val Ala Tyr His Glu Lys Tyr Pro Thr Ile Tyr His
   165 170 175
   Leu Arg Lys Lys Leu Val Asp Ser Thr Asp Lys Ala Asp Leu Arg Leu
   180 185 190
   Ile Tyr Leu Ala Leu Ala His Met Ile Lys Phe Arg Gly His Phe Leu
   195 200 205
   Ile Glu Gly Asp Leu Asn Pro Asp Asn Ser Asp Val Asp Lys Leu Phe
   210 215 220
   Ile Gln Leu Val Gln Thr Tyr Asn Gln Leu Phe Glu Glu Asn Pro Ile
   225 230 235 240
   Asn Ala Ser Gly Val Asp Ala Lys Ala Ile Leu Ser Ala Arg Leu Ser
   245 250 255
   Lys Ser Arg Arg Leu Glu Asn Leu Ile Ala Gln Leu Pro Gly Glu Lys
   260 265 270
   Lys Asn Gly Leu Phe Gly Asn Leu Ile Ala Leu Ser Leu Gly Leu Thr
   275 280 285
   Pro Asn Phe Lys Ser Asn Phe Asp Leu Ala Glu Asp Ala Lys Leu Gln
   290 295 300
   Leu Ser Lys Asp Thr Tyr Asp Asp Asp Leu Asp Asn Leu Leu Ala Gln
   305 310 315 320
   Ile Gly Asp Gln Tyr Ala Asp Leu Phe Leu Ala Ala Lys Asn Leu Ser
   325 330 335
   Asp Ala Ile Leu Leu Ser Asp Ile Leu Arg Val Asn Thr Glu Ile Thr
   340 345 350
   Lys Ala Pro Leu Ser Ala Ser Met Ile Lys Arg Tyr Asp Glu His His
   355 360 365
   Gln Asp Leu Thr Leu Leu Lys Ala Leu Val Arg Gln Gln Leu Pro Glu
   370 375 380
   Lys Tyr Lys Glu Ile Phe Phe Asp Gln Ser Lys Asn Gly Tyr Ala Gly
   385 390 395 400
   Tyr Ile Asp Gly Gly Ala Ser Gln Glu Glu Phe Tyr Lys Phe Ile Lys
   405 410 415
   Pro Ile Leu Glu Lys Met Asp Gly Thr Glu Glu Leu Leu Val Lys Leu
   420 425 430
   Asn Arg Glu Asp Leu Leu Arg Lys Gln Arg Thr Phe Asp Asn Gly Ser
   435 440 445
   Ile Pro His Gln Ile His Leu Gly Glu Leu His Ala Ile Leu Arg Arg
   450 455 460
   Gln Glu Asp Phe Tyr Pro Phe Leu Lys Asp Asn Arg Glu Lys Ile Glu
   465 470 475 480
   Lys Ile Leu Thr Phe Arg Ile Pro Tyr Tyr Val Gly Pro Leu Ala Arg
   485 490 495
   Gly Asn Ser Arg Phe Ala Trp Met Thr Arg Lys Ser Glu Glu Thr Ile
   500 505 510
   Thr Pro Trp Asn Phe Glu Glu Val Val Asp Lys Gly Ala Ser Ala Gln
   515 520 525
   Ser Phe Ile Glu Arg Met Thr Asn Phe Asp Lys Asn Leu Pro Asn Glu
   530 535 540
   Lys Val Leu Pro Lys His Ser Leu Leu Tyr Glu Tyr Phe Thr Val Tyr
   545 550 555 560
   Asn Glu Leu Thr Lys Val Lys Tyr Val Thr Glu Gly Met Arg Lys Pro
   565 570 575
   Ala Phe Leu Ser Gly Glu Gln Lys Lys Ala Ile Val Asp Leu Leu Phe
   580 585 590
   Lys Thr Asn Arg Lys Val Thr Val Lys Gln Leu Lys Glu Asp Tyr Phe
   595 600 605
   Lys Lys Ile Glu Cys Phe Asp Ser Val Glu Ile Ser Gly Val Glu Asp
   610 615 620
   Arg Phe Asn Ala Ser Leu Gly Thr Tyr His Asp Leu Leu Lys Ile Ile
   625 630 635 640
   Lys Asp Lys Asp Phe Leu Asp Asn Glu Glu Asn Glu Asp Ile Leu Glu
   645 650 655
   Asp Ile Val Leu Thr Leu Thr Leu Phe Glu Asp Arg Glu Met Ile Glu
   660 665 670
   Glu Arg Leu Lys Thr Tyr Ala His Leu Phe Asp Asp Lys Val Met Lys
   675 680 685
   Gln Leu Lys Arg Arg Arg Tyr Thr Gly Trp Gly Arg Leu Ser Arg Lys
   690 695 700
   Leu Ile Asn Gly Ile Arg Asp Lys Gln Ser Gly Lys Thr Ile Leu Asp
   705 710 715 720
   Phe Leu Lys Ser Asp Gly Phe Ala Asn Arg Asn Phe Met Gln Leu Ile
   725 730 735
   His Asp Asp Ser Leu Thr Phe Lys Glu Asp Ile Gln Lys Ala Gln Val
   740 745 750
   Ser Gly Gln Gly Asp Ser Leu His Glu His Ile Ala Asn Leu Ala Gly
   755 760 765
   Ser Pro Ala Ile Lys Lys Gly Ile Leu Gln Thr Val Lys Val Val Asp
   770 775 780
   Glu Leu Val Lys Val Met Gly Arg His Lys Pro Glu Asn Ile Val Ile
   785 790 795 800
   Glu Met Ala Arg Glu Asn Gln Thr Thr Gln Lys Gly Gln Lys Asn Ser
   805 810 815
   Arg Glu Arg Met Lys Arg Ile Glu Glu Gly Ile Lys Glu Leu Gly Ser
   820 825 830
   Gln Ile Leu Lys Glu His Pro Val Glu Asn Thr Gln Leu Gln Asn Glu
   835 840 845
   Lys Leu Tyr Leu Tyr Tyr Leu Gln Asn Gly Arg Asp Met Tyr Val Asp
   850 855 860
   Gln Glu Leu Asp Ile Asn Arg Leu Ser Asp Tyr Asp Val Asp His Ile
   865 870 875 880
   Val Pro Gln Ser Phe Leu Lys Asp Asp Ser Ile Asp Asn Lys Val Leu
   885 890 895
   Thr Arg Ser Asp Lys Asn Arg Gly Lys Ser Asp Asn Val Pro Ser Glu
   900 905 910
   Glu Val Val Lys Lys Met Lys Asn Tyr Trp Arg Gln Leu Leu Asn Ala
   915 920 925
   Lys Leu Ile Thr Gln Arg Lys Phe Asp Asn Leu Thr Lys Ala Glu Arg
   930 935 940
   Gly Gly Leu Ser Glu Leu Asp Lys Ala Gly Phe Ile Lys Arg Gln Leu
   945 950 955 960
   Val Glu Thr Arg Gln Ile Thr Lys His Val Ala Gln Ile Leu Asp Ser
   965 970 975
   Arg Met Asn Thr Lys Tyr Asp Glu Asn Asp Lys Leu Ile Arg Glu Val
   980 985 990
   Lys Val Ile Thr Leu Lys Ser Lys Leu Val Ser Asp Phe Arg Lys Asp
   995 1000 1005
   Phe Gln Phe Tyr Lys Val Arg Glu Ile Asn Asn Tyr His His Ala
   1010 1015 1020
   His Asp Ala Tyr Leu Asn Ala Val Val Gly Thr Ala Leu Ile Lys
   1025 1030 1035
   Lys Tyr Pro Lys Leu Glu Ser Glu Phe Val Tyr Gly Asp Tyr Lys
   1040 1045 1050
   Val Tyr Asp Val Arg Lys Met Ile Ala Lys Ser Glu Gln Glu Ile
   1055 1060 1065
   Gly Lys Ala Thr Ala Lys Tyr Phe Phe Tyr Ser Asn Ile Met Asn
   1070 1075 1080
   Phe Phe Lys Thr Glu Ile Thr Leu Ala Asn Gly Glu Ile Arg Lys
   1085 1090 1095
   Arg Pro Leu Ile Glu Thr Asn Gly Glu Thr Gly Glu Ile Val Trp
   1100 1105 1110
   Asp Lys Gly Arg Asp Phe Ala Thr Val Arg Lys Val Leu Ser Met
   1115 1120 1125
   Pro Gln Val Asn Ile Val Lys Lys Thr Glu Val Gln Thr Gly Gly
   1130 1135 1140
   Phe Ser Lys Glu Ser Ile Leu Pro Lys Arg Asn Ser Asp Lys Leu
   1145 1150 1155
   Ile Ala Arg Lys Lys Asp Trp Asp Pro Lys Lys Tyr Gly Gly Phe
   1160 1165 1170
   Asp Ser Pro Thr Val Ala Tyr Ser Val Leu Val Val Ala Lys Val
   1175 1180 1185
   Glu Lys Gly Lys Ser Lys Lys Leu Lys Ser Val Lys Glu Leu Leu
   1190 1195 1200
   Gly Ile Thr Ile Met Glu Arg Ser Ser Phe Glu Lys Asn Pro Ile
   1205 1210 1215
   Asp Phe Leu Glu Ala Lys Gly Tyr Lys Glu Val Lys Lys Asp Leu
   1220 1225 1230
   Ile Ile Lys Leu Pro Lys Tyr Ser Leu Phe Glu Leu Glu Asn Gly
   1235 1240 1245
   Arg Lys Arg Met Leu Ala Ser Ala Gly Glu Leu Gln Lys Gly Asn
   1250 1255 1260
   Glu Leu Ala Leu Pro Ser Lys Tyr Val Asn Phe Leu Tyr Leu Ala
   1265 1270 1275
   Ser His Tyr Glu Lys Leu Lys Gly Ser Pro Glu Asp Asn Glu Gln
   1280 1285 1290
   Lys Gln Leu Phe Val Glu Gln His Lys His Tyr Leu Asp Glu Ile
   1295 1300 1305
   Ile Glu Gln Ile Ser Glu Phe Ser Lys Arg Val Ile Leu Ala Asp
   1310 1315 1320
   Ala Asn Leu Asp Lys Val Leu Ser Ala Tyr Asn Lys His Arg Asp
   1325 1330 1335
   Lys Pro Ile Arg Glu Gln Ala Glu Asn Ile Ile His Leu Phe Thr
   1340 1345 1350
   Leu Thr Asn Leu Gly Ala Pro Ala Ala Phe Lys Tyr Phe Asp Thr
   1355 1360 1365
   Thr Ile Asp Arg Lys Arg Tyr Thr Ser Thr Lys Glu Val Leu Asp
   1370 1375 1380
   Ala Thr Leu Ile His Gln Ser Ile Thr Gly Leu Tyr Glu Thr Arg
   1385 1390 1395
   Ile Asp Leu Ser Gln Leu Gly Gly Asp Ala Ala Ala Val Ser Lys
   1400 1405 1410
   Gly Glu Glu Leu Phe Thr Gly Val Val Pro Ile Leu Val Glu Leu
   1415 1420 1425
   Asp Gly Asp Val Asn Gly His Lys Phe Ser Val Ser Gly Glu Gly
   1430 1435 1440
   Glu Gly Asp Ala Thr Tyr Gly Lys Leu Thr Leu Lys Phe Ile Cys
   1445 1450 1455
   Thr Thr Gly Lys Leu Pro Val Pro Trp Pro Thr Leu Val Thr Thr
   1460 1465 1470
   Leu Thr Tyr Gly Val Gln Cys Phe Ser Arg Tyr Pro Asp His Met
   1475 1480 1485
   Lys Gln His Asp Phe Phe Lys Ser Ala Met Pro Glu Gly Tyr Val
   1490 1495 1500
   Gln Glu Arg Thr Ile Phe Phe Lys Asp Asp Gly Asn Tyr Lys Thr
   1505 1510 1515
   Arg Ala Glu Val Lys Phe Glu Gly Asp Thr Leu Val Asn Arg Ile
   1520 1525 1530
   Glu Leu Lys Gly Ile Asp Phe Lys Glu Asp Gly Asn Ile Leu Gly
   1535 1540 1545
   His Lys Leu Glu Tyr Asn Tyr Asn Ser His Asn Val Tyr Ile Met
   1550 1555 1560
   Ala Asp Lys Gln Lys Asn Gly Ile Lys Val Asn Phe Lys Ile Arg
   1565 1570 1575
   His Asn Ile Glu Asp Gly Ser Val Gln Leu Ala Asp His Tyr Gln
   1580 1585 1590
   Gln Asn Thr Pro Ile Gly Asp Gly Pro Val Leu Leu Pro Asp Asn
   1595 1600 1605
   His Tyr Leu Ser Thr Gln Ser Ala Leu Ser Lys Asp Pro Asn Glu
   1610 1615 1620
   Lys Arg Asp His Met Val Leu Leu Glu Phe Val Thr Ala Ala Gly
   1625 1630 1635
   Ile Thr Leu Gly Met Asp Glu Leu Tyr Lys
   1640 1645
<210> 44
   <211> 1625
   <212> PRT
   <213> Artificial Sequence
<220>
   <221> source
   <223> /note="Description of Artificial Sequence: Synthetic polypeptide"
<400> 44
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
   20 25 30
   Lys Val Leu Gly Asn Thr Asp Arg His Ser Ile Lys Lys Asn Leu Ile
   35 40 45
   Gly Ala Leu Leu Phe Asp Ser Gly Glu Thr Ala Glu Ala Thr Arg Leu
   50 55 60
   Lys Arg Thr Ala Arg Arg Arg Tyr Thr Arg Arg Lys Asn Arg Ile Cys
   65 70 75 80
   Tyr Leu Gln Glu Ile Phe Ser Asn Glu Met Ala Lys Val Asp Asp Ser
   85 90 95
   Phe Phe His Arg Leu Glu Glu Ser Phe Leu Val Glu Glu Asp Lys Lys
   100 105 110
   His Glu Arg His Pro Ile Phe Gly Asn Ile Val Asp Glu Val Ala Tyr
   115 120 125
   His Glu Lys Tyr Pro Thr Ile Tyr His Leu Arg Lys Lys Leu Val Asp
   130 135 140
   Ser Thr Asp Lys Ala Asp Leu Arg Leu Ile Tyr Leu Ala Leu Ala His
   145 150 155 160
   Met Ile Lys Phe Arg Gly His Phe Leu Ile Glu Gly Asp Leu Asn Pro
   165 170 175
   Asp Asn Ser Asp Val Asp Lys Leu Phe Ile Gln Leu Val Gln Thr Tyr
   180 185 190
   Asn Gln Leu Phe Glu Glu Asn Pro Ile Asn Ala Ser Gly Val Asp Ala
   195 200 205
   Lys Ala Ile Leu Ser Ala Arg Leu Ser Lys Ser Arg Arg Leu Glu Asn
   210 215 220
   Leu Ile Ala Gln Leu Pro Gly Glu Lys Lys Asn Gly Leu Phe Gly Asn
   225 230 235 240
   Leu Ile Ala Leu Ser Leu Gly Leu Thr Pro Asn Phe Lys Ser Asn Phe
   245 250 255
   Asp Leu Ala Glu Asp Ala Lys Leu Gln Leu Ser Lys Asp Thr Tyr Asp
   260 265 270
   Asp Asp Leu Asp Asn Leu Leu Ala Gln Ile Gly Asp Gln Tyr Ala Asp
   275 280 285
   Leu Phe Leu Ala Ala Lys Asn Leu Ser Asp Ala Ile Leu Leu Ser Asp
   290 295 300
   Ile Leu Arg Val Asn Thr Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala
   1010 1015 1020
   Lys Ser Glu Gln Glu Ile Gly Lys Ala Thr Ala Lys Tyr Phe Phe
   1025 1030 1035
   Tyr Ser Asn Ile Met Asn Phe Phe Lys Thr Glu Ile Thr Leu Ala
   1040 1045 1050
   Asn Gly Glu Ile Arg Lys Arg Pro Leu Ile Glu Thr Asn Gly Glu
   1055 1060 1065
   Thr Gly Glu Ile Val Trp Asp Lys Gly Arg Asp Phe Ala Thr Val
   1070 1075 1080
   Arg Lys Val Leu Ser Met Pro Gln Val Asn Ile Val Lys Lys Thr
   1085 1090 1095
   Glu Val Gln Thr Gly Gly Phe Ser Lys Glu Ser Ile Leu Pro Lys
   1100 1105 1110
   Arg Asn Ser Asp Lys Leu Ile Ala Arg Lys Lys Asp Trp Asp Pro
   1115 1120 1125
   Lys Lys Tyr Gly Gly Phe Asp Ser Pro Thr Val Ala Tyr Ser Val
   1130 1135 1140
   Leu Val Val Ala Lys Val Glu Lys Gly Lys Ser Lys Lys Leu Lys
   1145 1150 1155
   Ser Val Lys Glu Leu Leu Gly Ile Thr Ile Met Glu Arg Ser Ser
   1160 1165 1170
   Phe Glu Lys Asn Pro Ile Asp Phe Leu Glu Ala Lys Gly Tyr Lys
   1175 1180 1185
   Glu Val Lys Lys Asp Leu Ile Ile Lys Leu Pro Lys Tyr Ser Leu
   1190 1195 1200
   Phe Glu Leu Glu Asn Gly Arg Lys Arg Met Leu Ala Ser Ala Gly
   1205 1210 1215
   Glu Leu Gln Lys Gly Asn Glu Leu Ala Leu Pro Ser Lys Tyr Val
   1220 1225 1230
   Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys
   1250 1255 1260
   His Tyr Leu Asp Glu Ile Ile Glu Gln Ile Ser Glu Phe Ser Lys
       1265                1270                1275
   Arg Val Ile Leu Ala Asp Ala Asn Leu Asp Lys Val Leu Ser Ala
       1280                1285                1290
   Tyr Asn Lys His Arg Asp Lys Pro Ile Arg Glu Gln Ala Glu Asn
       1295                1300                1305
   Ile Ile His Leu Phe Thr Leu Thr Asn Leu Gly Ala Pro Ala Ala
       1310                1315                1320
   Phe Lys Tyr Phe Asp Thr Thr Ile Asp Arg Lys Arg Tyr Thr Ser
       1325                1330                1335
   Thr Lys Glu Val Leu Asp Ala Thr Leu Ile His Gln Ser Ile Thr
       1340                1345                1350
   Gly Leu Tyr Glu Thr Arg Ile Asp Leu Ser Gln Leu Gly Gly Asp
       1355                1360                1365
   Ala Ala Ala Val Ser Lys Gly Glu Glu Leu Phe Thr Gly Val Val
       1370                1375                1380
   Pro Ile Leu Val Glu Leu Asp Gly Asp Val Asn Gly His Lys Phe
       1385                1390                1395
   Ser Val Ser Gly Glu Gly Glu Gly Asp Ala Thr Tyr Gly Lys Leu
       1400                1405                1410
   Thr Leu Lys Phe Ile Cys Thr Thr Gly Lys Leu Pro Val Pro Trp
       1415                1420                1425
   Pro Thr Leu Val Thr Thr Leu Thr Tyr Gly Val Gln Cys Phe Ser
       1430                1435                1440
   Arg Tyr Pro Asp His Met Lys Gln His Asp Phe Phe Lys Ser Ala
       1445                1450                1455
   Met Pro Glu Gly Tyr Val Gln Glu Arg Thr Ile Phe Phe Lys Asp
       1460                1465                1470
   Asp Gly Asn Tyr Lys Thr Arg Ala Glu Val Lys Phe Glu Gly Asp
       1475                1480                1485
   Thr Leu Val Asn Arg Ile Glu Leu Lys Gly Ile Asp Phe Lys Glu
       1490                1495                1500
   Asp Gly Asn Ile Leu Gly His Lys Leu Glu Tyr Asn Tyr Asn Ser
   1505 1510 1515
   His Asn Val Tyr Ile Met Ala Asp Lys Gln Lys Asn Gly Ile Lys
   1520 1525 1530
   Val Asn Phe Lys Ile Arg His Asn Ile Glu Asp Gly Ser Val Gln
   1535 1540 1545
   Leu Ala Asp His Tyr Gln Gln Asn Thr Pro Ile Gly Asp Gly Pro
   1550 1555 1560
   Val Leu Leu Pro Asp Asn His Tyr Leu Ser Thr Gln Ser Ala Leu
   1565 1570 1575
   Ser Lys Asp Pro Asn Glu Lys Arg Asp His Met Val Leu Leu Glu
   1580 1585 1590
   Phe Val Thr Ala Ala Gly Ile Thr Leu Gly Met Asp Glu Leu Tyr
   1595 1600 1605
   Lys Lys Arg Pro Ala Ala Thr Lys Lys Ala Gly Gln Ala Lys Lys
   1610 1615 1620
   Lys Lys
   1625
<210> 45
   <211> 1664
   <212> PRT
   <213> Artificial Sequence
<220>
   <221> source
   <223> /note="Description of Artificial Sequence: Synthetic polypeptide"
<400> 45
   Met Asp Tyr Lys Asp His Asp Gly Asp Tyr Lys Asp His Asp Ile Asp
   1 5 10 15
   Tyr Lys Asp Asp Asp Asp Lys Met Ala Pro Lys Lys Lys Arg Lys Val
   20 25 30
   Gly Ile His Gly Val Pro Ala Ala Asp Lys Lys Tyr Ser Ile Gly Leu
   35 40 45
   Asp Ile Gly Thr Asn Ser Val Gly Trp Ala Val Ile Thr Asp Glu Tyr
   50 55 60
   Lys Val Pro Ser Lys Lys Phe Lys Val Leu Gly Asn Thr Asp Arg His
   65                  70                  75                  80
   Ser Ile Lys Lys Asn Leu Ile Gly Ala Leu Leu Phe Asp Ser Gly Glu
                   85                  90                  95
   Thr Ala Glu Ala Thr Arg Leu Lys Arg Thr Ala Arg Arg Arg Tyr Thr
               100                 105                 110
   Arg Arg Lys Asn Arg Ile Cys Tyr Leu Gln Glu Ile Phe Ser Asn Glu
           115                 120                 125
   Met Ala Lys Val Asp Asp Ser Phe Phe His Arg Leu Glu Glu Ser Phe
       130                 135                 140
   Leu Val Glu Glu Asp Lys Lys His Glu Arg His Pro Ile Phe Gly Asn
   145                 150                 155                 160
   Ile Val Asp Glu Val Ala Tyr His Glu Lys Tyr Pro Thr Ile Tyr His
                   165                 170                 175
   Leu Arg Lys Lys Leu Val Asp Ser Thr Asp Lys Ala Asp Leu Arg Leu
               180                 185                 190
   Ile Tyr Leu Ala Leu Ala His Met Ile Lys Phe Arg Gly His Phe Leu
           195                 200                 205
   Ile Glu Gly Asp Leu Asn Pro Asp Asn Ser Asp Val Asp Lys Leu Phe
       210                 215                 220
   Ile Gln Leu Val Gln Thr Tyr Asn Gln Leu Phe Glu Glu Asn Pro Ile
   225                 230                 235                 240
   Asn Ala Ser Gly Val Asp Ala Lys Ala Ile Leu Ser Ala Arg Leu Ser
                   245                 250                 255
   Lys Ser Arg Arg Leu Glu Asn Leu Ile Ala Gln Leu Pro Gly Glu Lys
               260                 265                 270
   Lys Asn Gly Leu Phe Gly Asn Leu Ile Ala Leu Ser Leu Gly Leu Thr
           275                 280                 285
   Pro Asn Phe Lys Ser Asn Phe Asp Leu Ala Glu Asp Ala Lys Leu Gln
       290                 295                 300
   Leu Ser Lys Asp Thr Tyr Asp Asp Asp Leu Asp Asn Leu Leu Ala Gln
   305                 310                 315                 320
   Ile Gly Asp Gln Tyr Ala Asp Leu Phe Leu Ala Ala Lys Asn Leu Ser
   325 330 335
   Asp Ala Ile Leu Leu Ser Asp Ile Leu Arg Val Asn Thr Glu Ile Thr
   340 345 350
   Lys Ala Pro Leu Ser Ala Ser Met Ile Lys Arg Tyr Asp Glu His His
   355 360 365
   Gln Asp Leu Thr Leu Leu Lys Ala Leu Val Arg Gln Gln Leu Pro Glu
   370 375 380
   Lys Tyr Lys Glu Ile Phe Phe Asp Gln Ser Lys Asn Gly Tyr Ala Gly
   385 390 395 400
   Tyr Ile Asp Gly Gly Ala Ser Gln Glu Glu Phe Tyr Lys Phe Ile Lys
   405 410 415
   Pro Ile Leu Glu Lys Met Asp Gly Thr Glu Glu Leu Leu Val Lys Leu
   420 425 430
   Asn Arg Glu Asp Leu Leu Arg Lys Gln Arg Thr Phe Asp Asn Gly Ser
   435 440 445
   Ile Pro His Gln Ile His Leu Gly Glu Leu His Ala Ile Leu Arg Arg
   450 455 460
   Gln Glu Asp Phe Tyr Pro Phe Leu Lys Asp Asn Arg Glu Lys Ile Glu
   465 470 475 480
   Lys Ile Leu Thr Phe Arg Ile Pro Tyr Tyr Val Gly Pro Leu Ala Arg
   485 490 495
   Gly Asn Ser Arg Phe Ala Trp Met Thr Arg Lys Ser Glu Glu Thr Ile
   500 505 510
   Thr Pro Trp Asn Phe Glu Glu Val Val Asp Lys Gly Ala Ser Ala Gln
   515 520 525
   Ser Phe Ile Glu Arg Met Thr Asn Phe Asp Lys Asn Leu Pro Asn Glu
   530 535 540
   Lys Val Leu Pro Lys His Ser Leu Leu Tyr Glu Tyr Phe Thr Val Tyr
   545 550 555 560
   Asn Glu Leu Thr Lys Val Lys Tyr Val Thr Glu Gly Met Arg Lys Pro
   565 570 575
   Ala Phe Leu Ser Gly Glu Gln Lys Lys Ala Ile Val Asp Leu Leu Phe
   580 585 590
   Lys Thr Asn Arg Lys Val Thr Val Lys Gln Leu Lys Glu Asp Tyr Phe
   595 600 605
   Lys Lys Ile Glu Cys Phe Asp Ser Val Glu Ile Ser Gly Val Glu Asp
   610 615 620
   Arg Phe Asn Ala Ser Leu Gly Thr Tyr His Asp Leu Leu Lys Ile Ile
   625 630 635 640
   Lys Asp Lys Asp Phe Leu Asp Asn Glu Glu Asn Glu Asp Ile Leu Glu
   645 650 655
   Asp Ile Val Leu Thr Leu Thr Leu Phe Glu Asp Arg Glu Met Ile Glu
   660 665 670
   Glu Arg Leu Lys Thr Tyr Ala His Leu Phe Asp Asp Lys Val Met Lys
   675 680 685
   Gln Leu Lys Arg Arg Arg Tyr Thr Gly Trp Gly Arg Leu Ser Arg Lys
   690 695 700
   Leu Ile Asn Gly Ile Arg Asp Lys Gln Ser Gly Lys Thr Ile Leu Asp
   705 710 715 720
   Phe Leu Lys Ser Asp Gly Phe Ala Asn Arg Asn Phe Met Gln Leu Ile
   725 730 735
   His Asp Asp Ser Leu Thr Phe Lys Glu Asp Ile Gln Lys Ala Gln Val
   740 745 750
   Ser Gly Gln Gly Asp Ser Leu His Glu His Ile Ala Asn Leu Ala Gly
   755 760 765
   Ser Pro Ala Ile Lys Lys Gly Ile Leu Gln Thr Val Lys Val Val Asp
   770 775 780
   Glu Leu Val Lys Val Met Gly Arg His Lys Pro Glu Asn Ile Val Ile
   785 790 795 800
   Glu Met Ala Arg Glu Asn Gln Thr Thr Gln Lys Gly Gln Lys Asn Ser
   805 810 815
   Arg Glu Arg Met Lys Arg Ile Glu Glu Gly Ile Lys Glu Leu Gly Ser
   820 825 830
   Gln Ile Leu Lys Glu His Pro Val Glu Asn Thr Gln Leu Gln Asn Glu
   835 840 845
   Lys Leu Tyr Leu Tyr Tyr Leu Gln Asn Gly Arg Asp Met Tyr Val Asp
   850 855 860
   Gln Glu Leu Asp Ile Asn Arg Leu Ser Asp Tyr Asp Val Asp His Ile
   865 870 875 880
   Val Pro Gln Ser Phe Leu Lys Asp Asp Ser Ile Asp Asn Lys Val Leu
   885 890 895
   Thr Arg Ser Asp Lys Asn Arg Gly Lys Ser Asp Asn Val Pro Ser Glu
   900 905 910
   Glu Val Val Lys Lys Met Lys Asn Tyr Trp Arg Gln Leu Leu Asn Ala
   915 920 925
   Lys Leu Ile Thr Gln Arg Lys Phe Asp Asn Leu Thr Lys Ala Glu Arg
   930 935 940
   Gly Gly Leu Ser Glu Leu Asp Lys Ala Gly Phe Ile Lys Arg Gln Leu
   945 950 955 960
   Val Glu Thr Arg Gln Ile Thr Lys His Val Ala Gln Ile Leu Asp Ser
   965 970 975
   Arg Met Asn Thr Lys Tyr Asp Glu Asn Asp Lys Leu Ile Arg Glu Val
   980 985 990
   Lys Val Ile Thr Leu Lys Ser Lys Leu Val Ser Asp Phe Arg Lys Asp
   995 1000 1005
   Phe Gln Phe Tyr Lys Val Arg Glu Ile Asn Asn Tyr His His Ala
   1010 1015 1020
   His Asp Ala Tyr Leu Asn Ala Val Val Gly Thr Ala Leu Ile Lys
   1025 1030 1035
   Lys Tyr Pro Lys Leu Glu Ser Glu Phe Val Tyr Gly Asp Tyr Lys
   1040 1045 1050
   Val Tyr Asp Val Arg Lys Met Ile Ala Lys Ser Glu Gln Glu Ile
   1055 1060 1065
   Gly Lys Ala Thr Ala Lys Tyr Phe Phe Tyr Ser Asn Ile Met Asn
   1070 1075 1080
   Phe Phe Lys Thr Glu Ile Thr Leu Ala Asn Gly Glu Ile Arg Lys
   1085 1090 1095
   Arg Pro Leu Ile Glu Thr Asn Gly Glu Thr Gly Glu Ile Val Trp
   1100 1105 1110
   Asp Lys Gly Arg Asp Phe Ala Thr Val Arg Lys Val Leu Ser Met
   1115 1120 1125
   Pro Gln Val Asn Ile Val Lys Lys Thr Glu Val Gln Thr Gly Gly
   1130 1135 1140
   Phe Ser Lys Glu Ser Ile Leu Pro Lys Arg Asn Ser Asp Lys Leu
   1145 1150 1155
   Ile Ala Arg Lys Lys Asp Trp Asp Pro Lys Lys Tyr Gly Gly Phe
   1160 1165 1170
   Asp Ser Pro Thr Val Ala Tyr Ser Val Leu Val Val Ala Lys Val
   1175 1180 1185
   Glu Lys Gly Lys Ser Lys Lys Leu Lys Ser Val Lys Glu Leu Leu
   1190 1195 1200
   Gly Ile Thr Ile Met Glu Arg Ser Ser Phe Glu Lys Asn Pro Ile
   1205 1210 1215
   Asp Phe Leu Glu Ala Lys Gly Tyr Lys Glu Val Lys Lys Asp Leu
   1220 1225 1230
   Ile Ile Lys Leu Pro Lys Tyr Ser Leu Phe Glu Leu Glu Asn Gly
   1235 1240 1245
   Arg Lys Arg Met Leu Ala Ser Ala Gly Glu Leu Gln Lys Gly Asn
   1250 1255 1260
   Glu Leu Ala Leu Pro Ser Lys Tyr Val Asn Phe Leu Tyr Leu Ala
   1265 1270 1275
   Ser His Tyr Glu Lys Leu Lys Gly Ser Pro Glu Asp Asn Glu Gln
   1280 1285 1290
   Lys Gln Leu Phe Val Glu Gln His Lys His Tyr Leu Asp Glu Ile
   1295 1300 1305
   Ile Glu Gln Ile Ser Glu Phe Ser Lys Arg Val Ile Leu Ala Asp
   1310 1315 1320
   Ala Asn Leu Asp Lys Val Leu Ser Ala Tyr Asn Lys His Arg Asp
   1325 1330 1335
   Lys Pro Ile Arg Glu Gln Ala Glu Asn Ile Ile His Leu Phe Thr
   1340 1345 1350
   Leu Thr Asn Leu Gly Ala Pro Ala Ala Phe Lys Tyr Phe Asp Thr
   1355 1360 1365
   Thr Ile Asp Arg Lys Arg Tyr Thr Ser Thr Lys Glu Val Leu Asp
   1370 1375 1380
   Ala Thr Leu Ile His Gln Ser Ile Thr Gly Leu Tyr Glu Thr Arg
   1385 1390 1395
   Ile Asp Leu Ser Gln Leu Gly Gly Asp Ala Ala Ala Val Ser Lys
   1400 1405 1410
   Gly Glu Glu Leu Phe Thr Gly Val Val Pro Ile Leu Val Glu Leu
   1415 1420 1425
   Asp Gly Asp Val Asn Gly His Lys Phe Ser Val Ser Gly Glu Gly
   1430 1435 1440
   Glu Gly Asp Ala Thr Tyr Gly Lys Leu Thr Leu Lys Phe Ile Cys
   1445 1450 1455
   Thr Thr Gly Lys Leu Pro Val Pro Trp Pro Thr Leu Val Thr Thr
   1460 1465 1470
   Leu Thr Tyr Gly Val Gln Cys Phe Ser Arg Tyr Pro Asp His Met
   1475 1480 1485
   Lys Gln His Asp Phe Phe Lys Ser Ala Met Pro Glu Gly Tyr Val
   1490 1495 1500
   Gln Glu Arg Thr Ile Phe Phe Lys Asp Asp Gly Asn Tyr Lys Thr
   1505 1510 1515
   Arg Ala Glu Val Lys Phe Glu Gly Asp Thr Leu Val Asn Arg Ile
   1520 1525 1530
   Glu Leu Lys Gly Ile Asp Phe Lys Glu Asp Gly Asn Ile Leu Gly
   1535 1540 1545
   His Lys Leu Glu Tyr Asn Tyr Asn Ser His Asn Val Tyr Ile Met
   1550 1555 1560
   Ala Asp Lys Gln Lys Asn Gly Ile Lys Val Asn Phe Lys Ile Arg
   1565 1570 1575
   His Asn Ile Glu Asp Gly Ser Val Gln Leu Ala Asp His Tyr Gln
   1580 1585 1590
   Gln Asn Thr Pro Ile Gly Asp Gly Pro Val Leu Leu Pro Asp Asn
   1595 1600 1605
   His Tyr Leu Ser Thr Gln Ser Ala Leu Ser Lys Asp Pro Asn Glu
   1610 1615 1620
   Lys Arg Asp His Met Val Leu Leu Glu Phe Val Thr Ala Ala Gly
   1625 1630 1635
   Ile Thr Leu Gly Met Asp Glu Leu Tyr Lys Lys Arg Pro Ala Ala
   1640 1645 1650
   Thr Lys Lys Ala Gly Gln Ala Lys Lys Lys Lys
   1655 1660
<210> 46
   <211> 1423
   <212> PRT
   <213> Artificial Sequence
<220>
   <221> source
   <223> /note="Description of Artificial Sequence: Synthetic polypeptide"
<400> 46
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Gly Val Pro Ala Ala Asp Lys Lys Tyr Ser Ile Gly Leu
           35                  40                  45
   Asp Ile Gly Thr Asn Ser Val Gly Trp Ala Val Ile Thr Asp Glu Tyr
       50                  55                  60
   Lys Val Pro Ser Lys Lys Phe Lys Val Leu Gly Asn Thr Asp Arg His
   65 70 75 80
   Ser Ile Lys Lys Asn Leu Ile Gly Ala Leu Leu Phe Asp Ser Gly Glu
   85 90 95
   Thr Ala Glu Ala Thr Arg Leu Lys Arg Thr Ala Arg Arg Arg Tyr Thr
   100 105 110
   Arg Arg Lys Asn Arg Ile Cys Tyr Leu Gln Glu Ile Phe Ser Asn Glu
   115 120 125
   Met Ala Lys Val Asp Asp Ser Phe Phe His Arg Leu Glu Glu Ser Phe
   130 135 140
   Leu Val Glu Glu Asp Lys Lys His Glu Arg His Pro Ile Phe Gly Asn
   145 150 155 160
   Ile Val Asp Glu Val Ala Tyr His Glu Lys Tyr Pro Thr Ile Tyr His
   165 170 175
   Leu Arg Lys Lys Leu Val Asp Ser Thr Asp Lys Ala Asp Leu Arg Leu
   180 185 190
   Ile Tyr Leu Ala Leu Ala His Met Ile Lys Phe Arg Gly His Phe Leu
   195 200 205
   Ile Glu Gly Asp Leu Asn Pro Asp Asn Ser Asp Val Asp Lys Leu Phe
   210 215 220
   Ile Gln Leu Val Gln Thr Tyr Asn Gln Leu Phe Glu Glu Asn Pro Ile
   225 230 235 240
   Asn Ala Ser Gly Val Asp Ala Lys Ala Ile Leu Ser Ala Arg Leu Ser
   245 250 255
   Lys Ser Arg Arg Leu Glu Asn Leu Ile Ala Gln Leu Pro Gly Glu Lys
   260 265 270
   Lys Asn Gly Leu Phe Gly Asn Leu Ile Ala Leu Ser Leu Gly Leu Thr
   275 280 285
   Pro Asn Phe Lys Ser Asn Phe Asp Leu Ala Glu Asp Ala Lys Leu Gln
   290 295 300
   Leu Ser Lys Asp Thr Tyr Asp Asp Asp Leu Asp Asn Leu Leu Ala Gln
   305 310 315 320
   Ile Gly Asp Gln Tyr Ala Asp Leu Phe Leu Ala Ala Lys Asn Leu Ser
                   325                 330                 335
   Asp Ala Ile Leu Leu Ser Asp Ile Leu Arg Val Asn Thr Glu Ile Thr
               340                 345                 350
   Lys Ala Pro Leu Ser Ala Ser Met Ile Lys Arg Tyr Asp Glu His His
           355                 360                 365
   Gln Asp Leu Thr Leu Leu Lys Ala Leu Val Arg Gln Gln Leu Pro Glu
       370                 375                 380
   Lys Tyr Lys Glu Ile Phe Phe Asp Gln Ser Lys Asn Gly Tyr Ala Gly
   385                 390                 395                 400
   Tyr Ile Asp Gly Gly Ala Ser Gln Glu Glu Phe Tyr Lys Phe Ile Lys
                   405                 410                 415
   Pro Ile Leu Glu Lys Met Asp Gly Thr Glu Glu Leu Leu Val Lys Leu
               420                 425                 430
   Asn Arg Glu Asp Leu Leu Arg Lys Gln Arg Thr Phe Asp Asn Gly Ser
           435                 440                 445
   Ile Pro His Gln Ile His Leu Gly Glu Leu His Ala Ile Leu Arg Arg
       450                 455                 460
   Gln Glu Asp Phe Tyr Pro Phe Leu Lys Asp Asn Arg Glu Lys Ile Glu
   465                 470                 475                 480
   Lys Ile Leu Thr Phe Arg Ile Pro Tyr Tyr Val Gly Pro Leu Ala Arg
                   485                 490                 495
   Gly Asn Ser Arg Phe Ala Trp Met Thr Arg Lys Ser Glu Glu Thr Ile
               500                 505                 510
   Thr Pro Trp Asn Phe Glu Glu Val Val Asp Lys Gly Ala Ser Ala Gln
           515                 520                 525
   Ser Phe Ile Glu Arg Met Thr Asn Phe Asp Lys Asn Leu Pro Asn Glu
       530                 535                 540
   Lys Val Leu Pro Lys His Ser Leu Leu Tyr Glu Tyr Phe Thr Val Tyr
   545                 550                 555                 560
   Asn Glu Leu Thr Lys Val Lys Tyr Val Thr Glu Gly Met Arg Lys Pro
                   565                 570                 575
   Ala Phe Leu Ser Gly Glu Gln Lys Lys Ala Ile Val Asp Leu Leu Phe
   580 585 590
   Lys Thr Asn Arg Lys Val Thr Val Lys Gln Leu Lys Glu Asp Tyr Phe
   595 600 605
   Lys Lys Ile Glu Cys Phe Asp Ser Val Glu Ile Ser Gly Val Glu Asp
   610 615 620
   Arg Phe Asn Ala Ser Leu Gly Thr Tyr His Asp Leu Leu Lys Ile Ile
   625 630 635 640
   Lys Asp Lys Asp Phe Leu Asp Asn Glu Glu Asn Glu Asp Ile Leu Glu
   645 650 655
   Asp Ile Val Leu Thr Leu Thr Leu Phe Glu Asp Arg Glu Met Ile Glu
   660 665 670
   Glu Arg Leu Lys Thr Tyr Ala His Leu Phe Asp Asp Lys Val Met Lys
   675 680 685
   Gln Leu Lys Arg Arg Arg Tyr Thr Gly Trp Gly Arg Leu Ser Arg Lys
   690 695 700
   Leu Ile Asn Gly Ile Arg Asp Lys Gln Ser Gly Lys Thr Ile Leu Asp
   705 710 715 720
   Phe Leu Lys Ser Asp Gly Phe Ala Asn Arg Asn Phe Met Gln Leu Ile
   725 730 735
   His Asp Asp Ser Leu Thr Phe Lys Glu Asp Ile Gln Lys Ala Gln Val
   740 745 750
   Ser Gly Gln Gly Asp Ser Leu His Glu His Ile Ala Asn Leu Ala Gly
   755 760 765
   Ser Pro Ala Ile Lys Lys Gly Ile Leu Gln Thr Val Lys Val Val Asp
   770 775 780
   Glu Leu Val Lys Val Met Gly Arg His Lys Pro Glu Asn Ile Val Ile
   785 790 795 800
   Glu Met Ala Arg Glu Asn Gln Thr Thr Gln Lys Gly Gln Lys Asn Ser
   805 810 815
   Arg Glu Arg Met Lys Arg Ile Glu Glu Gly Ile Lys Glu Leu Gly Ser
   820 825 830
   Gln Ile Leu Lys Glu His Pro Val Glu Asn Thr Gln Leu Gln Asn Glu
   835 840 845
   Lys Leu Tyr Leu Tyr Tyr Leu Gln Asn Gly Arg Asp Met Tyr Val Asp
   850 855 860
   Gln Glu Leu Asp Ile Asn Arg Leu Ser Asp Tyr Asp Val Asp His Ile
   865 870 875 880
   Val Pro Gln Ser Phe Leu Lys Asp Asp Ser Ile Asp Asn Lys Val Leu
   885 890 895
   Thr Arg Ser Asp Lys Asn Arg Gly Lys Ser Asp Asn Val Pro Ser Glu
   900 905 910
   Glu Val Val Lys Lys Met Lys Asn Tyr Trp Arg Gln Leu Leu Asn Ala
   915 920 925
   Lys Leu Ile Thr Gln Arg Lys Phe Asp Asn Leu Thr Lys Ala Glu Arg
   930 935 940
   Gly Gly Leu Ser Glu Leu Asp Lys Ala Gly Phe Ile Lys Arg Gln Leu
   945 950 955 960
   Val Glu Thr Arg Gln Ile Thr Lys His Val Ala Gln Ile Leu Asp Ser
   965 970 975
   Arg Met Asn Thr Lys Tyr Asp Glu Asn Asp Lys Leu Ile Arg Glu Val
   980 985 990
   Lys Val Ile Thr Leu Lys Ser Lys Leu Val Ser Asp Phe Arg Lys Asp
   995 1000 1005
   Phe Gln Phe Tyr Lys Val Arg Glu Ile Asn Asn Tyr His His Ala
   1010 1015 1020
   His Asp Ala Tyr Leu Asn Ala Val Val Gly Thr Ala Leu Ile Lys
   1025 1030 1035
   Lys Tyr Pro Lys Leu Glu Ser Glu Phe Val Tyr Gly Asp Tyr Lys
   1040 1045 1050
   Val Tyr Asp Val Arg Lys Met Ile Ala Lys Ser Glu Gln Glu Ile
   1055 1060 1065
   Gly Lys Ala Thr Ala Lys Tyr Phe Phe Tyr Ser Asn Ile Met Asn
   1070 1075 1080
   Phe Phe Lys Thr Glu Ile Thr Leu Ala Asn Gly Glu Ile Arg Lys
   1085 1090 1095
   Arg Pro Leu Ile Glu Thr Asn Gly Glu Thr Gly Glu Ile Val Trp
   1100 1105 1110
   Asp Lys Gly Arg Asp Phe Ala Thr Val Arg Lys Val Leu Ser Met
   1115 1120 1125
   Pro Gln Val Asn Ile Val Lys Lys Thr Glu Val Gln Thr Gly Gly
   1130 1135 1140
   Phe Ser Lys Glu Ser Ile Leu Pro Lys Arg Asn Ser Asp Lys Leu
   1145 1150 1155
   Ile Ala Arg Lys Lys Asp Trp Asp Pro Lys Lys Tyr Gly Gly Phe
   1160 1165 1170
   Asp Ser Pro Thr Val Ala Tyr Ser Val Leu Val Val Ala Lys Val
   1175 1180 1185
   Glu Lys Gly Lys Ser Lys Lys Leu Lys Ser Val Lys Glu Leu Leu
   1190 1195 1200
   Gly Ile Thr Ile Met Glu Arg Ser Ser Phe Glu Lys Asn Pro Ile
   1205 1210 1215
   Asp Phe Leu Glu Ala Lys Gly Tyr Lys Glu Val Lys Lys Asp Leu
   1220 1225 1230
   Ile Ile Lys Leu Pro Lys Tyr Ser Leu Phe Glu Leu Glu Asn Gly
   1235 1240 1245
   Arg Lys Arg Met Leu Ala Ser Ala Gly Glu Leu Gln Lys Gly Asn
   1250 1255 1260
   Glu Leu Ala Leu Pro Ser Lys Tyr Val Asn Phe Leu Tyr Leu Ala
   1265 1270 1275
   Ser His Tyr Glu Lys Leu Lys Gly Ser Pro Glu Asp Asn Glu Gln
   1280 1285 1290
   Lys Gln Leu Phe Val Glu Gln His Lys His Tyr Leu Asp Glu Ile
   1295 1300 1305
   Ile Glu Gln Ile Ser Glu Phe Ser Lys Arg Val Ile Leu Ala Asp
   1310 1315 1320
   Ala Asn Leu Asp Lys Val Leu Ser Ala Tyr Asn Lys His Arg Asp
   1325 1330 1335
   Lys Pro Ile Arg Glu Gln Ala Glu Asn Ile Ile His Leu Phe Thr
   1340 1345 1350
   Leu Thr Asn Leu Gly Ala Pro Ala Ala Phe Lys Tyr Phe Asp Thr
   1355 1360 1365
   Thr Ile Asp Arg Lys Arg Tyr Thr Ser Thr Lys Glu Val Leu Asp
   1370 1375 1380
   Ala Thr Leu Ile His Gln Ser Ile Thr Gly Leu Tyr Glu Thr Arg
   1385 1390 1395
   Ile Asp Leu Ser Gln Leu Gly Gly Asp Lys Arg Pro Ala Ala Thr
   1400 1405 1410
   Lys Lys Ala Gly Gln Ala Lys Lys Lys Lys
   1415 1420
<210> 47
   <211> 483
   <212> PRT
   <213> Artificial Sequence
<220>
   <221> source
   <223> /note="Description of Artificial Sequence: Synthetic polypeptide"
<400> 47
   Met Phe Leu Phe Leu Ser Leu Thr Ser Phe Leu Ser Ser Ser Arg Thr
   1 5 10 15
   Leu Val Ser Lys Gly Glu Glu Asp Asn Met Ala Ile Ile Lys Glu Phe
   20 25 30
   Met Arg Phe Lys Val His Met Glu Gly Ser Val Asn Gly His Glu Phe
   35 40 45
   Glu Ile Glu Gly Glu Gly Glu Gly Arg Pro Tyr Glu Gly Thr Gln Thr
   50 55 60
   Ala Lys Leu Lys Val Thr Lys Gly Gly Pro Leu Pro Phe Ala Trp Asp
   65 70 75 80
   Ile Leu Ser Pro Gln Phe Met Tyr Gly Ser Lys Ala Tyr Val Lys His
   85 90 95
   Pro Ala Asp Ile Pro Asp Tyr Leu Lys Leu Ser Phe Pro Glu Gly Phe
   100 105 110
   Lys Trp Glu Arg Val Met Asn Phe Glu Asp Gly Gly Val Val Thr Val
   115 120 125
   Thr Gln Asp Ser Ser Leu Gln Asp Gly Glu Phe Ile Tyr Lys Val Lys
   130 135 140
   Leu Arg Gly Thr Asn Phe Pro Ser Asp Gly Pro Val Met Gln Lys Lys
   145 150 155 160
   Thr Met Gly Trp Glu Ala Ser Ser Glu Arg Met Tyr Pro Glu Asp Gly
   165 170 175
   Ala Leu Lys Gly Glu Ile Lys Gln Arg Leu Lys Leu Lys Asp Gly Gly
   180 185 190
   His Tyr Asp Ala Glu Val Lys Thr Thr Tyr Lys Ala Lys Lys Pro Val
   195 200 205
   Gln Leu Pro Gly Ala Tyr Asn Val Asn Ile Lys Leu Asp Ile Thr Ser
   210 215 220
   His Asn Glu Asp Tyr Thr Ile Val Glu Gln Tyr Glu Arg Ala Glu Gly
   225 230 235 240
   Arg His Ser Thr Gly Gly Met Asp Glu Leu Tyr Lys Gly Ser Lys Gln
   245 250 255
   Leu Glu Glu Leu Leu Ser Thr Ser Phe Asp Ile Gln Phe Asn Asp Leu
   260 265 270
   Thr Leu Leu Glu Thr Ala Phe Thr His Thr Ser Tyr Ala Asn Glu His
   275 280 285
   Arg Leu Leu Asn Val Ser His Asn Glu Arg Leu Glu Phe Leu Gly Asp
   290 295 300
   Ala Val Leu Gln Leu Ile Ile Ser Glu Tyr Leu Phe Ala Lys Tyr Pro
   305 310 315 320
   Lys Lys Thr Glu Gly Asp Met Ser Lys Leu Arg Ser Met Ile Val Arg
   325 330 335
   Glu Glu Ser Leu Ala Gly Phe Ser Arg Phe Cys Ser Phe Asp Ala Tyr
   340 345 350
   Ile Lys Leu Gly Lys Gly Glu Glu Lys Ser Gly Gly Arg Arg Arg Asp
   355 360 365
   Thr Ile Leu Gly Asp Leu Phe Glu Ala Phe Leu Gly Ala Leu Leu Leu
   370 375 380
   Asp Lys Gly Ile Asp Ala Val Arg Arg Phe Leu Lys Gln Val Met Ile
   385 390 395 400
   Pro Gln Val Glu Lys Gly Asn Phe Glu Arg Val Lys Asp Tyr Lys Thr
   405 410 415
   Cys Leu Gln Glu Phe Leu Gln Thr Lys Gly Asp Val Ala Ile Asp Tyr
   420 425 430
   Gln Val Ile Ser Glu Lys Gly Pro Ala His Ala Lys Gln Phe Glu Val
   435 440 445
   Ser Ile Val Val Asn Gly Ala Val Leu Ser Lys Gly Leu Gly Lys Ser
   450 455 460
   Lys Lys Leu Ala Glu Gln Asp Ala Ala Lys Asn Ala Leu Ala Gln Leu
   465 470 475 480
   Ser Glu Val
<210> 48
   <211> 483
   <212> PRT
   <213> Artificial Sequence
<220>
   <221> source
   <223> /note="Description of Artificial Sequence: Synthetic polypeptide"
<400> 48
   Met Lys Gln Leu Glu Glu Leu Leu Ser Thr Ser Phe Asp Ile Gln Phe
   1 5 10 15
   Asn Asp Leu Thr Leu Leu Glu Thr Ala Phe Thr His Thr Ser Tyr Ala
   20 25 30
   Asn Glu His Arg Leu Leu Asn Val Ser His Asn Glu Arg Leu Glu Phe
   35 40 45
   Leu Gly Asp Ala Val Leu Gln Leu Ile Ile Ser Glu Tyr Leu Phe Ala
   50 55 60
   Lys Tyr Pro Lys Lys Thr Glu Gly Asp Met Ser Lys Leu Arg Ser Met
   65 70 75 80
   Ile Val Arg Glu Glu Ser Leu Ala Gly Phe Ser Arg Phe Cys Ser Phe
   85 90 95
   Asp Ala Tyr Ile Lys Leu Gly Lys Gly Glu Glu Lys Ser Gly Gly Arg
   100 105 110
   Arg Arg Asp Thr Ile Leu Gly Asp Leu Phe Glu Ala Phe Leu Gly Ala
   115 120 125
   Leu Leu Leu Asp Lys Gly Ile Asp Ala Val Arg Arg Phe Leu Lys Gln
   130 135 140
   Val Met Ile Pro Gln Val Glu Lys Gly Asn Phe Glu Arg Val Lys Asp
   145 150 155 160
   Tyr Lys Thr Cys Leu Gln Glu Phe Leu Gln Thr Lys Gly Asp Val Ala
   165 170 175
   Ile Asp Tyr Gln Val Ile Ser Glu Lys Gly Pro Ala His Ala Lys Gln
   180 185 190
   Phe Glu Val Ser Ile Val Val Asn Gly Ala Val Leu Ser Lys Gly Leu
   195 200 205
   Gly Lys Ser Lys Lys Leu Ala Glu Gln Asp Ala Ala Lys Asn Ala Leu
   210 215 220
   Ala Gln Leu Ser Glu Val Gly Ser Val Ser Lys Gly Glu Glu Asp Asn
   225 230 235 240
   Met Ala Ile Ile Lys Glu Phe Met Arg Phe Lys Val His Met Glu Gly
   245 250 255
   Ser Val Asn Gly His Glu Phe Glu Ile Glu Gly Glu Gly Glu Gly Arg
   260 265 270
   Pro Tyr Glu Gly Thr Gln Thr Ala Lys Leu Lys Val Thr Lys Gly Gly
   275 280 285
   Pro Leu Pro Phe Ala Trp Asp Ile Leu Ser Pro Gln Phe Met Tyr Gly
   290 295 300
   Ser Lys Ala Tyr Val Lys His Pro Ala Asp Ile Pro Asp Tyr Leu Lys
   305 310 315 320
   Leu Ser Phe Pro Glu Gly Phe Lys Trp Glu Arg Val Met Asn Phe Glu
   325 330 335
   Asp Gly Gly Val Val Thr Val Thr Gln Asp Ser Ser Leu Gln Asp Gly
   340 345 350
   Glu Phe Ile Tyr Lys Val Lys Leu Arg Gly Thr Asn Phe Pro Ser Asp
   355 360 365
   Gly Pro Val Met Gln Lys Lys Thr Met Gly Trp Glu Ala Ser Ser Glu
   370 375 380
   Arg Met Tyr Pro Glu Asp Gly Ala Leu Lys Gly Glu Ile Lys Gln Arg
   385 390 395 400
   Leu Lys Leu Lys Asp Gly Gly His Tyr Asp Ala Glu Val Lys Thr Thr
   405 410 415
   Tyr Lys Ala Lys Lys Pro Val Gln Leu Pro Gly Ala Tyr Asn Val Asn
   420 425 430
   Ile Lys Leu Asp Ile Thr Ser His Asn Glu Asp Tyr Thr Ile Val Glu
   435 440 445
   Gln Tyr Glu Arg Ala Glu Gly Arg His Ser Thr Gly Gly Met Asp Glu
   450 455 460
   Leu Tyr Lys Lys Arg Pro Ala Ala Thr Lys Lys Ala Gly Gln Ala Lys
   465 470 475 480
   Lys Lys Lys
<210> 49
   <211> 1423
   <212> PRT
   <213> Artificial Sequence
<220>
   <221> source
   <223> /note="Description of Artificial Sequence: Synthetic polypeptide"
<400> 49
   Met Asp Tyr Lys Asp His Asp Gly Asp Tyr Lys Asp His Asp Ile Asp
   1 5 10 15
   Tyr Lys Asp Asp Asp Asp Lys Met Ala Pro Lys Lys Lys Arg Lys Val
               20                  25                  30
   Gly Ile His Gly Val Pro Ala Ala Asp Lys Lys Tyr Ser Ile Gly Leu
           35                  40                  45
   Ala Ile Gly Thr Asn Ser Val Gly Trp Ala Val Ile Thr Asp Glu Tyr
       50                  55                  60
   Lys Val Pro Ser Lys Lys Phe Lys Val Leu Gly Asn Thr Asp Arg His
   65                  70                  75                  80
   Ser Ile Lys Lys Asn Leu Ile Gly Ala Leu Leu Phe Asp Ser Gly Glu
                   85                  90                  95
   Thr Ala Glu Ala Thr Arg Leu Lys Arg Thr Ala Arg Arg Arg Tyr Thr
               100                 105                 110
   Arg Arg Lys Asn Arg Ile Cys Tyr Leu Gln Glu Ile Phe Ser Asn Glu
           115                 120                 125
   Met Ala Lys Val Asp Asp Ser Phe Phe His Arg Leu Glu Glu Ser Phe
       130                 135                 140
   Leu Val Glu Glu Asp Lys Lys His Glu Arg His Pro Ile Phe Gly Asn
   145                 150                 155                 160
   Ile Val Asp Glu Val Ala Tyr His Glu Lys Tyr Pro Thr Ile Tyr His
                   165                 170                 175
   Leu Arg Lys Lys Leu Val Asp Ser Thr Asp Lys Ala Asp Leu Arg Leu
               180                 185                 190
   Ile Tyr Leu Ala Leu Ala His Met Ile Lys Phe Arg Gly His Phe Leu
           195                 200                 205
   Ile Glu Gly Asp Leu Asn Pro Asp Asn Ser Asp Val Asp Lys Leu Phe
       210                 215                 220
   Ile Gln Leu Val Gln Thr Tyr Asn Gln Leu Phe Glu Glu Asn Pro Ile
   225                 230                 235                 240
   Asn Ala Ser Gly Val Asp Ala Lys Ala Ile Leu Ser Ala Arg Leu Ser
                   245                 250                 255
   Lys Ser Arg Arg Leu Glu Asn Leu Ile Ala Gln Leu Pro Gly Glu Lys
   260 265 270
   Lys Asn Gly Leu Phe Gly Asn Leu Ile Ala Leu Ser Leu Gly Leu Thr
   275 280 285
   Pro Asn Phe Lys Ser Asn Phe Asp Leu Ala Glu Asp Ala Lys Leu Gln
   290 295 300
   Leu Ser Lys Asp Thr Tyr Asp Asp Asp Leu Asp Asn Leu Leu Ala Gln
   305 310 315 320
   Ile Gly Asp Gln Tyr Ala Asp Leu Phe Leu Ala Ala Lys Asn Leu Ser
   325 330 335
   Asp Ala Ile Leu Leu Ser Asp Ile Leu Arg Val Asn Thr Glu Ile Thr
   340 345 350
   Lys Ala Pro Leu Ser Ala Ser Met Ile Lys Arg Tyr Asp Glu His His
   355 360 365
   Gln Asp Leu Thr Leu Leu Lys Ala Leu Val Arg Gln Gln Leu Pro Glu
   370 375 380
   Lys Tyr Lys Glu Ile Phe Phe Asp Gln Ser Lys Asn Gly Tyr Ala Gly
   385 390 395 400
   Tyr Ile Asp Gly Gly Ala Ser Gln Glu Glu Phe Tyr Lys Phe Ile Lys
   405 410 415
   Pro Ile Leu Glu Lys Met Asp Gly Thr Glu Glu Leu Leu Val Lys Leu
   420 425 430
   Asn Arg Glu Asp Leu Leu Arg Lys Gln Arg Thr Phe Asp Asn Gly Ser
   435 440 445
   Ile Pro His Gln Ile His Leu Gly Glu Leu His Ala Ile Leu Arg Arg
   450 455 460
   Gln Glu Asp Phe Tyr Pro Phe Leu Lys Asp Asn Arg Glu Lys Ile Glu
   465 470 475 480
   Lys Ile Leu Thr Phe Arg Ile Pro Tyr Tyr Val Gly Pro Leu Ala Arg
   485 490 495
   Gly Asn Ser Arg Phe Ala Trp Met Thr Arg Lys Ser Glu Glu Thr Ile
   500 505 510
   Thr Pro Trp Asn Phe Glu Glu Val Val Asp Lys Gly Ala Ser Ala Gln
   515 520 525
   Ser Phe Ile Glu Arg Met Thr Asn Phe Asp Lys Asn Leu Pro Asn Glu
   530 535 540
   Lys Val Leu Pro Lys His Ser Leu Leu Tyr Glu Tyr Phe Thr Val Tyr
   545 550 555 560
   Asn Glu Leu Thr Lys Val Lys Tyr Val Thr Glu Gly Met Arg Lys Pro
   565 570 575
   Ala Phe Leu Ser Gly Glu Gln Lys Lys Ala Ile Val Asp Leu Leu Phe
   580 585 590
   Lys Thr Asn Arg Lys Val Thr Val Lys Gln Leu Lys Glu Asp Tyr Phe
   595 600 605
   Lys Lys Ile Glu Cys Phe Asp Ser Val Glu Ile Ser Gly Val Glu Asp
   610 615 620
   Arg Phe Asn Ala Ser Leu Gly Thr Tyr His Asp Leu Leu Lys Ile Ile
   625 630 635 640
   Lys Asp Lys Asp Phe Leu Asp Asn Glu Glu Asn Glu Asp Ile Leu Glu
   645 650 655
   Asp Ile Val Leu Thr Leu Thr Leu Phe Glu Asp Arg Glu Met Ile Glu
   660 665 670
   Glu Arg Leu Lys Thr Tyr Ala His Leu Phe Asp Asp Lys Val Met Lys
   675 680 685
   Gln Leu Lys Arg Arg Arg Tyr Thr Gly Trp Gly Arg Leu Ser Arg Lys
   690 695 700
   Leu Ile Asn Gly Ile Arg Asp Lys Gln Ser Gly Lys Thr Ile Leu Asp
   705 710 715 720
   Phe Leu Lys Ser Asp Gly Phe Ala Asn Arg Asn Phe Met Gln Leu Ile
   725 730 735
   His Asp Asp Ser Leu Thr Phe Lys Glu Asp Ile Gln Lys Ala Gln Val
   740 745 750
   Ser Gly Gln Gly Asp Ser Leu His Glu His Ile Ala Asn Leu Ala Gly
   755 760 765
   Ser Pro Ala Ile Lys Lys Gly Ile Leu Gln Thr Val Lys Val Val Asp
   770 775 780
   Glu Leu Val Lys Val Met Gly Arg His Lys Pro Glu Asn Ile Val Ile
   785 790 795 800
   Glu Met Ala Arg Glu Asn Gln Thr Thr Gln Lys Gly Gln Lys Asn Ser
   805 810 815
   Arg Glu Arg Met Lys Arg Ile Glu Glu Gly Ile Lys Glu Leu Gly Ser
   820 825 830
   Gln Ile Leu Lys Glu His Pro Val Glu Asn Thr Gln Leu Gln Asn Glu
   835 840 845
   Lys Leu Tyr Leu Tyr Tyr Leu Gln Asn Gly Arg Asp Met Tyr Val Asp
   850 855 860
   Gln Glu Leu Asp Ile Asn Arg Leu Ser Asp Tyr Asp Val Asp His Ile
   865 870 875 880
   Val Pro Gln Ser Phe Leu Lys Asp Asp Ser Ile Asp Asn Lys Val Leu
   885 890 895
   Thr Arg Ser Asp Lys Asn Arg Gly Lys Ser Asp Asn Val Pro Ser Glu
   900 905 910
   Glu Val Val Lys Lys Met Lys Asn Tyr Trp Arg Gln Leu Leu Asn Ala
   915 920 925
   Lys Leu Ile Thr Gln Arg Lys Phe Asp Asn Leu Thr Lys Ala Glu Arg
   930 935 940
   Gly Gly Leu Ser Glu Leu Asp Lys Ala Gly Phe Ile Lys Arg Gln Leu
   945 950 955 960
   Val Glu Thr Arg Gln Ile Thr Lys His Val Ala Gln Ile Leu Asp Ser
   965 970 975
   Arg Met Asn Thr Lys Tyr Asp Glu Asn Asp Lys Leu Ile Arg Glu Val
   980 985 990
   Lys Val Ile Thr Leu Lys Ser Lys Leu Val Ser Asp Phe Arg Lys Asp
   995 1000 1005
   Phe Gln Phe Tyr Lys Val Arg Glu Ile Asn Asn Tyr His His Ala
   1010 1015 1020
   His Asp  Ala Tyr Leu Asn Ala  Val Val Gly Thr Ala  Leu Ile Lys
       1025                 1030                 1035
   Lys Tyr  Pro Lys Leu Glu Ser  Glu Phe Val Tyr Gly  Asp Tyr Lys
       1040                 1045                 1050
   Val Tyr  Asp Val Arg Lys Met  Ile Ala Lys Ser Glu  Gln Glu Ile
       1055                 1060                 1065
   Gly Lys  Ala Thr Ala Lys Tyr  Phe Phe Tyr Ser Asn  Ile Met Asn
       1070                 1075                 1080
   Phe Phe  Lys Thr Glu Ile Thr  Leu Ala Asn Gly Glu  Ile Arg Lys
       1085                 1090                 1095
   Arg Pro  Leu Ile Glu Thr Asn  Gly Glu Thr Gly Glu  Ile Val Trp
       1100                 1105                 1110
   Asp Lys  Gly Arg Asp Phe Ala  Thr Val Arg Lys Val  Leu Ser Met
       1115                 1120                 1125
   Pro Gln  Val Asn Ile Val Lys  Lys Thr Glu Val Gln  Thr Gly Gly
       1130                 1135                 1140
   Phe Ser  Lys Glu Ser Ile Leu  Pro Lys Arg Asn Ser  Asp Lys Leu
       1145                 1150                 1155
   Ile Ala  Arg Lys Lys Asp Trp  Asp Pro Lys Lys Tyr  Gly Gly Phe
       1160                 1165                 1170
   Asp Ser  Pro Thr Val Ala Tyr  Ser Val Leu Val Val  Ala Lys Val
       1175                 1180                 1185
   Glu Lys  Gly Lys Ser Lys Lys  Leu Lys Ser Val Lys  Glu Leu Leu
       1190                 1195                 1200
   Gly Ile  Thr Ile Met Glu Arg  Ser Ser Phe Glu Lys  Asn Pro Ile
       1205                 1210                 1215
   Asp Phe  Leu Glu Ala Lys Gly  Tyr Lys Glu Val Lys  Lys Asp Leu
       1220                 1225                 1230
   Ile Ile  Lys Leu Pro Lys Tyr  Ser Leu Phe Glu Leu  Glu Asn Gly
       1235                 1240                 1245
   Arg Lys  Arg Met Leu Ala Ser  Ala Gly Glu Leu Gln  Lys Gly Asn
   1250 1255 1260
   Glu Leu Ala Leu Pro Ser Lys Tyr Val Asn Phe Leu Tyr Leu Ala
   1265 1270 1275
   Ser His Tyr Glu Lys Leu Lys Gly Ser Pro Glu Asp Asn Glu Gln
   1280 1285 1290
   Lys Gln Leu Phe Val Glu Gln His Lys His Tyr Leu Asp Glu Ile
   1295 1300 1305
   Ile Glu Gln Ile Ser Glu Phe Ser Lys Arg Val Ile Leu Ala Asp
   1310 1315 1320
   Ala Asn Leu Asp Lys Val Leu Ser Ala Tyr Asn Lys His Arg Asp
   1325 1330 1335
   Lys Pro Ile Arg Glu Gln Ala Glu Asn Ile Ile His Leu Phe Thr
   1340 1345 1350
   Leu Thr Asn Leu Gly Ala Pro Ala Ala Phe Lys Tyr Phe Asp Thr
   1355 1360 1365
   Thr Ile Asp Arg Lys Arg Tyr Thr Ser Thr Lys Glu Val Leu Asp
   1370 1375 1380
   Ala Thr Leu Ile His Gln Ser Ile Thr Gly Leu Tyr Glu Thr Arg
   1385 1390 1395
   Ile Asp Leu Ser Gln Leu Gly Gly Asp Lys Arg Pro Ala Ala Thr
   1400 1405 1410
   Lys Lys Ala Gly Gln Ala Lys Lys Lys Lys
   1415 1420
<210> 50
   <211> 2012
   <212> DNA
   <213> Artificial Sequence
<220>
   <221> source
   <223> /note="Description of Artificial Sequence: Synthetic polynucleotide"
<400> 50
   gaatgctgcc ctcagacccg cttcctccct gtccttgtct gtccaaggag aatgaggtct      60
   cactggtgga tttcggacta ccctgaggag ctggcacctg agggacaagg ccccccacct     120
   gcccagctcc agcctctgat gaggggtggg agagagctac atgaggttgc taagaaagcc     180
   tcccctgaag gagaccacac agtgtgtgag gttggagtct ctagcagcgg gttctgtgcc 240
   cccagggata gtctggctgt ccaggcactg ctcttgatat aaacaccacc tcctagttat 300
   gaaaccatgc ccattctgcc tctctgtatg gaaaagagca tggggctggc ccgtggggtg 360
   gtgtccactt taggccctgt gggagatcat gggaacccac gcagtgggtc ataggctctc 420
   tcatttacta ctcacatcca ctctgtgaag aagcgattat gatctctcct ctagaaactc 480
   gtagagtccc atgtctgccg gcttccagag cctgcactcc tccaccttgg cttggctttg 540
   ctggggctag aggagctagg atgcacagca gctctgtgac cctttgtttg agaggaacag 600
   gaaaaccacc cttctctctg gcccactgtg tcctcttcct gccctgccat ccccttctgt 660
   gaatgttaga cccatgggag cagctggtca gaggggaccc cggcctgggg cccctaaccc 720
   tatgtagcct cagtcttccc atcaggctct cagctcagcc tgagtgttga ggccccagtg 780
   gctgctctgg gggcctcctg agtttctcat ctgtgcccct ccctccctgg cccaggtgaa 840
   ggtgtggttc cagaaccgga ggacaaagta caaacggcag aagctggagg aggaagggcc 900
   tgagtccgag cagaagaaga agggctccca tcacatcaac cggtggcgca ttgccacgaa 960
   gcaggccaat ggggaggaca tcgatgtcac ctccaatgac aagcttgcta gcggtgggca 1020
   accacaaacc cacgagggca gagtgctgct tgctgctggc caggcccctg cgtgggccca 1080
   agctggactc tggccactcc ctggccaggc tttggggagg cctggagtca tggccccaca 1140
   gggcttgaag cccggggccg ccattgacag agggacaagc aatgggctgg ctgaggcctg 1200
   ggaccacttg gccttctcct cggagagcct gcctgcctgg gcgggcccgc ccgccaccgc 1260
   agcctcccag ctgctctccg tgtctccaat ctcccttttg ttttgatgca tttctgtttt 1320
   aatttatttt ccaggcacca ctgtagttta gtgatcccca gtgtccccct tccctatggg 1380
   aataataaaa gtctctctct taatgacacg ggcatccagc tccagcccca gagcctgggg 1440
   tggtagattc cggctctgag ggccagtggg ggctggtaga gcaaacgcgt tcagggcctg 1500
   ggagcctggg gtggggtact ggtggagggg gtcaagggta attcattaac tcctctcttt 1560
   tgttggggga ccctggtctc tacctccagc tccacagcag gagaaacagg ctagacatag 1620
   ggaagggcca tcctgtatct tgagggagga caggcccagg tctttcttaa cgtattgaga 1680
   ggtgggaatc aggcccaggt agttcaatgg gagagggaga gtgcttccct ctgcctagag 1740
   actctggtgg cttctccagt tgaggagaaa ccagaggaaa ggggaggatt ggggtctggg 1800
   ggagggaaca ccattcacaa aggctgacgg ttccagtccg aagtcgtggg cccaccagga 1860
   tgctcacctg tccttggaga accgctgggc aggttgagac tgcagagaca gggcttaagg 1920
   ctgagcctgc aaccagtccc cagtgactca gggcctcctc agcccaagaa agagcaacgt 1980
   gccagggccc gctgagctct tgtgttcacc tg 2012
<210> 51
   <211> 1153
   <212> PRT
   <213> Artificial Sequence
<220>
   <221> source
   <223> /note="Description of Artificial Sequence: Synthetic polypeptide"
<400> 51
   Met Lys Arg Pro Ala Ala Thr Lys Lys Ala Gly Gln Ala Lys Lys Lys
   1               5                   10                  15
   Lys Ser Asp Leu Val Leu Gly Leu Asp Ile Gly Ile Gly Ser Val Gly
               20                  25                  30
   Val Gly Ile Leu Asn Lys Val Thr Gly Glu Ile Ile His Lys Asn Ser
           35                  40                  45
   Arg Ile Phe Pro Ala Ala Gln Ala Glu Asn Asn Leu Val Arg Arg Thr
       50                  55                  60
   Asn Arg Gln Gly Arg Arg Leu Ala Arg Arg Lys Lys His Arg Arg Val
   65                  70                  75                  80
   Arg Leu Asn Arg Leu Phe Glu Glu Ser Gly Leu Ile Thr Asp Phe Thr
                   85                  90                  95
   Lys Ile Ser Ile Asn Leu Asn Pro Tyr Gln Leu Arg Val Lys Gly Leu
               100                 105                 110
   Thr Asp Glu Leu Ser Asn Glu Glu Leu Phe Ile Ala Leu Lys Asn Met
           115                 120                 125
   Val Lys His Arg Gly Ile Ser Tyr Leu Asp Asp Ala Ser Asp Asp Gly
       130                 135                 140
   Asn Ser Ser Val Gly Asp Tyr Ala Gln Ile Val Lys Glu Asn Ser Lys
   145                 150                 155                 160
   Gln Leu Glu Thr Lys Thr Pro Gly Gln Ile Gln Leu Glu Arg Tyr Gln
                   165                 170                 175
   Thr Tyr Gly Gln Leu Arg Gly Asp Phe Thr Val Glu Lys Asp Gly Lys
               180                 185                 190
   Lys His Arg Leu Ile Asn Val Phe Pro Thr Ser Ala Tyr Arg Ser Glu
           195                 200                 205
   Ala Leu Arg Ile Leu Gln Thr Gln Gln Glu Phe Asn Pro Gln Ile Thr
       210                 215                 220
   Asp Glu Phe Ile Asn Arg Tyr Leu Glu Ile Leu Thr Gly Lys Arg Lys
   225                 230                 235                 240
   Tyr Tyr His Gly Pro Gly Asn Glu Lys Ser Arg Thr Asp Tyr Gly Arg
                   245                 250                 255
   Tyr Arg Thr Ser Gly Glu Thr Leu Asp Asn Ile Phe Gly Ile Leu Ile
               260                 265                 270
   Gly Lys Cys Thr Phe Tyr Pro Asp Glu Phe Arg Ala Ala Lys Ala Ser
           275                 280                 285
   Tyr Thr Ala Gln Glu Phe Asn Leu Leu Asn Asp Leu Asn Asn Leu Thr
       290                 295                 300
   Val Pro Thr Glu Thr Lys Lys Leu Ser Lys Glu Gln Lys Asn Gln Ile
   305                 310                 315                 320
   Ile Asn Tyr Val Lys Asn Glu Lys Ala Met Gly Pro Ala Lys Leu Phe
                   325                 330                 335
   Lys Tyr Ile Ala Lys Leu Leu Ser Cys Asp Val Ala Asp Ile Lys Gly
               340                 345                 350
   Tyr Arg Ile Asp Lys Ser Gly Lys Ala Glu Ile His Thr Phe Glu Ala
           355                 360                 365
   Tyr Arg Lys Met Lys Thr Leu Glu Thr Leu Asp Ile Glu Gln Met Asp
       370                 375                 380
   Arg Glu Thr Leu Asp Lys Leu Ala Tyr Val Leu Thr Leu Asn Thr Glu
   385                 390                 395                 400
   Arg Glu Gly Ile Gln Glu Ala Leu Glu His Glu Phe Ala Asp Gly Ser
                   405                 410                 415
   Phe Ser Gln Lys Gln Val Asp Glu Leu Val Gln Phe Arg Lys Ala Asn
               420                 425                 430
   Ser Ser Ile Phe Gly Lys Gly Trp His Asn Phe Ser Val Lys Leu Met
           435                 440                 445
   Met Glu Leu Ile Pro Glu Leu Tyr Glu Thr Ser Glu Glu Gln Met Thr
       450                 455                 460
   Ile Leu Thr Arg Leu Gly Lys Gln Lys Thr Thr Ser Ser Ser Asn Lys
   465 470 475 480
   Thr Lys Tyr Ile Asp Glu Lys Leu Leu Thr Glu Glu Ile Tyr Asn Pro
   485 490 495
   Val Val Ala Lys Ser Val Arg Gln Ala Ile Lys Ile Val Asn Ala Ala
   500 505 510
   Ile Lys Glu Tyr Gly Asp Phe Asp Asn Ile Val Ile Glu Met Ala Arg
   515 520 525
   Glu Thr Asn Glu Asp Asp Glu Lys Lys Ala Ile Gln Lys Ile Gln Lys
   530 535 540
   Ala Asn Lys Asp Glu Lys Asp Ala Ala Met Leu Lys Ala Ala Asn Gln
   545 550 555 560
   Tyr Asn Gly Lys Ala Glu Leu Pro His Ser Val Phe His Gly His Lys
   565 570 575
   Gln Leu Ala Thr Lys Ile Arg Leu Trp His Gln Gln Gly Glu Arg Cys
   580 585 590
   Leu Tyr Thr Gly Lys Thr Ile Ser Ile His Asp Leu Ile Asn Asn Ser
   595 600 605
   Asn Gln Phe Glu Val Asp His Ile Leu Pro Leu Ser Ile Thr Phe Asp
   610 615 620
   Asp Ser Leu Ala Asn Lys Val Leu Val Tyr Ala Thr Ala Asn Gln Glu
   625 630 635 640
   Lys Gly Gln Arg Thr Pro Tyr Gln Ala Leu Asp Ser Met Asp Asp Ala
   645 650 655
   Trp Ser Phe Arg Glu Leu Lys Ala Phe Val Arg Glu Ser Lys Thr Leu
   660 665 670
   Ser Asn Lys Lys Lys Glu Tyr Leu Leu Thr Glu Glu Asp Ile Ser Lys
   675 680 685
   Phe Asp Val Arg Lys Lys Phe Ile Glu Arg Asn Leu Val Asp Thr Arg
   690 695 700
   Tyr Ala Ser Arg Val Val Leu Asn Ala Leu Gln Glu His Phe Arg Ala
   705 710 715 720
   His Lys Ile Asp Thr Lys Val Ser Val Val Arg Gly Gln Phe Thr Ser
   725 730 735
   Gln Leu Arg Arg His Trp Gly Ile Glu Lys Thr Arg Asp Thr Tyr His
   740 745 750
   His His Ala Val Asp Ala Leu Ile Ile Ala Ala Ser Ser Gln Leu Asn
   755 760 765
   Leu Trp Lys Lys Gln Lys Asn Thr Leu Val Ser Tyr Ser Glu Asp Gln
   770 775 780
   Leu Leu Asp Ile Glu Thr Gly Glu Leu Ile Ser Asp Asp Glu Tyr Lys
   785 790 795 800
   Glu Ser Val Phe Lys Ala Pro Tyr Gln His Phe Val Asp Thr Leu Lys
   805 810 815
   Ser Lys Glu Phe Glu Asp Ser Ile Leu Phe Ser Tyr Gln Val Asp Ser
   820 825 830
   Lys Phe Asn Arg Lys Ile Ser Asp Ala Thr Ile Tyr Ala Thr Arg Gln
   835 840 845
   Ala Lys Val Gly Lys Asp Lys Ala Asp Glu Thr Tyr Val Leu Gly Lys
   850 855 860
   Ile Lys Asp Ile Tyr Thr Gln Asp Gly Tyr Asp Ala Phe Met Lys Ile
   865 870 875 880
   Tyr Lys Lys Asp Lys Ser Lys Phe Leu Met Tyr Arg His Asp Pro Gln
   885 890 895
   Thr Phe Glu Lys Val Ile Glu Pro Ile Leu Glu Asn Tyr Pro Asn Lys
   900 905 910
   Gln Ile Asn Glu Lys Gly Lys Glu Val Pro Cys Asn Pro Phe Leu Lys
   915 920 925
   Tyr Lys Glu Glu His Gly Tyr Ile Arg Lys Tyr Ser Lys Lys Gly Asn
   930 935 940
   Gly Pro Glu Ile Lys Ser Leu Lys Tyr Tyr Asp Ser Lys Leu Gly Asn
   945 950 955 960
   His Ile Asp Ile Thr Pro Lys Asp Ser Asn Asn Lys Val Val Leu Gln
   965 970 975
   Ser Val Ser Pro Trp Arg Ala Asp Val Tyr Phe Asn Lys Thr Thr Gly
   980 985 990
   Lys Tyr Glu Ile Leu Gly Leu Lys Tyr Ala Asp Leu Gln Phe Glu Lys
   995 1000 1005
   Gly Thr Gly Thr Tyr Lys Ile Ser Gln Glu Lys Tyr Asn Asp Ile
   1010 1015 1020
   Lys Lys Lys Glu Gly Val Asp Ser Asp Ser Glu Phe Lys Phe Thr
   1025 1030 1035
   Leu Tyr Lys Asn Asp Leu Leu Leu Val Lys Asp Thr Glu Thr Lys
   1040 1045 1050
   Glu Gln Gln Leu Phe Arg Phe Leu Ser Arg Thr Met Pro Lys Gln
   1055 1060 1065
   Lys His Tyr Val Glu Leu Lys Pro Tyr Asp Lys Gln Lys Phe Glu
   1070 1075 1080
   Gly Gly Glu Ala Leu Ile Lys Val Leu Gly Asn Val Ala Asn Ser
   1085 1090 1095
   Gly Gln Cys Lys Lys Gly Leu Gly Lys Ser Asn Ile Ser Ile Tyr
   1100 1105 1110
   Lys Val Arg Thr Asp Val Leu Gly Asn Gln His Ile Ile Lys Asn
   1115 1120 1125
   Glu Gly Asp Lys Pro Lys Leu Asp Phe Lys Arg Pro Ala Ala Thr
   1130 1135 1140
   Lys Lys Ala Gly Gln Ala Lys Lys Lys Lys
   1145 1150
<210> 52
   <211> 340
   <212> DNA
   <213> Artificial Sequence
<220>
   <221> source
   <223> /note="Description of Artificial Sequence: Synthetic polynucleotide"
<400> 52
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ttacttaaat cttgcagaag ctacaaagat aaggcttcat gccgaaatca 300
   acaccctgtc attttatggc agggtgtttt cgttatttaa 340
<210> 53
   <211> 360
   <212> DNA
   <213> Artificial Sequence
<220>
   <221> source
   <223> /note="Description of Artificial Sequence: Synthetic polynucleotide"
<220>
   <221> modified_base
   <222> (288)..(317)
   <223> a, c, t, g, unknown or other
<400> 53
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ggttttagag ctatgctgtt ttgaatggtc ccaaaacnnn nnnnnnnnnn 300
   nnnnnnnnnn nnnnnnngtt ttagagctat gctgttttga atggtcccaa aacttttttt 360
<210> 54
   <211> 318
   <212> DNA
   <213> Artificial Sequence
<220>
   <221> source
   <223> /note="Description of Artificial Sequence: Synthetic polynucleotide"
<220>
   <221> modified_base
   <222> (250).. (269)
   <223> a, c, t, g, unknown or other
<400> 54
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccn nnnnnnnnnn nnnnnnnnng ttttagagct agaaatagca agttaaaata 300
   aggctagtcc gttttttt 318
<210> 55
   <211> 325
   <212> DNA
   <213> Artificial Sequence
<220>
   <221> source
   <223> /note="Description of Artificial Sequence: Synthetic polynucleotide"
<220>
   <221> modified_base
   <222> (250)..(269)
   <223> a, c, t, g, unknown or other
<400> 55
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccn nnnnnnnnnn nnnnnnnnng ttttagagct agaaatagca agttaaaata 300
   aggctagtcc gttatcattt ttttt 325
<210> 56
   <211> 337
   <212> DNA
   <213> Artificial Sequence
<220>
   <221> source
   <223> /note="Description of Artificial Sequence: Synthetic polynucleotide"
<220>
   <221> modified_base
   <222> (250)..(269)
   <223> a, c, t, g, unknown or other
<400> 56
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccn nnnnnnnnnn nnnnnnnnng ttttagagct agaaatagca agttaaaata 300
   aggctagtcc gttatcaact tgaaaaagtg ttttttt 337
<210> 57
   <211> 352
   <212> DNA
   <213> Artificial Sequence
<220>
   <221> source
   <223> /note="Description of Artificial Sequence: Synthetic polynucleotide"
<220>
   <221> modified_base
   <222> (250) .. (269)
   <223> a, c, t, g, unknown or other
<400> 57
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccn nnnnnnnnnn nnnnnnnnng ttttagagct agaaatagca agttaaaata 300
   aggctagtcc gttatcaact tgaaaaagtg gcaccgagtc ggtgcttttt tt 352
<210> 58
   <211> 5101
   <212> DNA
   <213> Artificial Sequence
<220>
   <221> source
   <223> /note="Description of Artificial Sequence: Synthetic polynucleotide"
<400> 58
   cgttacataa cttacggtaa atggcccgcc tggctgaccg cccaacgacc cccgcccatt 60
   gacgtcaata atgacgtatg ttcccatagt aacgccaata gggactttcc attgacgtca 120
   atgggtggag tatttacggt aaactgccca cttggcagta catcaagtgt atcatatgcc 180
   aagtacgccc cctattgacg tcaatgacgg taaatggccc gcctggcatt atgcccagta 240
   catgacctta tgggactttc ctacttggca gtacatctac gtattagtca tcgctattac 300
   catggtcgag gtgagcccca cgttctgctt cactctcccc atctcccccc cctccccacc 360
   cccaattttg tatttattta ttttttaatt attttgtgca gcgatggggg cggggggggg 420
   gggggggcgc gcgccaggcg gggcggggcg gggcgagggg cggggcgggg cgaggcggag 480
   aggtgcggcg gcagccaatc agagcggcgc gctccgaaag tttcctttta tggcgaggcg 540
   gcggcggcgg cggccctata aaaagcgaag cgcgcggcgg gcgggagtcg ctgcgacgct 600
   gccttcgccc cgtgccccgc tccgccgccg cctcgcgccg cccgccccgg ctctgactga 660
   ccgcgttact cccacaggtg agcgggcggg acggcccttc tcctccgggc tgtaattagc 720
   tgagcaagag gtaagggttt aagggatggt tggttggtgg ggtattaatg tttaattacc 780
   tggagcacct gcctgaaatc actttttttc aggttggacc ggtgccacca tggactataa 840
   ggaccacgac ggagactaca aggatcatga tattgattac aaagacgatg acgataagat 900
   ggccccaaag aagaagcgga aggtcggtat ccacggagtc ccagcagccg acaagaagta 960
   cagcatcggc ctggacatcg gcaccaactc tgtgggctgg gccgtgatca ccgacgagta 1020
   caaggtgccc agcaagaaat tcaaggtgct gggcaacacc gaccggcaca gcatcaagaa 1080
   gaacctgatc ggagccctgc tgttcgacag cggcgaaaca gccgaggcca cccggctgaa 1140
   gagaaccgcc agaagaagat acaccagacg gaagaaccgg atctgctatc tgcaagagat 1200
   cttcagcaac gagatggcca aggtggacga cagcttcttc cacagactgg aagagtcctt 1260
   cctggtggaa gaggataaga agcacgagcg gcaccccatc ttcggcaaca tcgtggacga 1320
   ggtggcctac cacgagaagt accccaccat ctaccacctg agaaagaaac tggtggacag 1380
   caccgacaag gccgacctgc ggctgatcta tctggccctg gcccacatga tcaagttccg 1440
   gggccacttc ctgatcgagg gcgacctgaa ccccgacaac agcgacgtgg acaagctgtt 1500
   catccagctg gtgcagacct acaaccagct gttcgaggaa aaccccatca acgccagcgg 1560
   cgtggacgcc aaggccatcc tgtctgccag actgagcaag agcagacggc tggaaaatct 1620
   gatcgcccag ctgcccggcg agaagaagaa tggcctgttc ggcaacctga ttgccctgag 1680
   cctgggcctg acccccaact tcaagagcaa cttcgacctg gccgaggatg ccaaactgca 1740
   gctgagcaag gacacctacg acgacgacct ggacaacctg ctggcccaga tcggcgacca 1800
   gtacgccgac ctgtttctgg ccgccaagaa cctgtccgac gccatcctgc tgagcgacat 1860
   cctgagagtg aacaccgaga tcaccaaggc ccccctgagc gcctctatga tcaagagata 1920
   cgacgagcac caccaggacc tgaccctgct gaaagctctc gtgcggcagc agctgcctga 1980
   gaagtacaaa gagattttct tcgaccagag caagaacggc tacgccggct acattgacgg 2040
   cggagccagc caggaagagt tctacaagtt catcaagccc atcctggaaa agatggacgg 2100
   caccgaggaa ctgctcgtga agctgaacag agaggacctg ctgcggaagc agcggacctt 2160
   cgacaacggc agcatccccc accagatcca cctgggagag ctgcacgcca ttctgcggcg 2220
   gcaggaagat ttttacccat tcctgaagga caaccgggaa aagatcgaga agatcctgac 2280
   cttccgcatc ccctactacg tgggccctct ggccagggga aacagcagat tcgcctggat 2340
   gaccagaaag agcgaggaaa ccatcacccc ctggaacttc gaggaagtgg tggacaaggg 2400
   cgcttccgcc cagagcttca tcgagcggat gaccaacttc gataagaacc tgcccaacga 2460
   gaaggtgctg cccaagcaca gcctgctgta cgagtacttc accgtgtata acgagctgac 2520
   caaagtgaaa tacgtgaccg agggaatgag aaagcccgcc ttcctgagcg gcgagcagaa 2580
   aaaggccatc gtggacctgc tgttcaagac caaccggaaa gtgaccgtga agcagctgaa 2640
   agaggactac ttcaagaaaa tcgagtgctt cgactccgtg gaaatctccg gcgtggaaga 2700
   tcggttcaac gcctccctgg gcacatacca cgatctgctg aaaattatca aggacaagga 2760
   cttcctggac aatgaggaaa acgaggacat tctggaagat atcgtgctga ccctgacact 2820
   gtttgaggac agagagatga tcgaggaacg gctgaaaacc tatgcccacc tgttcgacga 2880
   caaagtgatg aagcagctga agcggcggag atacaccggc tggggcaggc tgagccggaa 2940
   gctgatcaac ggcatccggg acaagcagtc cggcaagaca atcctggatt tcctgaagtc 3000
   cgacggcttc gccaacagaa acttcatgca gctgatccac gacgacagcc tgacctttaa 3060
   agaggacatc cagaaagccc aggtgtccgg ccagggcgat agcctgcacg agcacattgc 3120
   caatctggcc ggcagccccg ccattaagaa gggcatcctg cagacagtga aggtggtgga 3180
   cgagctcgtg aaagtgatgg gccggcacaa gcccgagaac atcgtgatcg aaatggccag 3240
   agagaaccag accacccaga agggacagaa gaacagccgc gagagaatga agcggatcga 3300
   agagggcatc aaagagctgg gcagccagat cctgaaagaa caccccgtgg aaaacaccca 3360
   gctgcagaac gagaagctgt acctgtacta cctgcagaat gggcgggata tgtacgtgga 3420
   ccaggaactg gacatcaacc ggctgtccga ctacgatgtg gaccatatcg tgcctcagag 3480
   ctttctgaag gacgactcca tcgacaacaa ggtgctgacc agaagcgaca agaaccgggg 3540
   caagagcgac aacgtgccct ccgaagaggt cgtgaagaag atgaagaact actggcggca 3600
   gctgctgaac gccaagctga ttacccagag aaagttcgac aatctgacca aggccgagag 3660
   aggcggcctg agcgaactgg ataaggccgg cttcatcaag agacagctgg tggaaacccg 3720
   gcagatcaca aagcacgtgg cacagatcct ggactcccgg atgaacacta agtacgacga 3780
   gaatgacaag ctgatccggg aagtgaaagt gatcaccctg aagtccaagc tggtgtccga 3840
   tttccggaag gatttccagt tttacaaagt gcgcgagatc aacaactacc accacgccca 3900
   cgacgcctac ctgaacgccg tcgtgggaac cgccctgatc aaaaagtacc ctaagctgga 3960
   aagcgagttc gtgtacggcg actacaaggt gtacgacgtg cggaagatga tcgccaagag 4020
   cgagcaggaa atcggcaagg ctaccgccaa gtacttcttc tacagcaaca tcatgaactt 4080
   tttcaagacc gagattaccc tggccaacgg cgagatccgg aagcggcctc tgatcgagac 4140
   aaacggcgaa accggggaga tcgtgtggga taagggccgg gattttgcca ccgtgcggaa 4200
   agtgctgagc atgccccaag tgaatatcgt gaaaaagacc gaggtgcaga caggcggctt 4260
   cagcaaagag tctatcctgc ccaagaggaa cagcgataag ctgatcgcca gaaagaagga 4320
   ctgggaccct aagaagtacg gcggcttcga cagccccacc gtggcctatt ctgtgctggt 4380
   ggtggccaaa gtggaaaagg gcaagtccaa gaaactgaag agtgtgaaag agctgctggg 4440
   gatcaccatc atggaaagaa gcagcttcga gaagaatccc atcgactttc tggaagccaa 4500
   gggctacaaa gaagtgaaaa aggacctgat catcaagctg cctaagtact ccctgttcga 4560
   gctggaaaac ggccggaaga gaatgctggc ctctgccggc gaactgcaga agggaaacga 4620
   actggccctg ccctccaaat atgtgaactt cctgtacctg gccagccact atgagaagct 4680
   gaagggctcc cccgaggata atgagcagaa acagctgttt gtggaacagc acaagcacta 4740
   cctggacgag atcatcgagc agatcagcga gttctccaag agagtgatcc tggccgacgc 4800
   taatctggac aaagtgctgt ccgcctacaa caagcaccgg gataagccca tcagagagca 4860
   ggccgagaat atcatccacc tgtttaccct gaccaatctg ggagcccctg ccgccttcaa 4920
   gtactttgac accaccatcg accggaagag gtacaccagc accaaagagg tgctggacgc 4980
   caccctgatc caccagagca tcaccggcct gtacgagaca cggatcgacc tgtctcagct 5040
   gggaggcgac tttctttttc ttagcttgac cagctttctt agtagcagca ggacgcttta 5100
   a 5101
<210> 59
   <211> 137
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 59
   nnnnnnnnnn nnnnnnnnnn gtttttgtac tctcaagatt tagaaataaa tcttgcagaa 60
   gctacaaaga taaggcttca tgccgaaatc aacaccctgt cattttatgg cagggtgttt 120
   tcgttattta atttttt 137
<210> 60
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 60
   nnnnnnnnnn nnnnnnnnnn gtttttgtac tctcagaaat gcagaagcta caaagataag 60
   gcttcatgcc gaaatcaaca ccctgtcatt ttatggcagg gtgttttcgt tatttaattt 120
   ttt 123
<210> 61
   <211> 110
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 61
   nnnnnnnnnn nnnnnnnnnn gtttttgtac tctcagaaat gcagaagcta caaagataag 60
   gcttcatgcc gaaatcaaca ccctgtcatt ttatggcagg gtgttttttt 110
<210> 62
   <211> 137
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 62
   nnnnnnnnnn nnnnnnnnnn gttattgtac tctcaagatt tagaaataaa tcttgcagaa 60
   gctacaaaga taaggcttca tgccgaaatc aacaccctgt cattttatgg cagggtgttt 120
   tcgttattta atttttt 137
<210> 63
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 63
   nnnnnnnnnn nnnnnnnnnn gttattgtac tctcagaaat gcagaagcta caaagataag 60
   gcttcatgcc gaaatcaaca ccctgtcatt ttatggcagg gtgttttcgt tatttaattt 120
   ttt 123
<210> 64
   <211> 110
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 64
   nnnnnnnnnn nnnnnnnnnn gttattgtac tctcagaaat gcagaagcta caaagataag 60
   gcttcatgcc gaaatcaaca ccctgtcatt ttatggcagg gtgttttttt 110
<210> 65
   <211> 137
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 65
   nnnnnnnnnn nnnnnnnnnn gttattgtac tctcaagatt tagaaataaa tcttgcagaa 60
   gctacaatga taaggcttca tgccgaaatc aacaccctgt cattttatgg cagggtgttt 120
   tcgttattta atttttt 137
<210> 66
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 66
   nnnnnnnnnn nnnnnnnnnn gttattgtac tctcagaaat gcagaagcta caatgataag 60
   gcttcatgcc gaaatcaaca ccctgtcatt ttatggcagg gtgttttcgt tatttaattt 120
   ttt 123
<210> 67
   <211> 110
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 67
   nnnnnnnnnn nnnnnnnnnn gttattgtac tctcagaaat gcagaagcta caatgataag 60
   gcttcatgcc gaaatcaaca ccctgtcatt ttatggcagg gtgttttttt 110
<210> 68
   <211> 107
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 68
   nnnnnnnnnn nnnnnnnnnn gttttagagc tgtggaaaca cagcgagtta aaataaggct     60
   tagtccgtac tcaacttgaa aaggtggcac cgattcggtg ttttttt                  107
<210> 69
   <211> 4263
   <212> DNA
   <213> Artificial Sequence
<220>
   <221> source
   <223> /note="Description of Artificial Sequence: Synthetic polynucleotide"
<400> 69
   atgaaaaggc cggcggccac gaaaaaggcc ggccaggcaa aaaagaaaaa gaccaagccc      60
   tacagcatcg gcctggacat cggcaccaat agcgtgggct gggccgtgac caccgacaac     120
   tacaaggtgc ccagcaagaa aatgaaggtg ctgggcaaca cctccaagaa gtacatcaag     180
   aaaaacctgc tgggcgtgct gctgttcgac agcggcatta cagccgaggg cagacggctg     240
   aagagaaccg ccagacggcg gtacacccgg cggagaaaca gaatcctgta tctgcaagag     300
   atcttcagca ccgagatggc taccctggac gacgccttct tccagcggct ggacgacagc     360
   ttcctggtgc ccgacgacaa gcgggacagc aagtacccca tcttcggcaa cctggtggaa     420
   gagaaggcct accacgacga gttccccacc atctaccacc tgagaaagta cctggccgac     480
   agcaccaaga aggccgacct gagactggtg tatctggccc tggcccacat gatcaagtac     540
   cggggccact tcctgatcga gggcgagttc aacagcaaga acaacgacat ccagaagaac     600
   ttccaggact tcctggacac ctacaacgcc atcttcgaga gcgacctgtc cctggaaaac     660
   agcaagcagc tggaagagat cgtgaaggac aagatcagca agctggaaaa gaaggaccgc     720
   atcctgaagc tgttccccgg cgagaagaac agcggaatct tcagcgagtt tctgaagctg     780
   atcgtgggca accaggccga cttcagaaag tgcttcaacc tggacgagaa agccagcctg     840
   cacttcagca aagagagcta cgacgaggac ctggaaaccc tgctgggata tatcggcgac     900
   gactacagcg acgtgttcct gaaggccaag aagctgtacg acgctatcct gctgagcggc     960
   ttcctgaccg tgaccgacaa cgagacagag gccccactga gcagcgccat gattaagcgg    1020
   tacaacgagc acaaagagga tctggctctg ctgaaagagt acatccggaa catcagcctg    1080
   aaaacctaca atgaggtgtt caaggacgac accaagaacg gctacgccgg ctacatcgac    1140
   ggcaagacca accaggaaga tttctatgtg tacctgaaga agctgctggc cgagttcgag    1200
   ggggccgact actttctgga aaaaatcgac cgcgaggatt tcctgcggaa gcagcggacc 1260
   ttcgacaacg gcagcatccc ctaccagatc catctgcagg aaatgcgggc catcctggac 1320
   aagcaggcca agttctaccc attcctggcc aagaacaaag agcggatcga gaagatcctg 1380
   accttccgca tcccttacta cgtgggcccc ctggccagag gcaacagcga ttttgcctgg 1440
   tccatccgga agcgcaatga gaagatcacc ccctggaact tcgaggacgt gatcgacaaa 1500
   gagtccagcg ccgaggcctt catcaaccgg atgaccagct tcgacctgta cctgcccgag 1560
   gaaaaggtgc tgcccaagca cagcctgctg tacgagacat tcaatgtgta taacgagctg 1620
   accaaagtgc ggtttatcgc cgagtctatg cgggactacc agttcctgga ctccaagcag 1680
   aaaaaggaca tcgtgcggct gtacttcaag gacaagcgga aagtgaccga taaggacatc 1740
   atcgagtacc tgcacgccat ctacggctac gatggcatcg agctgaaggg catcgagaag 1800
   cagttcaact ccagcctgag cacataccac gacctgctga acattatcaa cgacaaagaa 1860
   tttctggacg actccagcaa cgaggccatc atcgaagaga tcatccacac cctgaccatc 1920
   tttgaggacc gcgagatgat caagcagcgg ctgagcaagt tcgagaacat cttcgacaag 1980
   agcgtgctga aaaagctgag cagacggcac tacaccggct ggggcaagct gagcgccaag 2040
   ctgatcaacg gcatccggga cgagaagtcc ggcaacacaa tcctggacta cctgatcgac 2100
   gacggcatca gcaaccggaa cttcatgcag ctgatccacg acgacgccct gagcttcaag 2160
   aagaagatcc agaaggccca gatcatcggg gacgaggaca agggcaacat caaagaagtc 2220
   gtgaagtccc tgcccggcag ccccgccatc aagaagggaa tcctgcagag catcaagatc 2280
   gtggacgagc tcgtgaaagt gatgggcggc agaaagcccg agagcatcgt ggtggaaatg 2340
   gctagagaga accagtacac caatcagggc aagagcaaca gccagcagag actgaagaga 2400
   ctggaaaagt ccctgaaaga gctgggcagc aagattctga aagagaatat ccctgccaag 2460
   ctgtccaaga tcgacaacaa cgccctgcag aacgaccggc tgtacctgta ctacctgcag 2520
   aatggcaagg acatgtatac aggcgacgac ctggatatcg accgcctgag caactacgac 2580
   atcgaccata ttatccccca ggccttcctg aaagacaaca gcattgacaa caaagtgctg 2640
   gtgtcctccg ccagcaaccg cggcaagtcc gatgatgtgc ccagcctgga agtcgtgaaa 2700
   aagagaaaga ccttctggta tcagctgctg aaaagcaagc tgattagcca gaggaagttc 2760
   gacaacctga ccaaggccga gagaggcggc ctgagccctg aagataaggc cggcttcatc 2820
   cagagacagc tggtggaaac ccggcagatc accaagcacg tggccagact gctggatgag 2880
   aagtttaaca acaagaagga cgagaacaac cgggccgtgc ggaccgtgaa gatcatcacc 2940
   ctgaagtcca ccctggtgtc ccagttccgg aaggacttcg agctgtataa agtgcgcgag 3000
   atcaatgact ttcaccacgc ccacgacgcc tacctgaatg ccgtggtggc ttccgccctg 3060
   ctgaagaagt accctaagct ggaacccgag ttcgtgtacg gcgactaccc caagtacaac 3120
   tccttcagag agcggaagtc cgccaccgag aaggtgtact tctactccaa catcatgaat 3180
   atctttaaga agtccatctc cctggccgat ggcagagtga tcgagcggcc cctgatcgaa 3240
   gtgaacgaag agacaggcga gagcgtgtgg aacaaagaaa gcgacctggc caccgtgcgg 3300
   cgggtgctga gttatcctca agtgaatgtc gtgaagaagg tggaagaaca gaaccacggc 3360
   ctggatcggg gcaagcccaa gggcctgttc aacgccaacc tgtccagcaa gcctaagccc 3420
   aactccaacg agaatctcgt gggggccaaa gagtacctgg accctaagaa gtacggcgga 3480
   tacgccggca tctccaatag cttcaccgtg ctcgtgaagg gcacaatcga gaagggcgct 3540
   aagaaaaaga tcacaaacgt gctggaattt caggggatct ctatcctgga ccggatcaac 3600
   taccggaagg ataagctgaa ctttctgctg gaaaaaggct acaaggacat tgagctgatt 3660
   atcgagctgc ctaagtactc cctgttcgaa ctgagcgacg gctccagacg gatgctggcc 3720
   tccatcctgt ccaccaacaa caagcggggc gagatccaca agggaaacca gatcttcctg 3780
   agccagaaat ttgtgaaact gctgtaccac gccaagcgga tctccaacac catcaatgag 3840
   aaccaccgga aatacgtgga aaaccacaag aaagagtttg aggaactgtt ctactacatc 3900
   ctggagttca acgagaacta tgtgggagcc aagaagaacg gcaaactgct gaactccgcc 3960
   ttccagagct ggcagaacca cagcatcgac gagctgtgca gctccttcat cggccctacc 4020
   ggcagcgagc ggaagggact gtttgagctg acctccagag gctctgccgc cgactttgag 4080
   ttcctgggag tgaagatccc ccggtacaga gactacaccc cctctagtct gctgaaggac 4140
   gccaccctga tccaccagag cgtgaccggc ctgtacgaaa cccggatcga cctggctaag 4200
   ctgggcgagg gaaagcgtcc tgctgctact aagaaagctg gtcaagctaa gaaaaagaaa 4260
   taa 4263
<210> 70
   <211> 84
   <212> DNA
   <213> Artificial Sequence
<220>
   <221> source
   <223> /note="Description of Artificial Sequence: Synthetic oligonucleotide"
<400> 70
   ggaaccattc ataacagcat agcaagttat aataaggcta gtccgttatc aacttgaaaa 60
   agtggcaccg agtcggtgct tttt 84
<210> 71
   <211> 36
   <212> DNA
   <213> Artificial Sequence
<220>
   <221> source
   <223> /note="Description of Artificial Sequence: Synthetic oligonucleotide"
<400> 71
   gttatagagc tatgctgtta tgaatggtcc caaaac 36
<210> 72
   <211> 84
   <212> DNA
   <213> Artificial Sequence
<220>
   <221> source
   <223> /note="Description of Artificial Sequence: Synthetic oligonucleotide"
<400> 72
   ggaaccattc aatacagcat agcaagttaa tataaggcta gtccgttatc aacttgaaaa 60
   agtggcaccg agtcggtgct tttt 84
<210> 73
   <211> 36
   <212> DNA
   <213> Artificial Sequence
<220>
   <221> source
   <223> /note="Description of Artificial Sequence: Synthetic oligonucleotide"
<400> 73
   gtattagagc tatgctgtat tgaatggtcc caaaac 36
<210> 74
   <211> 103
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 74
   nnnnnnnnnn nnnnnnnnnn gttttagagc tagaaatagc aagttaaaat aaggctagtc 60
   cgttatcaac ttgaaaaagt ggcaccgagt cggtgctttt ttt 103
<210> 75
   <211> 103
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 75
   nnnnnnnnnn nnnnnnnnnn gtattagagc tagaaatagc aagttaatat aaggctagtc 60
   cgttatcaac ttgaaaaagt ggcaccgagt cggtgctttt ttt 103
<210> 76
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 76
   nnnnnnnnnn nnnnnnnnnn gttttagagc tatgctgttt tggaaacaaa acagcatagc 60
   aagttaaaat aaggctagtc cgttatcaac ttgaaaaagt ggcaccgagt cggtgctttt 120
   ttt 123
<210> 77
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 77
   nnnnnnnnnn nnnnnnnnnn gtattagagc tatgctgtat tggaaacaat acagcatagc 60
   aagttaatat aaggctagtc cgttatcaac ttgaaaaagt ggcaccgagt cggtgctttt 120
   ttt 123
<210> 78
   <211> 20
   <212> DNA
   <213> Homo sapiens
<400> 78
   gtcacctcca atgactaggg 20
<210> 79
   <211> 984
   <212> PRT
   <213> Campylobacter jejuni
<400> 79
   Met Ala Arg Ile Leu Ala Phe Asp Ile Gly Ile Ser Ser Ile Gly Trp
   1 5 10 15
   Ala Phe Ser Glu Asn Asp Glu Leu Lys Asp Cys Gly Val Arg Ile Phe
   20 25 30
   Thr Lys Val Glu Asn Pro Lys Thr Gly Glu Ser Leu Ala Leu Pro Arg
   35 40 45
   Arg Leu Ala Arg Ser Ala Arg Lys Arg Leu Ala Arg Arg Lys Ala Arg
   50 55 60
   Leu Asn His Leu Lys His Leu Ile Ala Asn Glu Phe Lys Leu Asn Tyr
   65 70 75 80
   Glu Asp Tyr Gln Ser Phe Asp Glu Ser Leu Ala Lys Ala Tyr Lys Gly
   85 90 95
   Ser Leu Ile Ser Pro Tyr Glu Leu Arg Phe Arg Ala Leu Asn Glu Leu
   100 105 110
   Leu Ser Lys Gln Asp Phe Ala Arg Val Ile Leu His Ile Ala Lys Arg
   115 120 125
   Arg Gly Tyr Asp Asp Ile Lys Asn Ser Asp Asp Lys Glu Lys Gly Ala
   130 135 140
   Ile Leu Lys Ala Ile Lys Gln Asn Glu Glu Lys Leu Ala Asn Tyr Gln
   145 150 155 160
   Ser Val Gly Glu Tyr Leu Tyr Lys Glu Tyr Phe Gln Lys Phe Lys Glu
   165 170 175
   Asn Ser Lys Glu Phe Thr Asn Val Arg Asn Lys Lys Glu Ser Tyr Glu
   180 185 190
   Arg Cys Ile Ala Gln Ser Phe Leu Lys Asp Glu Leu Lys Leu Ile Phe
   195 200 205
   Lys Lys Gln Arg Glu Phe Gly Phe Ser Phe Ser Lys Lys Phe Glu Glu
   210 215 220
   Glu Val Leu Ser Val Ala Phe Tyr Lys Arg Ala Leu Lys Asp Phe Ser
   225 230 235 240
   His Leu Val Gly Asn Cys Ser Phe Phe Thr Asp Glu Lys Arg Ala Pro
   245 250 255
   Lys Asn Ser Pro Leu Ala Phe Met Phe Val Ala Leu Thr Arg Ile Ile
   260 265 270
   Asn Leu Leu Asn Asn Leu Lys Asn Thr Glu Gly Ile Leu Tyr Thr Lys
   275 280 285
   Asp Asp Leu Asn Ala Leu Leu Asn Glu Val Leu Lys Asn Gly Thr Leu
   290 295 300
   Thr Tyr Lys Gln Thr Lys Lys Leu Leu Gly Leu Ser Asp Asp Tyr Glu
   305 310 315 320
   Phe Lys Gly Glu Lys Gly Thr Tyr Phe Ile Glu Phe Lys Lys Tyr Lys
   325 330 335
   Glu Phe Ile Lys Ala Leu Gly Glu His Asn Leu Ser Gln Asp Asp Leu
   340 345 350
   Asn Glu Ile Ala Lys Asp Ile Thr Leu Ile Lys Asp Glu Ile Lys Leu
   355 360 365
   Lys Lys Ala Leu Ala Lys Tyr Asp Leu Asn Gln Asn Gln Ile Asp Ser
   370 375 380
   Leu Ser Lys Leu Glu Phe Lys Asp His Leu Asn Ile Ser Phe Lys Ala
   385 390 395 400
   Leu Lys Leu Val Thr Pro Leu Met Leu Glu Gly Lys Lys Tyr Asp Glu
   405 410 415
   Ala Cys Asn Glu Leu Asn Leu Lys Val Ala Ile Asn Glu Asp Lys Lys
   420 425 430
   Asp Phe Leu Pro Ala Phe Asn Glu Thr Tyr Tyr Lys Asp Glu Val Thr
   435 440 445
   Asn Pro Val Val Leu Arg Ala Ile Lys Glu Tyr Arg Lys Val Leu Asn
   450 455 460
   Ala Leu Leu Lys Lys Tyr Gly Lys Val His Lys Ile Asn Ile Glu Leu
   465 470 475 480
   Ala Arg Glu Val Gly Lys Asn His Ser Gln Arg Ala Lys Ile Glu Lys
   485 490 495
   Glu Gln Asn Glu Asn Tyr Lys Ala Lys Lys Asp Ala Glu Leu Glu Cys
   500 505 510
   Glu Lys Leu Gly Leu Lys Ile Asn Ser Lys Asn Ile Leu Lys Leu Arg
   515 520 525
   Leu Phe Lys Glu Gln Lys Glu Phe Cys Ala Tyr Ser Gly Glu Lys Ile
   530 535 540
   Lys Ile Ser Asp Leu Gln Asp Glu Lys Met Leu Glu Ile Asp His Ile
   545 550 555 560
   Tyr Pro Tyr Ser Arg Ser Phe Asp Asp Ser Tyr Met Asn Lys Val Leu
   565 570 575
   Val Phe Thr Lys Gln Asn Gln Glu Lys Leu Asn Gln Thr Pro Phe Glu
   580 585 590
   Ala Phe Gly Asn Asp Ser Ala Lys Trp Gln Lys Ile Glu Val Leu Ala
   595 600 605
   Lys Asn Leu Pro Thr Lys Lys Gln Lys Arg Ile Leu Asp Lys Asn Tyr
   610 615 620
   Lys Asp Lys Glu Gln Lys Asn Phe Lys Asp Arg Asn Leu Asn Asp Thr
   625 630 635 640
   Arg Tyr Ile Ala Arg Leu Val Leu Asn Tyr Thr Lys Asp Tyr Leu Asp
   645 650 655
   Phe Leu Pro Leu Ser Asp Asp Glu Asn Thr Lys Leu Asn Asp Thr Gln
   660 665 670
   Lys Gly Ser Lys Val His Val Glu Ala Lys Ser Gly Met Leu Thr Ser
   675 680 685
   Ala Leu Arg His Thr Trp Gly Phe Ser Ala Lys Asp Arg Asn Asn His
   690 695 700
   Leu His His Ala Ile Asp Ala Val Ile Ile Ala Tyr Ala Asn Asn Ser
   705 710 715 720
   Ile Val Lys Ala Phe Ser Asp Phe Lys Lys Glu Gln Glu Ser Asn Ser
   725 730 735
   Ala Glu Leu Tyr Ala Lys Lys Ile Ser Glu Leu Asp Tyr Lys Asn Lys
   740 745 750
   Arg Lys Phe Phe Glu Pro Phe Ser Gly Phe Arg Gln Lys Val Leu Asp
   755 760 765
   Lys Ile Asp Glu Ile Phe Val Ser Lys Pro Glu Arg Lys Lys Pro Ser
   770 775 780
   Gly Ala Leu His Glu Glu Thr Phe Arg Lys Glu Glu Glu Phe Tyr Gln
   785 790 795 800
   Ser Tyr Gly Gly Lys Glu Gly Val Leu Lys Ala Leu Glu Leu Gly Lys
   805 810 815
   Ile Arg Lys Val Asn Gly Lys Ile Val Lys Asn Gly Asp Met Phe Arg
   820 825 830
   Val Asp Ile Phe Lys His Lys Lys Thr Asn Lys Phe Tyr Ala Val Pro
   835 840 845
   Ile Tyr Thr Met Asp Phe Ala Leu Lys Val Leu Pro Asn Lys Ala Val
   850 855 860
   Ala Arg Ser Lys Lys Gly Glu Ile Lys Asp Trp Ile Leu Met Asp Glu
   865 870 875 880
   Asn Tyr Glu Phe Cys Phe Ser Leu Tyr Lys Asp Ser Leu Ile Leu Ile
   885 890 895
   Gln Thr Lys Asp Met Gln Glu Pro Glu Phe Val Tyr Tyr Asn Ala Phe
   900 905 910
   Thr Ser Ser Thr Val Ser Leu Ile Val Ser Lys His Asp Asn Lys Phe
   915 920 925
   Glu Thr Leu Ser Lys Asn Gln Lys Ile Leu Phe Lys Asn Ala Asn Glu
   930 935 940
   Lys Glu Val Ile Ala Lys Ser Ile Gly Ile Gln Asn Leu Lys Val Phe
   945 950 955 960
   Glu Lys Tyr Ile Val Ser Ala Leu Gly Glu Val Thr Lys Ala Glu Phe
   965 970 975
   Arg Gln Arg Glu Asp Phe Lys Lys
   980
<210> 80
   <211> 91
   <212> DNA
   <213> Artificial Sequence
<220>
   <221> source
   <223> /note="Description of Artificial Sequence: Synthetic oligonucleotide"
<400> 80
   tataatctca taagaaattt aaaaagggac taaaataaag agtttgcggg actctgcggg 60
   gttacaatcc cctaaaaccg cttttaaaat t 91
<210> 81
   <211> 36
   <212> DNA
   <213> Artificial Sequence
<220>
   <221> source
   <223> /note="Description of Artificial Sequence: Synthetic oligonucleotide"
<400> 81
   attttaccat aaagaaattt aaaaagggac taaaac 36
<210> 82
   <211> 95
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<400> 82
   nnnnnnnnnn nnnnnnnnnn guuuuagucc cgaaagggac uaaaauaaag aguuugcggg 60
   acucugcggg guuacaaucc ccuaaaaccg cuuuu 95
<210> 83
   <211> 69
   <212> RNA
   <213> Artificial Sequence
<220>
   <221> source
   <223> /note="Description of Artificial Sequence: Synthetic oligonucleotide"
<400> 83
   gucaccucca augacuaggg guuuuagagc uagaaauagc aaguuaaaau aaggcuaguc 60
   cguuuuuuu 69
<210> 84
   <211> 69
   <212> RNA
   <213> Artificial Sequence
<220>
   <221> source
   <223> /note="Description of Artificial Sequence: Synthetic oligonucleotide"
<400> 84
   gacaucgaug uccuccccau guuuuagagc uagaaauagc aaguuaaaau aaggcuaguc 60
   cguuuuuuu 69
<210> 85
   <211> 69
   <212> RNA
   <213> Artificial Sequence
<220>
   <221> source
   <223> /note="Description of Artificial Sequence: Synthetic oligonucleotide"
<400> 85
   gaguccgagc agaagaagaa guuuuagagc uagaaauagc aaguuaaaau aaggcuaguc 60
   cguuuuuuu 69
<210> 86
   <211> 69
   <212> RNA
   <213> Artificial Sequence
<220>
   <221> source
   <223> /note="Description of Artificial Sequence: Synthetic oligonucleotide"
<400> 86
   ggggccgaga uuggguguuc guuuuagagc uagaaauagc aaguuaaaau aaggcuaguc 60
   cguuuuuuu 69
<210> 87
   <211> 69
   <212> RNA
   <213> Artificial Sequence
<220>
   <221> source
   <223> /note="Description of Artificial Sequence: Synthetic oligonucleotide"
<400> 87
   guggcgagag gggccgagau guuuuagagc uagaaauagc aaguuaaaau aaggcuaguc 60
   cguuuuuuu 69
<210> 88
   <211> 76
   <212> RNA
   <213> Artificial Sequence
<220>
   <221> source
   <223> /note="Description of Artificial Sequence: Synthetic oligonucleotide"
<400> 88
   gucaccucca augacuaggg guuuuagagc uagaaauagc aaguuaaaau aaggcuaguc 60
   cguuaucauu uuuuuu 76
<210> 89
   <211> 76
   <212> RNA
   <213> Artificial Sequence
<220>
   <221> source
   <223> /note="Description of Artificial Sequence: Synthetic oligonucleotide"
<400> 89
   gacaucgaug uccuccccau guuuuagagc uagaaauagc aaguuaaaau aaggcuaguc 60
   cguuaucauu uuuuuu 76
<210> 90
   <211> 76
   <212> RNA
   <213> Artificial Sequence
<220>
   <221> source
   <223> /note="Description of Artificial Sequence: Synthetic oligonucleotide"
<400> 90
   gaguccgagc agaagaagaa guuuuagagc uagaaauagc aaguuaaaau aaggcuaguc 60
   cguuaucauu uuuuuu 76
<210> 91
   <211> 76
   <212> RNA
   <213> Artificial Sequence
<220>
   <221> source
   <223> /note="Description of Artificial Sequence: Synthetic oligonucleotide"
<400> 91
   ggggccgaga uuggguguuc guuuuagagc uagaaauagc aaguuaaaau aaggcuaguc 60
   cguuaucauu uuuuuu 76
<210> 92
   <211> 76
   <212> RNA
   <213> Artificial Sequence
<220>
   <221> source
   <223> /note="Description of Artificial Sequence: Synthetic oligonucleotide"
<400> 92
   guggcgagag gggccgagau guuuuagagc uagaaauagc aaguuaaaau aaggcuaguc 60
   cguuaucauu uuuuuu 76
<210> 93
   <211> 88
   <212> RNA
   <213> Artificial Sequence
<220>
   <221> source
   <223> /note="Description of Artificial Sequence: Synthetic oligonucleotide"
<400> 93
   gucaccucca augacuaggg guuuuagagc uagaaauagc aaguuaaaau aaggcuaguc 60
   cguuaucaac uugaaaaagu guuuuuuu 88
<210> 94
   <211> 88
   <212> RNA
   <213> Artificial Sequence
<220>
   <221> source
   <223> /note="Description of Artificial Sequence: Synthetic oligonucleotide"
<400> 94
   gacaucgaug uccuccccau guuuuagagc uagaaauagc aaguuaaaau aaggcuaguc 60
   cguuaucaac uugaaaaagu guuuuuuu 88
<210> 95
   <211> 88
   <212> RNA
   <213> Artificial Sequence
<220>
   <221> source
   <223> /note="Description of Artificial Sequence: Synthetic oligonucleotide"
<400> 95
   gaguccgagc agaagaagaa guuuuagagc uagaaauagc aaguuaaaau aaggcuaguc 60
   cguuaucaac uugaaaaagu guuuuuuu 88
<210> 96
   <211> 88
   <212> RNA
   <213> Artificial Sequence
<220>
   <221> source
   <223> /note="Description of Artificial Sequence: Synthetic oligonucleotide"
<400> 96
   ggggccgaga uuggguguuc guuuuagagc uagaaauagc aaguuaaaau aaggcuaguc 60
   cguuaucaac uugaaaaagu guuuuuuu 88
<210> 97
   <211> 88
   <212> RNA
   <213> Artificial Sequence
<220>
   <221> source
   <223> /note="Description of Artificial Sequence: Synthetic oligonucleotide"
<400> 97
   guggcgagag gggccgagau guuuuagagc uagaaauagc aaguuaaaau aaggcuaguc 60
   cguuaucaac uugaaaaagu guuuuuuu 88
<210> 98
   <211> 103
   <212> RNA
   <213> Artificial Sequence
<220>
   <221> source
   <223> /note="Description of Artificial Sequence: Synthetic oligonucleotide"
<400> 98
   gucaccucca augacuaggg guuuuagagc uagaaauagc aaguuaaaau aaggcuaguc 60
   cguuaucaac uugaaaaagu ggcaccgagu cggugcuuuu uuu 103
<210> 99
   <211> 103
   <212> RNA
   <213> Artificial Sequence
<220>
   <221> source
   <223> /note="Description of Artificial Sequence: Synthetic oligonucleotide"
<400> 99
   gacaucgaug uccuccccau guuuuagagc uagaaauagc aaguuaaaau aaggcuaguc 60
   cguuaucaac uugaaaaagu ggcaccgagu cggugcuuuu uuu 103
<210> 100
   <211> 103
   <212> RNA
   <213> Artificial Sequence
<220>
   <221> source
   <223> /note="Description of Artificial Sequence: Synthetic oligonucleotide"
<400> 100
   gaguccgagc agaagaagaa guuuuagagc uagaaauagc aaguuaaaau aaggcuaguc 60
   cguuaucaac uugaaaaagu ggcaccgagu cggugcuuuu uuu 103
<210> 101
   <211> 103
   <212> RNA
   <213> Artificial Sequence
<220>
   <221> source
   <223> /note="Description of Artificial Sequence: Synthetic oligonucleotide"
<400> 101
   ggggccgaga uuggguguuc guuuuagagc uagaaauagc aaguuaaaau aaggcuaguc 60
   cguuaucaac uugaaaaagu ggcaccgagu cggugcuuuu uuu 103
<210> 102
   <211> 103
   <212> RNA
   <213> Artificial Sequence
<220>
   <221> source
   <223> /note="Description of Artificial Sequence: Synthetic oligonucleotide"
<400> 102
   guggcgagag gggccgagau guuuuagagc uagaaauagc aaguuaaaau aaggcuaguc 60
   cguuaucaac uugaaaaagu ggcaccgagu cggugcuuuu uuu 103
<210> 103
   <211> 102
   <212> DNA
   <213> Artificial Sequence
<220>
   <221> source
   <223> /note="Description of Artificial Sequence: Synthetic polynucleotide"
<400> 103
   gttttagagc tatgctgttt tgaatggtcc caaaacggaa gggcctgagt ccgagcagaa 60
   gaagaagttt tagagctatg ctgttttgaa tggtcccaaa ac 102
<210> 104
   <211> 100
   <212> DNA
   <213> Homo sapiens
<400> 104
   cggaggacaa agtacaaacg gcagaagctg gaggaggaag ggcctgagtc cgagcagaag 60
   aagaagggct cccatcacat caaccggtgg cgcattgcca 100
<210> 105
   <211> 50
   <212> DNA
   <213> Homo sapiens
<400> 105
   agctggagga ggaagggcct gagtccgagc agaagaagaa gggctcccac 50
<210> 106
   <211> 30
   <212> RNA
   <213> Artificial Sequence
<220>
   <221> source
   <223> /note="Description of Artificial Sequence: Synthetic oligonucleotide"
<400> 106
   gaguccgagc agaagaagaa guuuuagagc 30
<210> 107
   <211> 49
   <212> DNA
   <213> Artificial Sequence
<220>
   <221> source
   <223> /note="Description of Artificial Sequence: Synthetic oligonucleotide"
<400> 107
   agctggagga ggaagggcct gagtccgagc agaagagaag ggctcccat 49
<210> 108
   <211> 53
   <212> DNA
   <213> Homo sapiens
<400> 108
   ctggaggagg aagggcctga gtccgagcag aagaagaagg gctcccatca cat 53
<210> 109
   <211> 52
   <212> DNA
   <213> Homo sapiens
<400> 109
   ctggaggagg aagggcctga gtccgagcag aagagaaggg ctcccatcac at 52
<210> 110
   <211> 54
   <212> DNA
   <213> Homo sapiens
<400> 110
   ctggaggagg aagggcctga gtccgagcag aagaaagaag ggctcccatc acat 54
<210> 111
   <211> 50
   <212> DNA
   <213> Homo sapiens
<400> 111
   ctggaggagg aagggcctga gtccgagcag aagaagggct cccatcacat 50
<210> 112
   <211> 47
   <212> DNA
   <213> Homo sapiens
<400> 112
   ctggaggagg aagggcctga gcccgagcag aagggctccc atcacat 47
<210> 113
   <211> 66
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> modified_base
   <222> (1) .. (20)
   <223> a, c, t, g, unknown or other
<400> 113
   nnnnnnnnnn nnnnnnnnnn guuuuagagc uagaaauagc aaguuaaaau aaggctagtc 60
   cguuuu 66
<210> 114
   <211> 20
   <212> RNA
   <213> Artificial Sequence
<220>
   <221> source
   <223> /note="Description of Artificial Sequence: Synthetic oligonucleotide"
<400> 114
   gaguccgagc agaagaagaa 20
<210> 115
   <211> 20
   <212> RNA
   <213> Artificial Sequence
<220>
   <221> source
   <223> /note="Description of Artificial Sequence: Synthetic oligonucleotide"
<400> 115
   gacaucgaug uccuccccau 20
<210> 116
   <211> 20
   <212> RNA
   <213> Artificial Sequence
<220>
   <221> source
   <223> /note="Description of Artificial Sequence: Synthetic oligonucleotide"
<400> 116
   gucaccucca augacuaggg 20
<210> 117
   <211> 20
   <212> RNA
   <213> Artificial Sequence
<220>
   <221> source
   <223> /note="Description of Artificial Sequence: Synthetic oligonucleotide"
<400> 117
   auuggguguu cagggcagag 20
<210> 118
   <211> 20
   <212> RNA
   <213> Artificial Sequence
<220>
   <221> source
   <223> /note="Description of Artificial Sequence: Synthetic oligonucleotide"
<400> 118
   guggcgagag gggccgagau 20
<210> 119
   <211> 20
   <212> RNA
   <213> Artificial Sequence
<220>
   <221> source
   <223> /note="Description of Artificial Sequence: Synthetic oligonucleotide"
<400> 119
   ggggccgaga uuggguguuc 20
<210> 120
   <211> 20
   <212> RNA
   <213> Artificial Sequence
<220>
   <221> source
   <223> /note="Description of Artificial Sequence: Synthetic oligonucleotide"
<400> 120
   gugccauuag cuaaaugcau 20
<210> 121
   <211> 20
   <212> RNA
   <213> Artificial Sequence
<220>
   <221> source
   <223> /note="Description of Artificial Sequence: Synthetic oligonucleotide"
<400> 121
   guaccaccca caggugccag 20
<210> 122
   <211> 20
   <212> RNA
   <213> Artificial Sequence
<220>
   <221> source
   <223> /note="Description of Artificial Sequence: Synthetic oligonucleotide"
<400> 122
   gaaagccucu gggccaggaa 20
<210> 123
   <211> 48
   <212> DNA
   <213> Homo sapiens
<400> 123
   ctggaggagg aagggcctga gtccgagcag aagaagaagg gctcccat 48
<210> 124
   <211> 20
   <212> RNA
   <213> Artificial Sequence
<220>
   <221> source
   <223> /note="Description of Artificial Sequence: Synthetic oligonucleotide"
<400> 124
   gaguccgagc agaagaagau 20
<210> 125
   <211> 20
   <212> RNA
   <213> Artificial Sequence
<220>
   <221> source
   <223> /note="Description of Artificial Sequence: Synthetic oligonucleotide"
<400> 125
   gaguccgagc agaagaagua 20
<210> 126
   <211> 20
   <212> RNA
   <213> Artificial Sequence
<220>
   <221> source
   <223> /note="Description of Artificial Sequence: Synthetic oligonucleotide"
<400> 126
   gaguccgagc agaagaacaa 20
<210> 127
   <211> 20
   <212> RNA
   <213> Artificial Sequence
<220>
   <221> source
   <223> /note="Description of Artificial Sequence: Synthetic oligonucleotide"
<400> 127
   gaguccgagc agaagaugaa 20
<210> 128
   <211> 20
   <212> RNA
   <213> Artificial Sequence
<220>
   <221> source
   <223> /note="Description of Artificial Sequence: Synthetic oligonucleotide"
<400> 128
   gaguccgagc agaaguagaa 20
<210> 129
   <211> 20
   <212> RNA
   <213> Artificial Sequence
<220>
   <221> source
   <223> /note="Description of Artificial Sequence: Synthetic oligonucleotide"
<400> 129
   gaguccgagc agaugaagaa 20
<210> 130
   <211> 20
   <212> RNA
   <213> Artificial Sequence
<220>
   <221> source
   <223> /note="Description of Artificial Sequence: Synthetic oligonucleotide"
<400> 130
   gaguccgagc acaagaagaa 20
<210> 131
   <211> 20
   <212> RNA
   <213> Artificial Sequence
<220>
   <221> source
   <223> /note="Description of Artificial Sequence: Synthetic oligonucleotide"
<400> 131
   gaguccgagg agaagaagaa 20
<210> 132
   <211> 20
   <212> RNA
   <213> Artificial Sequence
<220>
   <221> source
   <223> /note="Description of Artificial Sequence: Synthetic oligonucleotide"
<400> 132
   gaguccgugc agaagaagaa 20
<210> 133
   <211> 20
   <212> RNA
   <213> Artificial Sequence
<220>
   <221> source
   <223> /note="Description of Artificial Sequence: Synthetic oligonucleotide"
<400> 133
   gagucggagc agaagaagaa 20
<210> 134
   <211> 20
   <212> RNA
   <213> Artificial Sequence
<220>
   <221> source
   <223> /note="Description of Artificial Sequence: Synthetic oligonucleotide"
<400> 134
   gagaccgagc agaagaagaa 20
<210> 135
   <211> 24
   <212> DNA
   <213> Artificial Sequence
<220>
   <221> source
   <223> /note="Description of Artificial Sequence: Synthetic oligonucleotide"
<400> 135
   aatgacaagc ttgctagcgg tggg 24
<210> 136
   <211> 39
   <212> DNA
   <213> Artificial Sequence
<220>
   <221> source
   <223> /note="Description of Artificial Sequence: Synthetic oligonucleotide"
<400> 136
   aaaacggaag ggcctgagtc cgagcagaag aagaagttt 39
<210> 137
   <211> 39
   <212> DNA
   <213> Artificial Sequence
<220>
   <221> source
   <223> /note="Description of Artificial Sequence: Synthetic oligonucleotide"
<400> 137
   aaacaggggc cgagattggg tgttcagggc agaggtttt 39
<210> 138
   <211> 38
   <212> DNA
   <213> Artificial Sequence
<220>
   <221> source
   <223> /note="Description of Artificial Sequence: Synthetic oligonucleotide"
<400> 138
   aaaacggaag ggcctgagtc cgagcagaag aagaagtt 38
<210> 139
   <211> 40
   <212> DNA
   <213> Artificial Sequence
<220>
   <221> source
   <223> /note="Description of Artificial Sequence: Synthetic oligonucleotide"
<400> 139
   aacggaggga ggggcacaga tgagaaactc agggttttag 40
<210> 140
   <211> 38
   <212> DNA
   <213> Homo sapiens
<400> 140
   agcccttctt cttctgctcg gactcaggcc cttcctcc 38
<210> 141
   <211> 40
   <212> DNA
   <213> Homo sapiens
<400> 141
   cagggaggga ggggcacaga tgagaaactc aggaggcccc 40
<210> 142
   <211> 80
   <212> DNA
   <213> Artificial Sequence
<220>
   <221> source
   <223> /note="Description of Artificial Sequence: Synthetic oligonucleotide"
<400> 142
   ggcaatgcgc caccggttga tgtgatggga gcccttctag gaggccccca gagcagccac 60
   tggggcctca acactcaggc 80
<210> 143
   <211> 98
   <212> DNA
   <213> Artificial Sequence
<220>
   <221> source
   <223> /note="Description of Artificial Sequence: Synthetic oligonucleotide"
<400> 143
   ggacgaaaca ccggaaccat tcaaaacagc atagcaagtt aaaataaggc tagtccgtta 60
   tcaacttgaa aaagtggcac cgagtcggtg cttttttt 98
<210> 144
   <211> 186
   <212> DNA
   <213> Artificial Sequence
<220>
   <221> source
   <223> /note="Description of Artificial Sequence: Synthetic polynucleotide"
<400> 144
   ggacgaaaca ccggtagtat taagtattgt tttatggctg ataaatttct ttgaatttct 60
   ccttgattat ttgttataaa agttataaaa taatcttgtt ggaaccattc aaaacagcat 120
   agcaagttaa aataaggcta gtccgttatc aacttgaaaa agtggcaccg agtcggtgct 180
   tttttt 186
<210> 145
   <211> 46
   <212> RNA
   <213> Artificial Sequence
<220>
   <221> source
   <223> /note="Description of Artificial Sequence: Synthetic oligonucleotide"
<220>
   <221> modified_base
   <222> (1) .. (19)
   <223> a, c, u, g, unknown or other
<400> 145
   nnnnnnnnnn nnnnnnnnng uuauuguacu cucaagauuu auuuuu 46
<210> 146
   <211> 91
   <212> RNA
   <213> Artificial Sequence
<220>
   <221> source
   <223> /note="Description of Artificial Sequence: Synthetic oligonucleotide"
<400> 146
   guuacuuaaa ucuugcagaa gcuacaaaga uaaggcuuca ugccgaaauc aacacccugu 60
   cauuuuaugg cagggguguu ucguuauuua a 91
<210> 147
   <211> 70
   <212> DNA
   <213> Homo sapiens
<400> 147
   ttttctagtg ctgagtttct gtgactcctc tacattctac ttctctgtgt ttctgtatac 60
   tacctcctcc 70
<210> 148
   <211> 122
   <212> DNA
   <213> Homo sapiens
<400> 148
   ggaggaaggg cctgagtccg agcagaagaa gaagggctcc catcacatca accggtggcg 60
   cattgccacg aagcaggcca atggggagga catcgatgtc acctccaatg actagggtgg 120
   gc 122
<210> 149
   <211> 48
   <212> RNA
   <213> Artificial Sequence
<220>
   <221> source
   <223> /note="Description of Artificial Sequence: Synthetic oligonucleotide"
<220>
   <221> modified_base
   <222> (3) .. (32)
   <223> a, c, u, g, unknown or other
<400> 149
   acnnnnnnnn nnnnnnnnnn nnnnnnnnnn nnguuuuaga gcuaugcu 48
<210> 150
   <211> 67
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 150
   agcauagcaa guuaaaauaa ggctaguccg uuaucaacuu gaaaaagugg caccgagucg 60
   gugcuuu 67
<210> 151
   <211> 62
   <212> RNA
   <213> Artificial Sequence
<220>
   <221> source
   <223> /note="Description of Artificial Sequence: Synthetic oligonucleotide"
<220>
   <221> modified_base
   <222> (1) .. (20)
   <223> a, c, u, g, unknown or other
<400> 151
   nnnnnnnnnn nnnnnnnnnn guuuuagagc uagaaauagc aaguuaaaau aaggcuaguc 60
   cg 62
<210> 152
   <211> 73
   <212> DNA
   <213> Artificial Sequence
<220>
   <221> source
   <223> /note="Description of Artificial Sequence: Synthetic oligonucleotide"
<400> 152
   tgaatggtcc caaaacggaa gggcctgagt ccgagcagaa gaagaagttt tagagctatg 60
   ctgttttgaa tgg 73
<210> 153
   <211> 99
   <212> RNA
   <213> Artificial Sequence
<220>
   <221> source
   <223> /note="Description of Artificial Sequence: Synthetic oligonucleotide"
<220>
   <221> modified_base
   <222> (1) .. (20)
   <223> a, c, u, g, unknown or other
<400> 153
   nnnnnnnnnn nnnnnnnnnn guuuuagagc uagaaauagc aaguuaaaau aaggcuaguc 60
   cguuaucaac uugaaaaagu ggcaccgagu cggugcuuu 99
<210> 154
   <211> 127
   <212> RNA
   <213> Artificial Sequence
<220>
   <221> source
   <223> /note="Description of Artificial Sequence: Synthetic polynucleotide"
<400> 154
   guuuuuguac ucucaagauu uaaguaacug uacaacguua cuuaaaucuu gcagaagcua 60
   caaagauaag gcuucaugcc gaaaucaaca cccugucauu uuauggcagg guguuuucgu 120
   uauuuaa 127
<210> 155
   <211> 56
   <212> DNA
   <213> Artificial Sequence
<220>
   <221> source
   <223> /note="Description of Artificial Sequence: Synthetic oligonucleotide"
<220>
   <221> modified_base
   <222> (1) .. (20)
   <223> a, c, t, g, unknown or other
<400> 155
   nnnnnnnnnn nnnnnnnnnn gtttttgtac tctcaagatt taagtaactg tacaac 56
<210> 156
   <211> 91
   <212> DNA
   <213> Artificial Sequence
<220>
   <221> source
   <223> /note="Description of Artificial Sequence: Synthetic oligonucleotide"
<400> 156
   gttacttaaa tcttgcagaa gctacaaaga taaggcttca tgccgaaatc aacaccctgt 60
   cattttatgg cagggtgttt tcgttattta a 91
<210> 157
   <211> 134
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 157
   nnnnnnnnnn nnnnnnnnnn gtttttgtac tctcaagatt taaggaaact aaatcttgca 60
   gaagctacaa agataaggct tcatgccgaa atcaacaccc tgtcatttta tggcagggtg 120
   ttttcgttat ttaa 134
<210> 158
   <211> 131
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 158
   nnnnnnnnnn nnnnnnnnnn gtttttgtac tctcaagatt tagaaataaa tcttgcagaa 60
   gctacaaaga taaggcttca tgccgaaatc aacaccctgt cattttatgg cagggtgttt 120
   tcgttattta a 131
<210> 159
   <211> 125
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 159
   nnnnnnnnnn nnnnnnnnnn gtttttgtac tctcaagatg aaaatcttgc agaagctaca 60
   aagataaggc ttcatgccga aatcaacacc ctgtcatttt atggcagggt gttttcgtta 120
   tttaa 125
<210> 160
   <211> 112
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 160
   nnnnnnnnnn nnnnnnnnnn gtttttgtac tctgaaaaga agctacaaag ataaggcttc 60
   atgccgaaat caacaccctg tcattttatg gcagggtgtt ttcgttattt aa 112
<210> 161
   <211> 107
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 161
   nnnnnnnnnn nnnnnnnnnn gtttttgtac tgaaaagcta caaagataag gcttcatgcc 60
   gaaatcaaca ccctgtcatt ttatggcagg gtgttttcgt tatttaa 107
<210> 162
   <211> 108
   <212> DNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, t, g, unknown or other
<400> 162
   nnnnnnnnnn nnnnnnnnnn gtttttgtac tctcaagatt tagaaataaa tcttgcagaa 60
   gctacaaaga taaggcttca tgccgaaatc aacaccctgt cattttat 108
<210> 163
   <211> 86
   <212> DNA
   <213> Artificial Sequence
<220>
   <221> source
   <223> /note="Description of Artificial Sequence: Synthetic oligonucleotide"
<220>
   <221> modified_base
   <222> (1) .. (20)
   <223> a, c, t, g, unknown or other
<400> 163
   nnnnnnnnnn nnnnnnnnnn gtttttgtac tctcaagatt tagaaataaa tcttgcagaa 60
   gctacaaaga taaggcttca tgccga 86
<210> 164
   <211> 79
   <212> DNA
   <213> Artificial Sequence
<220>
   <221> source
   <223> /note="Description of Artificial Sequence: Synthetic oligonucleotide"
<220>
   <221> modified_base
   <222> (1) .. (20)
   <223> a, c, t, g, unknown or other
<400> 164
   nnnnnnnnnn nnnnnnnnnn gtttttgtac tctcaagatt tagaaataaa tcttgcagaa 60
   gctacaaaga taaggcttc 79
<210> 165
   <211> 73
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t, g, unknown or other
<400> 165
   nnnnnnnnnn nnnnnnnnnn gtttttgtac tctcaagatt tagaaataaa tcttgcagaa 60
   gctacaaaga taa 73
<210> 166
   <211> 125
   <212> RNA
   <213> Artificial Sequence
<220>
   <221> source
   <223> /note="Description of Artificial Sequence: Synthetic polynucleotide"
<400> 166
   guuuuagucc cuuuuuaaau uucuuuaugg uaaaauuaua aucucauaag aaauuuaaaa 60
   agggacuaaa auaaagaguu ugcgggacuc ugcgggguua caauccccua aaaccgcuuu 120
   uaaaa 125
<210> 167
   <211> 91
   <212> RNA
   <213> Artificial Sequence
<220>
   <221> source
   <223> /note="Description of Artificial Sequence: Synthetic oligonucleotide"
<400> 167
   guuacuuaaa ucuugcagaa gcuacaaaga uaaggcuuca ugccgaaauc aacacccugu 60
   cauuuuaugg caggguguuu ucguuauuua a 91
<210> 168
   <211> 56
   <212> RNA
   <213> Artificial Sequence
<220>
   <221> source
   <223> /note="Description of Artificial Sequence: Synthetic oligonucleotide"
<400> 168
   gggacucaac caagucauuc guuuuuguac ucucaagauu uaaguaacug uacaac 56
<210> 169
   <211> 147
   <212> RNA
   <213> Artificial Sequence
<220>
   <221> source
   <223> /note="Description of Artificial Sequence: Synthetic polynucleotide"
<400> 169
   gggacucaac caagucauuc guuuuuguac ucucaagauu uaaguaacug uacaacguua 60
   cuuaaaucuu gcagaagcua caaagauaag gcuucaugcc gaaaucaaca cccugucauu 120
   uuauggcagg guguuuucgu uauuuaa 147
<210> 170
   <211> 70
   <212> RNA
   <213> Artificial Sequence
<220>
   <221> source
   <223> /note="Description of Artificial Sequence: Synthetic oligonucleotide"
<400> 170
   cuugcagaag cuacaaagau aaggcuucau gccgaaauca acacccuguc auuuuauggc 60
   aggguguuuu 70
<210> 171
   <211> 42
   <212> RNA
   <213> Artificial Sequence
<220>
   <221> source
   <223> /note="Description of Artificial Sequence: Synthetic oligonucleotide"
<400> 171
   gggacucaac caagucauuc guuuuuguac ucucaagauu ua 42
<210> 172
   <211> 112
   <212> RNA
   <213> Artificial Sequence
<220>
   <221> source
   <223> /note="Description of Artificial Sequence: Synthetic polynucleotide"
<400> 172
   gggacucaac caagucauuc guuuuuguac ucucaagauu uacuugcaga agcuacaaag 60
   auaaggcuuc augccgaaau caacacccug ucauuuuaug gcaggguguu uu 112
<210> 173
   <211> 116
   <212> RNA
   <213> Artificial Sequence
<220>
   <221> source
   <223> /note="Description of Artificial Sequence: Synthetic polynucleotide"
<400> 173
   gggacucaac caagucauuc guuuuuguac ucucaagauu uagaaacuug cagaagcuac 60
   aaagauaagg cuucaugccg aaaucaacac ccugucauuu uauggcaggg uguuuu 116
<210> 174
   <211> 116
   <212> RNA
   <213> Artificial Sequence
<220>
   <221> source
   <223> /note="Description of Artificial Sequence: Synthetic polynucleotide"
<400> 174
   gggacucaac caagucauuc guuuuuguac ucucaagauu uagaaacuug cagaagcuac 60
   aaagauaagg cuucaugccg aaaucaacac ccugucauuu uauggcaggg uguuuu 116
<210> 175
   <211> 102
   <212> RNA
   <213> Artificial Sequence
<220>
   <221> source
   <223> /note="Description of Artificial Sequence: Synthetic polynucleotide"
<400> 175
   gggacucaac caagucauuc guuuuuguag aaauacaaag auaaggcuuc augccgaaau 60
   caacacccug ucauuuuaug gcaggguguu uucguuauuu aa 102
<210> 176
   <211> 102
   <212> RNA
   <213> Artificial Sequence
<220>
   <221> source
   <223> /note="Description of Artificial Sequence: Synthetic polynucleotide"
<400> 176
   gggacucaac caagucauuc guuuuuguag aaauacaaag auaaggcuuc augccgaaau 60
   caacacccug ucauuuuaug gcaggguguu uucguuauuu aa 102
<210> 177
   <211> 57
   <212> RNA
   <213> Artificial Sequence
<220>
   <221> source
   <223> /note="Description of Artificial Sequence: Synthetic oligonucleotide"
<400> 177
   gggacucaac caagucauuc guuuuuguag aaauacaaag auaaggcuuc augccga 57
<210> 178
   <211> 57
   <212> RNA
   <213> Artificial Sequence
<220>
   <221> source
   <223> /note="Description of Artificial Sequence: Synthetic oligonucleotide"
<400> 178
   gggacucaac caagucauuc guuuuuguag aaauacaaag auaaggcuuc augccga 57
<210> 179
   <211> 23
   <212> DNA
   <213> Artificial Sequence
<220>
   <221> source
   <223> /note="Description of Artificial Sequence: Synthetic oligonucleotide"
<400> 179
   gtggtgtcac gctcgtcgtt tgg 23
<210> 180
   <211> 23
   <212> DNA
   <213> Artificial Sequence
<220>
   <221> source
   <223> /note="Description of Artificial Sequence: Synthetic oligonucleotide"
<400> 180
   tccagtctat taattgttgc cgg 23
<210> 181
   <211> 64
   <212> DNA
   <213> Homo sapiens
<400> 181
   caagaggctt gagtaggaga ggagtgccgc cgaggcgggg cggggcgggg cgtggagctg 60
   ggct 64
<210> 182
   <211> 99
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<400> 182
   nnnnnnnnnn nnnnnnnnnn guauuagagc uagaaauagc aaguuaauau aaggcuaguc 60
   cguuaucaac uugaaaaagu ggcaccgagu cggugcuuu 99
<210> 183
   <211> 119
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 183
   nnnnnnnnnn nnnnnnnnnn guuuuagagc uaugcuguuu uggaaacaaa acagcauagc 60
   aaguuaaaau aaggcuaguc cguuaucaac uugaaaaagu ggcaccgagu cggugcuuu 119
<210> 184
   <211> 119
   <212> RNA
   <213> Artificial Sequence
<220>
   <221> source
   <223> /note="Description of Artificial Sequence: Synthetic polynucleotide"
<220>
   <221> modified_base
   <222> (1) .. (20)
   <223> a, c, u, g, unknown or other
<400> 184
   nnnnnnnnnn nnnnnnnnnn guauuagagc uaugcuguau uggaaacaau acagcauagc 60
   aaguuaauau aaggcuaguc cguuaucaac uugaaaaagu ggcaccgagu cggugcuuu 119
<210> 185
   <211> 12
   <212> DNA
   <213> Homo sapiens
<400> 185
   tagcgggtaa gc 12
<210> 186
   <211> 12
   <212> DNA
   <213> Homo sapiens
<400> 186
   tcggtgacat gt 12
<210> 187
   <211> 12
   <212> DNA
   <213> Homo sapiens
<400> 187
   actccccgta gg 12
<210> 188
   <211> 12
   <212> DNA
   <213> Homo sapiens
<400> 188
   actgcgtgtt aa 12
<210> 189
   <211> 12
   <212> DNA
   <213> Homo sapiens
<400> 189
   acgtcgcctg at 12
<210> 190
   <211> 12
   <212> DNA
   <213> Homo sapiens
<400> 190
   taggtcgacc ag 12
<210> 191
   <211> 12
   <212> DNA
   <213> Homo sapiens
<400> 191
   ggcgttaatg at 12
<210> 192
   <211> 12
   <212> DNA
   <213> Homo sapiens
<400> 192
   tgtcgcatgt ta 12
<210> 193
   <211> 12
   <212> DNA
   <213> Homo sapiens
<400> 193
   atggaaacgc at 12
<210> 194
   <211> 12
   <212> DNA
   <213> Homo sapiens
<400> 194
   gccgaattcc tc 12
<210> 195
   <211> 12
   <212> DNA
   <213> Homo sapiens
<400> 195
   gcatggtacg ga 12
<210> 196
   <211> 12
   <212> DNA
   <213> Homo sapiens
<400> 196
   cggtactctt ac 12
<210> 197
   <211> 12
   <212> DNA
   <213> Homo sapiens
<400> 197
   gcctgtgccg ta 12
<210> 198
   <211> 12
   <212> DNA
   <213> Homo sapiens
<400> 198
   tacggtaagt cg 12
<210> 199
   <211> 12
   <212> DNA
   <213> Homo sapiens
<400> 199
   cacgaaatta cc 12
<210> 200
   <211> 12
   <212> DNA
   <213> Homo sapiens
<400> 200
   aaccaagata cg 12
<210> 201
   <211> 12
   <212> DNA
   <213> Homo sapiens
<400> 201
   gagtcgatac gc 12
<210> 202
   <211> 12
   <212> DNA
   <213> Homo sapiens
<400> 202
   gtctcacgat cg 12
<210> 203
   <211> 12
   <212> DNA
   <213> Homo sapiens
<400> 203
   tcgtcgggtg ca 12
<210> 204
   <211> 12
   <212> DNA
   <213> Homo sapiens
<400> 204
   actccgtagt ga 12
<210> 205
   <211> 12
   <212> DNA
   <213> Homo sapiens
<400> 205
   caggacgtcc gt 12
<210> 206
   <211> 12
   <212> DNA
   <213> Homo sapiens
<400> 206
   tcgtatccct ac 12
<210> 207
   <211> 12
   <212> DNA
   <213> Homo sapiens
<400> 207
   tttcaaggcc gg 12
<210> 208
   <211> 12
   <212> DNA
   <213> Homo sapiens
<400> 208
   cgccggtgga at 12
<210> 209
   <211> 12
   <212> DNA
   <213> Homo sapiens
<400> 209
   gaacccgtcc ta 12
<210> 210
   <211> 12
   <212> DNA
   <213> Homo sapiens
<400> 210
   gattcatcag cg 12
<210> 211
   <211> 12
   <212> DNA
   <213> Homo sapiens
<400> 211
   acaccggtct tc 12
<210> 212
   <211> 12
   <212> DNA
   <213> Homo sapiens
<400> 212
   atcgtgccct aa 12
<210> 213
   <211> 12
   <212> DNA
   <213> Homo sapiens
<400> 213
   gcgtcaatgt tc 12
<210> 214
   <211> 12
   <212> DNA
   <213> Homo sapiens
<400> 214
   ctccgtatct cg 12
<210> 215
   <211> 12
   <212> DNA
   <213> Homo sapiens
<400> 215
   ccgattcctt cg 12
<210> 216
   <211> 12
   <212> DNA
   <213> Homo sapiens
<400> 216
   tgcgcctcca gt 12
<210> 217
   <211> 12
   <212> DNA
   <213> Homo sapiens
<400> 217
   taacgtcgga gc 12
<210> 218
   <211> 12
   <212> DNA
   <213> Homo sapiens
<400> 218
   aaggtcgccc at 12
<210> 219
   <211> 12
   <212> DNA
   <213> Homo sapiens
<400> 219
   gtcggggact at 12
<210> 220
   <211> 12
   <212> DNA
   <213> Homo sapiens
<400> 220
   ttcgagcgat tt 12
<210> 221
   <211> 12
   <212> DNA
   <213> Homo sapiens
<400> 221
   tgagtcgtcg ag 12
<210> 222
   <211> 12
   <212> DNA
   <213> Homo sapiens
<400> 222
   tttacgcaga gg 12
<210> 223
   <211> 12
   <212> DNA
   <213> Homo sapiens
<400> 223
   aggaagtatc gc 12
<210> 224
   <211> 12
   <212> DNA
   <213> Homo sapiens
<400> 224
   actcgatacc at 12
<210> 225
   <211> 12
   <212> DNA
   <213> Homo sapiens
<400> 225
   cgctacatag ca 12
<210> 226
   <211> 12
   <212> DNA
   <213> Homo sapiens
<400> 226
   ttcataaccg gc 12
<210> 227
   <211> 12
   <212> DNA
   <213> Homo sapiens
<400> 227
   ccaaacggtt aa 12
<210> 228
   <211> 12
   <212> DNA
   <213> Homo sapiens
<400> 228
   cgattccttc gt 12
<210> 229
   <211> 12
   <212> DNA
   <213> Homo sapiens
<400> 229
   cgtcatgaat aa 12
<210> 230
   <211> 12
   <212> DNA
   <213> Homo sapiens
<400> 230
   agtggcgatg ac 12
<210> 231
   <211> 12
   <212> DNA
   <213> Homo sapiens
<400> 231
   cccctacggc ac 12
<210> 232
   <211> 12
   <212> DNA
   <213> Homo sapiens
<400> 232
   gccaacccgc ac 12
<210> 233
   <211> 12
   <212> DNA
   <213> Homo sapiens
<400> 233
   tgggacaccg gt 12
<210> 234
   <211> 12
   <212> DNA
   <213> Homo sapiens
<400> 234
   ttgactgcgg cg 12
<210> 235
   <211> 12
   <212> DNA
   <213> Homo sapiens
<400> 235
   actatgcgta gg 12
<210> 236
   <211> 12
   <212> DNA
   <213> Homo sapiens
<400> 236
   tcacccaaag cg 12
<210> 237
   <211> 12
   <212> DNA
   <213> Homo sapiens
<400> 237
   gcaggacgtc cg 12
<210> 238
   <211> 12
   <212> DNA
   <213> Homo sapiens
<400> 238
   acaccgaaaa cg 12
<210> 239
   <211> 12
   <212> DNA
   <213> Homo sapiens
<400> 239
   cggtgtattg ag 12
<210> 240
   <211> 12
   <212> DNA
   <213> Homo sapiens
<400> 240
   cacgaggtat gc 12
<210> 241
   <211> 12
   <212> DNA
   <213> Homo sapiens
<400> 241
   taaagcgacc cg 12
<210> 242
   <211> 12
   <212> DNA
   <213> Homo sapiens
<400> 242
   cttagtcggc ca 12
<210> 243
   <211> 12
   <212> DNA
   <213> Homo sapiens
<400> 243
   cgaaaacgtg gc 12
<210> 244
   <211> 12
   <212> DNA
   <213> Homo sapiens
<400> 244
   cgtgccctga ac 12
<210> 245
   <211> 12
   <212> DNA
   <213> Homo sapiens
<400> 245
   tttaccatcg aa 12
<210> 246
   <211> 12
   <212> DNA
   <213> Homo sapiens
<400> 246
   cgtagccatg tt 12
<210> 247
   <211> 12
   <212> DNA
   <213> Homo sapiens
<400> 247
   cccaaacggt ta 12
<210> 248
   <211> 12
   <212> DNA
   <213> Homo sapiens
<400> 248
   gcgttatcag aa 12
<210> 249
   <211> 12
   <212> DNA
   <213> Homo sapiens
<400> 249
   tcgatggtaa ac 12
<210> 250
   <211> 12
   <212> DNA
   <213> Homo sapiens
<400> 250
   cgactttttg ca 12
<210> 251
   <211> 12
   <212> DNA
   <213> Homo sapiens
<400> 251
   tcgacgactc ac 12
<210> 252
   <211> 12
   <212> DNA
   <213> Homo sapiens
<400> 252
   acgcgtcaga ta 12
<210> 253
   <211> 12
   <212> DNA
   <213> Homo sapiens
<400> 253
   cgtacggcac ag 12
<210> 254
   <211> 12
   <212> DNA
   <213> Homo sapiens
<400> 254
   ctatgccgtg ca 12
<210> 255
   <211> 12
   <212> DNA
   <213> Homo sapiens
<400> 255
   cgcgtcagat at 12
<210> 256
   <211> 12
   <212> DNA
   <213> Homo sapiens
<400> 256 12
   aagatcggta gc 12
<210> 257
   <211> 12
   <212> DNA
   <213> Homo sapiens
<400> 257
   cttcgcaagg ag 12
<210> 258
   <211> 12
   <212> DNA
   <213> Homo sapiens
<400> 258
   gtcgtggact ac 12
<210> 259
   <211> 12
   <212> DNA
   <213> Homo sapiens
<400> 259
   ggtcgtcatc aa 12
<210> 260
   <211> 12
   <212> DNA
   <213> Homo sapiens
<400> 260
   gttaacagcg tg 12
<210> 261
   <211> 12
   <212> DNA
   <213> Homo sapiens
<400> 261
   tagctaaccg tt 12
<210> 262
   <211> 12
   <212> DNA
   <213> Homo sapiens
<400> 262
   agtaaaggcg ct 12
<210> 263
   <211> 12
   <212> DNA
   <213> Homo sapiens
<400> 263
   ggtaatttcg tg 12
<210> 264
   <211> 147
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 264
   nnnnnnnnnn nnnnnnnnnn guuuuaguac ucuguaauuu uagguaugag guagacgaaa      60
   auuguacuua uaccuaaaau uacagaaucu acuaaaacaa ggcaaaaugc cguguuuauc     120
   ucgucaacuu guuggcgaga uuuuuuu                                         147